# EUROPEAN PATENT SPECIFICATION

(11) **EP 3 377 504 B1**
(45) Date of publication and mention of the grant of the patent: **19.08.2020**
(21) Application number: 16815699.0
(22) Date of filing: 18.11.2016
(51) Int. Cl.: C07F 9/30, C07F 9/38, A61K 49/06, A61B 5/00

(54) **CYCLAM BASED COMPOUNDS, THEIR CONJUGATES, CO-ORDINATION COMPOUNDS, PHARMACEUTICAL COMPOSITIONS THEREOF, METHOD OF PREPARATION AND USE THEREOF**
CYCLAM-BASIERTE VERBINDUNGEN, DEREN KONJUGATE, KOORDINATIONSVERBINDUNGEN, PHARMAZEUTISCHE ZUSAMMENSETZUNG DAMIT, VERFAHREN ZUR HERSTELLUNG UND VERWENDUNG DAVON
COMPOSÉS À BASE DE CYCLAME, LEURS CONJUGUÉS, COMPOSÉS DE COORDINATION, COMPOSITION PHARMACEUTIQUE LES CONTENANT, PROCÉDÉ DE PRÉPARATION ET UTILISATION ASSOCIÉS

(30) Priority: 20.11.2015 CZ 20150825
(43) Date of publication of application: 26.09.2018
(73) Proprietor: Univerzita Karlova, 11636 Praha 1 (CZ)
(72) Inventor: DAVID, Tomas, 25064 Hovorcovice Praha - vychod (CZ); HERMANN, Petr, 19900 Praha - Letnany (CZ); KUBICEK, Vojtech, 18200 Praha 8 (CZ)
(74) Representative: Hartvichova, Katerina
(86) International application number: PCT/CZ2016/050043
(87) International publication number: WO 2017/084645

(56) References cited:
- WO-A1-2012/150302
- TOMÁS DAVID ET AL: "Cyclam Derivatives with a Bis(phosphinate) or a Phosphinato-Phosphonate Pendant Arm: Ligands for Fast and Efficient Copper(II) Complexation for Nuclear Medical Applications", INORGANIC CHEMISTRY, vol. 54, no. 24, 21 December 2015 (2015-12-21), pages 11751-11766, XP055335693, EASTON, US ISSN: 0020-1669, DOI: 10.1021/acs.inorgchem.5b01791
- MONIKA PAÚROVÁ ET AL: "Bifunctional Cyclam-Based Ligands with Phosphorus Acid Pendant Moieties for Radiocopper Separation: Thermodynamic and Kinetic Studies", CHEMISTRY - A EUROPEAN JOURNAL., vol. 21, no. 12, 3 February 2015 (2015-02-03), pages 4671-4687, XP055335710, WEINHEIM, DE ISSN: 0947-6539, DOI: 10.1002/chem.201405777

## Description

### Field of Art

The present invention relates to cyclam based compounds, containing tetraazamacrocycle and bis-phosphorus acid, conjugates of such compounds with conjugation groups, their coordination compounds, targeting conjugate, method of preparation of cyclam based compounds, intermediate products for the preparation of cyclam based compounds, pharmaceutical preparation containing them, and the use thereof.

### Background Art

Polyazamacrocycles with coordinating side chains form thermodynamically very stable complexes with a wide scale of metal ions that are often also highly kinetically inert. These properties thereof are very desirable for applications in medicine and molecular biology. Therefore, these ligands and complexes thereof are widely studied and their properties adapted to a particular application, as for example MRI contract agents, carriers for metal radionuclides in diagnostic or therapeutic methods, targeted medicaments or luminescent probes.
Very powerful diagnostic methods of modern medicine are positron emission tomography (PET) or tomographic scintigraphy (single-photon emission tomography, SPECT), which are based on the application of radio-isotopes, i.e. proton-rich isotopes of elements that undergo β⁺ decay (for PET), or emitting gamma radiation (for SPECT). In PET, a collision of emitted positron with electron from the surrounding tissue leads to origination of a pair of collinear γ-photons (having the energy of 512 keV), which allow a precise detection of place of annihilation thus also the distribution of the radio-isotope in the body. PET requires using radio-isotopes with suitable half-life, low energy of emitted positrons and good availability (for example non-metallic radio-isotopes ¹⁸F, ¹¹C, ¹⁵O, or radioisotopes of metals, such as ⁶⁸Ga, ⁴⁴Sc, ⁸⁹Zr). Great interest was raised up by the radioisotope of copper ⁶⁴Cu (61 % β⁺) thanks to its long half-life τ_{½} 12.8 h) and low energy of positrons (*E*ₐᵥ 0.65 MeV), which leads to a high resolution in PET. Further radioisotopes of copper, positron emitting ⁶⁰Cu (τ_{½} 23.7 min, 100 % β⁺) and ⁶¹Cu (τ_{½} 3.3 h, 100 % β⁺), are also used in PET, and the β⁻-emitting ⁶⁷Cu (τ_{½} 61.8 h, 100 % β⁻) is used in radionuclide therapy. Radio-isotopes ⁶⁴Cu and ⁶⁷Cu form an interesting theranostic pair (one isotope is used for diagnostics and the second isotope for therapy).
Radioisotopes of metals mostly cannot be applied in a form of free ions because of nonspecific accumulation in tissues. In order to achieve the required bio-distribution, the metal ion shall be bound in a thermodynamically stable and kinetically inert complex, and this complex is often conjugated to the targeting vector. There are various chelators for a formation of thermodynamically stable and kinetically inert complexes of metals, while the macrocyclic ligands are preferred. For the complexation of copper radioisotopes, both acyclic and macrocyclic ligands are used, for example diethylenetriaminepentaacetic acid (H₅dtpa), 1,4,8,11-tetraazacyclotetradecane (cyclam), 1,4,8,11-tetraazacyclotetradecane-1,4,8,11-tetraacetic acid (H₄teta), 1,4,7,10-tetraazacyclododecane-1,4,7,10-tetraacetic acid (H₄dota), their monoamides and other derivatives. However, their properties are not optimal for complexation of copper(II) ions, especially because of a slow complexation, low kinetic inertness, and unsuitable redox potential shifted towards copper(I) ions, and, therefore, other ligands were studied, for example derivatives of bridged cyclam, which form kinetically very inert complexes, however, they are burdened by a very slow complexation.
Preparation of various macrocyclic ligands on the basis of cyclen (1,4,7,10-tetraazacyclododecane) or cyclam (1,4,8,11-tetraazacyclotetradecane), with a pendant group containing phosphorus or eventually arsenic, is described for example in US 2008/0312430 A1 or WO2015/038968. The document Fuzerová S., Kotek J., Císařová I., Hermann P., Binnemans K, Lukeš I., Dalton Trans., 2005, 2908-2915, describes the synthesis, the potentiometric and NMR titrations, and the crystallographic structure of a ligand on the basis of cyclam with a pendant phosphonic acid and its copper(II) complexes. The document Kotek J., Lubal P., Hermann P., Císařová I., Lukeš I., Godula T., Svobodová I., Táborský P., Havel J., Chem. Eur. J., 2003, 9, No. 1, 233-248, describes an analogous compound, which, however, contains two pendant phosphonic acids in a trans position. This publication also studies the synthesis, potentiometry, and crystallographic study of the ligand and its copper complexes. It results from the two last mentioned documents that one phosphonic acid group is coordinated to Cu^{II} and it forms a thermodynamically stable and kinetically inert complex. The second phosphonic acid group present in the compound studied in Kotek J., Lubal P., Hermann P., Císařová I., Lukeš I., Godula T., Svobodová I., Táborský P., Havel J., Chem. Eur. J., 2003, 9, No. 1, 233-248 does not coordinate to the copper(II) ion and the thermodynamic stability of copper(II) complexes of both ligands mentioned above is comparable. The cited compounds, however, are not bifunctional, they cannot thus be linked to any targeting vector in order to get to a specific place in the body during biodistribution. Moreover, although the substances cited form stable copper complexes, the complexation rate is not sufficient for that the resulting complex to be created quickly and, if possible, at room temperature. Thus, for the practical use in radiodiagnostics or radiotherapy, these substances are not suitable because of their non-selective biodistribution and low complexation rate.
For targeted biodistribution and dosage reduction, the conjugation of a complex with a targeting vector is used. Targeting vectors may be, for example, various peptides, antibodies and their fragments or derivatives, biotin, folic acid, cyclodextrins, dendrimers, polymers, polysaccharides.
WO 2012/150302 A1 describes metal ion chelators for use in nuclear medicine and molecular imaging (radiopharmaceuticals for imaging and targeted radiotherapy). The azamacrocyclic chelators are functionalized with peptidic or non-peptidic ligands or radioactive or non-radioactive signaling units. The disclosed chelators contain phosphinic groups as pendant arms, and the intended use is in gamma scintigraphy, SPECT, PET or targeted radiotherapy.
Monika Paúrová et al., Chemistry - A European Journal, vol. 21, no. 12, 3 Feb. 2015, 4671-4687, discloses cyclam based macrocyclic ligands with two methylphosphinic or methylphosphonic pendant arms. The ligands are intended to use for complexation of transition metal ions, such as Cu²⁺, and radiocopper separation techniques.
The disadvantage of the prior art is the absence of substances with high specific activity, which is a value indicating the percentage representation of the complex of the radioisotope in the total dose of the medicament. This value is closely related with the capability of ligands to form a thermodynamically stable and kinetically inert coordination compound with radionuclide, with a suitable half-life, quickly and, if possible, at room temperature, which would, at the same time, contain a targeting vector, and therefore would be suitable for applications in diagnostic (for example PET, SPECT) and therapeutic methods. The existing ligands effectively complex radionuclides at temperatures of approximately 80 °C and higher, while at room temperature their complexation is very slow, which causes, for example, loss of radioisotope complexes with a short half-life, high financial costs associated with preparation of radioisotope complexes, as well as the stress of the patient exposed to an application of high doses of the medicament in order to achieve the desired effect.

### Disclosure of Invention

The present invention relates to compounds based on cyclam and/or bridged cyclam for therapy and diagnostics, containing a bis-phosphorus acid, which are characterized by a high specific activity. The compounds according to the present invention complex metal ions quickly and selectively, in particular Cu²⁺ ions, and they form thermodynamically stable and kinetically inert coordination compounds. The compounds according to the present invention may further form conjugates with conjugation groups and further targeting conjugates with targeting vectors that ensure their targeted distribution in the organism.

The subject of the present invention are cyclam based compounds for therapy and diagnostics of the general formula (I) 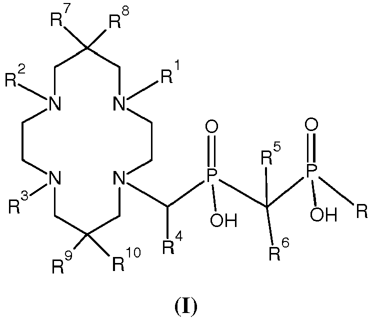 wherein
R, R^{a}, R¹, R², R³ are independently selected from the group comprising
H, OH, (C1 to C6)alkyl, which may be linear or branched, (C3 to C6)cycloalkyl, benzyl, and/or R¹ and/or R² and/or R³ is a bis-phosphorus acid of the general formula **(II)** 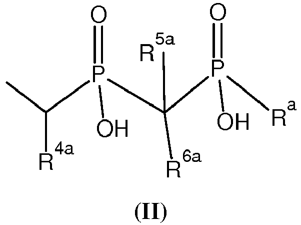
and/or R¹ and R³ together form (C2 to C3)alkylene, which may be substituted with one or more linear or branched (C1 to C6)alkyls,
and/or R, R^{a} are independently selected from phenyl and (C5 to C6)heterocycle, containing at least one heteroatom of N, S, O,
   wherein the (C1 to C6)alkyl, (C3 to C6)cycloalkyl, phenyl, benzyl and (C5 to C6) heterocycle, containing at least one heteroatom of N, S, O, may be independently substituted with one or more groups selected from COOH; NH₂; NO₂, NX₂; C(O)NX₂; NHX; C(O)NHX; OH; SH; -NCS; tetrazine of the general formula **(III);** 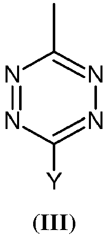 (C7 to C8)cycloalkenyl in *trans* configuration, which may eventually be further substituted with one or more groups selected from COOH, NH₂, NO₂, -NCS, -NCO, N₃ and SH; norbornenyl, eventually substituted with one or more groups selected from COOH, NH₂, NO₂,-NCS, -NCO, N₃ and SH; -N₃; (C5 to C6)heterocycle, containing at least one heteroatom of N, S, O; (C2 to C6)alkynyl, eventually substituted with one or more groups selected from COOH, NH₂, NO₂,-NCS, -NCO, N₃ and SH; cyclooctynyl, eventually substituted with one or more groups chosen from COOH, NH₂, NO₂,-NCS, - NCO, N₃ and SH; cycloazaoctynyl, eventually substituted with one or more groups selected from COOH, NH₂, NO₂,-NCS, -NCO, N₃ and SH; maleimide; phenyl; benzyl and -P(O)(OH)Z;
and wherein R⁴, R^{4a}, R⁵ and R^{5a} are independently H, (C1 to C6)alkyl, which may be linear or branched, (C3 to C6)cycloalkyl, benzyl;
   wherein (C1 to C6)alkyl, (C3 to C6)cycloalkyl and benzyl may be independently substituted with one or more groups selected from COOH; NH₂; NO₂,NX₂; C(O)NX₂; NHX; C(O)NHX; OH; SH; -NCS; tetrazine of the general formula **(III);** (C7 to C8)cycloalkenyl in *trans* configuration, which may eventually be further substituted with one or more groups selected from COOH, NH₂, NO₂,-NCS, -NCO, N₃ and SH; norbornenyl, eventually substituted with one or more groups selected from COOH, NH₂, NO₂, -NCS, -NCO, N₃ and SH; -N₃; (C5 to C6)heterocycle, containing at least one heteroatom of N, S, O; (C2 to C6)alkynyl, eventually substituted with one or more groups selected from COOH, NH₂, NO₂, -NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂, -NCS, -NCO, N₃ and SH; maleimide; phenyl; benzyl and -P(O)(OH)Z;
and wherein R⁶ and R^{6a} is H;
and wherein R⁷, R⁸, R⁹ and R¹⁰ are independently selected from the group comprising H, (C1 to C6)alkyl, (C3 to C6)cycloalkyl, benzyl, phenyl, NO₂, COOH, (C5 to C6)heterocycle, containing at least one heteroatom of N, S, O, and/or altogether R⁷ and R⁸ contain (C2 to C3)alkylene or vinylene and/or altogether R⁹ and R¹⁰ contain (C2 to C3)alkylene or vinylene,
   wherein (C1 to C6)alkyl, (C3 to C6)cycloalkyl, phenyl, benzyl, (C5 to C6)heterocycle, containing at least one heteroatom of N, S, O, (C2 to C3)alkylene and vinylene may be independently substituted with a group or groups selected from COOH; NH₂; NO₂; NX₂; C(O)NX₂; SH; tetrazine of the general formula **(III);** (C7 to C8)cycloalkenyl in *trans* configuration, eventually substituted with one or more groups selected from COOH, NH₂, NO₂,-NCS, -NCO, N₃ and SH; norbornenyl, eventually substituted with one or more groups selected from COOH, NH₂, NO₂,-NCS, -NCO, N₃ and SH; - N₃; (C5 to C6)heterocycle, containing at least one heteroatom of N, S, O; (C2 to C6)alkynyl, eventually substituted with one or more groups selected from COOH, NH₂, NO₂,-NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂,-NCS, -NCO, N₃ and SH; maleimide; phenyl; benzyl and -P(O)(OH)Z,
wherein Y is H, (C1 to C6)alkyl, -CH₂COOH, (C5 to C6)heterocycle, containing at least one heteroatom of N, S, O, or heteroaryl, containing at least one heteroatom of N, S, O, preferably Y is an atom of hydrogen, methyl or pyridyl;
wherein Z is H; OH; (C1 to C6)alkyl, which may be linear or branched; (C1 to C6)hydroxyalkyl; (C1 to C6)alkoxyl; phenyl; (C5 to C6)heterocycle, containing at least one heteroatom of N, S, O; or benzyl, wherein phenyl, (C5 to C6)heterocycle or benzyl may eventually be substituted with one or more groups selected from COOH, NH₂, NO₂, N₃ and SH, preferably the substituent is in *para* position;
and wherein X is independently H; (C1 to C6)alkyl, which may be linear or branched; (C3 to C6)cycloalkyl; phenyl and/or benzyl;
   while for X, Y and Z applies, that (C1 to C6)alkyl, (C3 to C6)cycloalkyl, phenyl and/or benzyl may be independently substituted with one or more groups selected from COOH; NH₂; NO₂,NX₂; C(O)NX₂; NHX; C(O)NHX; SH; tetrazine of the general formula **(III);** (C7 to C8)cycloalkenyl in *trans* configuration, eventually substituted with one or more groups selected from COOH, NH₂, NO₂,-NCS, -NCO, N₃ and SH; norbornenyl, eventually substituted with one or more groups selected from COOH, NH₂, NO₂, -NCS, -NCO, N₃ and SH; -N₃; (C2 to C6)alkynyl, eventually substituted with one or more groups selected from COOH, NH₂, NO₂,-NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂,-NCS, -NCO, N₃ and SH; maleimide; phenyl; benzyl and -P(O)(OH)Z;
wherein at least one of the groups R, R^{a}, R¹, R², R³, R⁴, R^{4a}, R⁵, R^{5a}, R⁶, R^{6a}, R⁷, R⁸, R⁹ a R¹⁰ contains at least one of the groups -COOH; NH₂; NO₂,NX₂, NHX; C(O)NHX; N₃; SH; -NCS; tetrazine of the general formula **(III);** (C7 to C8)cycloalkenyl in *trans* configuration, eventually substituted with one or more groups selected from COOH, NH₂, NO₂,NX₂, -NCS, -NCO, NHX, C(O)NHX, N₃ and SH; norbornenyl, eventually substituted with one or more groups selected from COOH, NH₂, NO₂, NX₂, -NCS, -NCO, NHX, C(O)NHX, N₃ and SH; -N₃; (C2 to C6)alkynyl; cyclooctynyl, eventually substituted with one or more groups selected from COOH, NH₂, NO₂, NX₂, -NCS, -NCO, NHX, C(O)NHX, N₃ and SH; cycloazaoctynyl, eventually substituted with one or more groups selected from COOH, NH₂, NO₂, NX₂, -NCS, -NCO, NHX, C(O)NHX, N₃ and SH; maleimide; phenyl, eventually substituted with one or more groups selected from COOH, NH₂, NO₂, NX₂, -NCS, -NCO, NHX, C(O)NHX, N₃ and SH; benzyl, eventually substituted with one or more groups selected from COOH, NH₂, NO₂, NX₂, -NCS, -NCO, NHX, C(O)NHX, N₃ and SH; (C1 to C6)alkyl, eventually substituted with one or more groups selected from COOH, NH₂, NO₂, NX₂, -NCS, -NCO, NHX, C(O)NHX, N₃ and SH; and -P(O)(OH)Z.

In one embodiment of the invention, R¹ and R³ together form (C2 to C3)alkylene, which may be substituted with (C1 to C6)alkyl, which may be linear or branched.

In another embodiment of the invention, R, R^{a}, R¹, R², R³, R⁴, R^{4a}, R⁵, R^{5a}, R⁶ and R^{6a} are independently selected from the group comprising H; (C1 to C6)alkyl, which may be linear or branched; (C3 to C6) cycloalkyl; benzyl; phenyl; (C5 to C6)heterocycle, containing at least one heteroatom of N, S, O;
and/or R¹ and/or R² and/or R³ is the bis-phosphorus acid of the general formula **(II),** preferably at least one of the groups R¹, R² and R³ is the bis-phosphorus acid of the general formula **(II),** most preferably R² is the bis-phosphorus acid of the general formula **(II).**

In one preferred embodiment, R² is selected from the group comprising H; (C1 to C6)alkyl, which may be linear or branched; (C3 to C6)cycloalkyl; benzyl; phenyl; (C5 to C6)heterocycle, containing at least one heteroatom of N, S, O; and bis-phosphorus acid of the general formula **(II).**

In another preferred embodiment, the cyclam-based compounds according to the present invention are selected from the group comprising compounds of the general formula **(I),** whose the substituents are represented in the following combinations:

| Compound number | R | R¹ | | R³ | R² | R⁴ | R⁵ | R⁶ | R⁷ | R⁸ | R⁹ | R¹⁰ |
|---|---|---|---|---|---|---|---|---|---|---|---|---|
| 1 | H | H | | H | H | CH₃ | H | H | H | H | H | H |
| 2 | H | CH₃ | | CH₃ | CH₃ | CH₃ | H | H | H | H | H | H |
| 3 | H | CH₃ | | CH₃ | H | H | H | H | H | H | H | H |
| 4 | H | CH₃ | | CH₃ | CH₃ | H | H | H | H | H | H | H |
| 5 | OH | benzyl | | benzyl | H | H | H | H | H | H | H | H |
| 7 | OH | H | | benzyl | benzyl | H | H | H | H | H | H | H |
| 8 | *p*-nitrobenzyl | H | | H | H | H | H | H | H | H | H | H |
| 9 | *p*-aminobenzyl | H | | H | H | H | H | H | H | H | H | H |
| 10 | *p*-SCN-benzyl | H | | H | H | H | H | H | H | H | H | H |
| 11 | *p*-azidobenzyl | H | | H | H | H | H | H | H | H | H | H |
| 12 | -CH₂N(Bz)₂ | H | | H | H | H | H | H | H | H | H | H |
| 13 | -CH₂NH₂ | H | | H | H | H | H | H | H | H | H | H |
| 14 | -(CH₂)₂COOH | H | | H | H | H | H | H | H | H | H | H |
| 15 | H | H | | H | H | H | -(CH₂)₄COOH | H | H | H | H | H |
| 16 | H | H | | H | H | H | H | H | CH₃ | NO₂ | NO₂ | CH₃ |
| 22 | H | CH₃ | | CH₃ | acid of formula **(II),** wherein R^{a} = R, R^{4a} to R^{6a} = H | H | H | H | H | H | H | H |
| 23 | -(CH₂)₂COOH | CH₃ | | CH₃ | acid of formula **(II),** wherein R^{a} = R, R^{4a} to R^{6a} = H | H | H | H | H | H | H | H |
| 24 | OH | benzyl | | benzyl | -CH₂P(O)(OH)₂ | H | H | H | H | H | H | H |
| 25 | OH | H | | H | -CH₂P(O)(OH)₂ | H | H | H | H | H | H | H |
| 26 | OH | -CH₂P(O)(OH)₂ | | benzyl | benzyl | H | H | H | H | H | H | H |
| 27 | OH | -CH₂P(O)(OH)₂ | | H | H | H | H | H | H | H | H | H |
| 30 | H | -(CH₂)₂- | | | H | CH₃ | H | H | H | H | H | H |
| 31 | *p*-nitrobenzyl | -(CH₂)₂- | | | H | H | H | H | H | H | H | H |
| 32 | H | -(CH₂)₂- | | | acid of formula **(II),** wherein R^{a} = R, R^{4a} to R^{6a} = H | H | H | H | H | H | H | H |
| 33 | -CH₂OH | -(CH₂)₂- | | | acid of formula **(II),** wherein R^{a} = R, R^{4a} to R^{6a} = H | H | H | H | H | H | H | H |
| 34 | OH | -(CH₂)₂- | | | acid of formula **(II),** wherein R^{a} = R, R^{4a} to R^{6a} = H | H | H | H | H | H | H | H |
| 35 | -(CH₂)₂COOH | -(CH₂)₂- | | | acid of formula **(II),** wherein R^{a} = R, R^{4a} to R^{6a} = H | H | H | H | H | H | H | H |
| 36 | H | -(CH₂)₂- | | | acid of formula (II), wherein R^{a} = OH, R^{4a} to R^{6a} = H | H | H | H | H | H | H | H |
| 37 | *p*-nitrobenzyl | -(CH₂)₂- | | | acid of formula **(II),** wherein R^{a} = OH, R^{4a} to R^{6a} = H | H | H | H | H | H | H | H |
| 38 | *p*-aminobenzyl | -(CH₂)₂- | | | acid of formula **(II),** wherein R^{a} = OH, R^{4a} to R^{6a} = H | H | H | H | H | H | H | H |
| 39 | *p*-SCN-benzyl | -(CH₂)₂- | | | acid of formula **(II),** wherein R^{a} = OH, R^{4a} to R^{6a} = H | H | H | H | H | H | H | H |
| 40 | *p*-azidobenzyl | -(CH₂)₂- | | | acid of formula **(II),** wherein R^{a} = OH, R^{4a} to R^{6a} = H | H | H | H | H | H | H | H |
| 41 | *p*-nitrobenzyl | -(CH₂)₂- | | | -CH₂P(O)(OH)(CH₂OH) | H | H | H | H | H | H | H |
| 42 | *p*-nitrobenzyl | -(CH₂)₂- | | | -CH₂P(O)(OH)₂ | H | H | H | H | H | H | H |
| 43 | *p*-aminobenzyl | -(CH₂)₂- | | | -CH₂P(O)(OH)₂ | H | H | H | H | H | H | H |
| 50 | H | -(CH₂)₂- | | | H | H | -(CH₂)₄CH₃ | H | H | H | H | H |
| 51 | H | -(CH₂)₂- | | | 2-picolyl-*N*-oxide | H | -(CH₂)₄CH₃ | H | H | H | H | H |
| 52 | H | -(CH₂)₂- | | | -CH₂COOH | H | H | H | H | H | H | H |
| 53 | OH | -(CH₂)₂- | | | -CH₂P(O)(OH)-(CH₂)₂COOH | H | H | H | H | H | H | H |
| 54 | H | -CH₃ | -CH₃ | | -CH₂P(O)(OH)Ph | H | H | H | H | H | H | H |
| 55 | *p*-nitrobenzyl | -(CH₂)₂- | | | -CH₂P(O)(OH)-CH₂N(2-picolyl)₂ | H | H | H | H | H | H | H |
| 56 | -(CH₂)₂COOH | H | H | | H | -(CH₂)₃N₃ | H | H | H | H | H | H |
| 57 | *p*-nitrobenzyl | -(CH₂)₂- | | | -(CH₂)₂S-(dimer) | H | H | H | H | H | H | H |
| 58 | H | H | | H | H | H | H | H | -CH₃ | -NH₂ | -NH₂ | -CH₃ |
| 59 | *p*-nitrobenzyl | H | | H | H | H | H | H | -COOH | H | H | H |
| 60 | *p*-nitrobenzyl | H | | H | H | H | H | H | -(CH₂)₂OH | H | H | H |
| 61 | -(CH₂)₂COOH | H | | H | H | H | H | H | *2*-pyridyl | H | *2*-pyridyl | H |

The subject of the present invention is also a conjugate of the cyclam-based compound according to the present invention and of at least one conjugation group, which is covalently bound to the cyclam-based compound, and which is selected from the group containing OH, -COOH; NH₂; NO₂, NX₂, NHX; C(O)NHX; N₃; SH; -NCS; tetrazine of the general formula **(III);** (C7 to C8)cycloalkenyl in *trans* configuration, eventually substituted with one or more groups selected from COOH, NH₂, NO₂, NX₂, -NCS, -NCO, NHX; C(O)NHX; N₃ and SH; norbornenyl, eventually substituted with one or more groups selected from COOH, NH₂, NO₂, NX₂, -NCS, -NCO, NHX; C(O)NHX; N₃ and SH; -N₃; (C2 to C6)alkynyl; cyclooctynyl, eventually substituted with one or more groups selected from COOH, NH₂, NO₂, NX₂, -NCS, -NCO, NHX; C(O)NHX; N₃ and SH; cycloazaoctynyl, eventually substituted with one or more groups selected from COOH, NH₂, NO₂, NX₂, -NCS, -NCO, NHX, C(O)NHX, (C1 to C6)alkyl, (C6)aryl, N₃ and SH; maleimide; and -P(O)(OH)Z; wherein the conjugate may further contain a spacer between the cyclam-based compound and the conjugation group and/or between two conjugation groups, the spacer selected from the group containing (C1 to C6)*n*-alkyl, eventually substituted with C=O and/or -NH- group; phenylene; amino-acids chains with the length of 1 to 5 amino-acids; polyethylene glycols of 1 to 10 monomeric units.

The subject of the present invention are also coordination compounds of the cyclam based compounds of the general formula (I) or the conjugates according to the present invention, with metal cations selected from the group consisting of Cu²⁺, Bi³⁺, lanthanide cations, Sc³⁺, Y³⁺, Pb²⁺, Zr⁴⁺, Ac³⁺, Mn²⁺ and Mn³⁺, preferably lanthanide cations, Bi³⁺, Y³⁺ a Cu²⁺, more preferably Lu³⁺, Bi³⁺, Y³⁺ and Cu²⁺. Metal cations may preferably be radioisotopes of metals, especially radioisotopes of copper, lutetium, bismuth, and yttrium.

The subject of the present invention is also a targeting conjugate, which contains the cyclam based compound of the general formula (I) and/or the conjugate and/or the coordination compound according to the present invention, and a targeting vector, selected from the group comprising bis(phosphonates); groups capable of fluorescence, for example fluoresceines, rhodamines or the boron-dipyrromethene (BODIPY) type substances, preferably emitting in the red or near infra-red (NIR) part of the spectrum, most preferably fluorescein and rhodamine; oligopeptides of 1 to 15 aminoacids; antibodies or fragments thereof, especially the Fab antigen binding fragments; folic acid; biotin; compounds targeting to PSMA receptor, preferably urea derivatives or organo-phosphorous compounds; cyclodextrins; dendrimers, preferably PAMAM dendrimers; hydrophilic polymers on the basis of derivatives of acrylic acid, especially hydroxoalkylamides of acrylic acid, preferably hydroxoalkylamides of acrylic acid modified with amino groups and/or maleimido groups, most preferably the block copolymer of poly[*N*-(2-hydroxopropyl)methacrylamide and poly-L-lysine, poly[*N*-(2-hydroxopropyl)methacrylamide with free amino groups and poly[*N*-(2-hydroxopropyl)methacrylamide with free maleimide groups. Cyclodextrins, dendrimers and hydrophilic polymers based on derivatives of the acrylic acid are preferably modified with functional groups allowing for further conjugations of targeting vectors, serving thus for, not limited to, to increase the number of molecules of the compound of the general formula (I) in the targeting conjugate. Targeting conjugates containing Fab fragments of antibodies have, compared to the whole antibodies, faster pharmacokinetics in the organism, therefore, they are pharmacokinetically more preferred comparing to antibodies. Targeting conjugates containing groups capable of fluorescence are suitable especially for targeted image-guided surgery (IGS).

The subject of the present invention is also a method of preparation of cyclam based compounds of the general formula **(I)** according to the present invention, wherein the intermediate product of the general formula (IV), 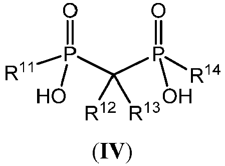 wherein R¹¹, R¹², R¹³ and R¹⁴ are in the following combinations:

| Intermediate | R¹¹ | R¹² | R¹³ | R¹⁴ |
|---|---|---|---|---|
| A | H | H | H | OH |
| B | H | H | H | *p*-nitrobenzyl |
| C | H | H | H | -CH₂N(benzyl)₂ |
| D | H | H | H | -(CH₂)₂COOH |
| E | H | -(CH₂)₄COOH | H | H |
| F | OH | -(CH₂)₃NH(4-dibenzylcyclooktyn amidobutoxyl) | OH | OH |

or a compound selected from the group containing methylene-bis(phosphinic acid), phosphorous acid, hydroxymethylphosphinic acid, reacts with a cyclam derivative and aldehyde in aqueous solution of an acid with the concentration within the range of from 10 to 40 % (weight), preferably from 18 to 36 % (weight), at the temperature in the range of from 40 °C to 80 °C for the period of time of at least 12 hours;
wherein the cyclam derivative is 1,4,8,11-tetraazacyclotetradecane and derivatives thereof and 1,4,8,11-tetraazabicyclo[6.6.2]hexadecane and derivatives thereof, preferably the cyclam derivative is selected from the group containing 1,4,8,11-tetraazacyclotetradecane, 1,4,8,11-tetraazabicyclo[6.6.2]hexadecane, 1,4,8-trimethyl-1,4,8,11-tetraazacyclotetradecane, 1,8-dimethyl-1,4,8,11-tetraazacyclotetradecane, 1,8-dibenzyl-1,4,8,11-tetraazacyclotetradecane, 1,4-dibenzyl-1,4,8,11-tetraazacyclotetradecane, 6,13-dimethyl-6,13-dinitro-1,4,8,11-tetraazacyclotetradecane; the aldehyde is selected from the group containing acetaldehyde, formaldehyde and paraformaldehyde, and the acid is preferably HCl and/or CF₃COOH; and wherein the reaction may be preceded by the step of preparation of the intermediate product.

In one embodiment of the method of preparation of the cyclam based compounds of the general formula **(I)** according to the present invention, the resulting product is further subjected to a reduction reaction. Preferably, the reduction reaction is performed as a hydrogenation in the presence of catalysts, reduction with hydride complexes, reduction with metals in acid solutions or using sulfides.

In another embodiment of the method of preparation of the cyclam based compounds of the general formula (I) according to the present invention, the resulting product is further subjected to an oxidation reaction. Preferably, the oxidation reaction is performed with oxidation agents selected from the group containing Cl₂, I₂ or Br₂ in water or in organic solvents, oxygen-containing oxidation agents, such as hydrogen peroxide or (organic) peroxoacids or salts thereof, metal ions in oxidation states capable of reduction, such as Hg²⁺ or Cu²⁺, or (organic) oxoacids of iodine in high formal oxidation degrees of +III or +V.

In another embodiment of method of preparation of cyclam based compounds of the general formula **(I)** according to the present invention, the resulting product is further subjected to reaction with an aldehyde; and eventually with an intermediate product of the general formula (IV), or with a compound, selected from the group containing methylene-bis(phosphinic acid), phosphorous acid, alkylphosphinic acid or arylphosphinic acid, preferably hydroxymethylphosphinic acid; in aqueous solution of an acid, preferably HCl or CF₃COOH.

In another embodiment of the method of preparation of cyclam based compounds of the general formula **(I)** according to the present invention, the reduction step is followed by a step when the product of the reduction undergoes a reaction with CSCl₂ or COCl₂.

In another embodiment of the method of preparation of cyclam based compounds of the general formula **(I)** according to the present invention, the reduction step is followed by a step when the resulting product of the reduction undergoes a reaction with NaN₃.

The subject of the present invention is also an intermediate product of the general formula **(IV),** 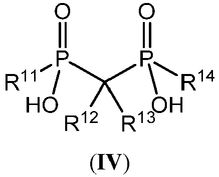 wherein R¹¹ , R¹² , R¹³ and R¹⁴ are in the following combinations:

| Intermediate | R¹¹ | R¹² | R¹³ | R¹⁴ |
|---|---|---|---|---|
| B | H | H | H | *p*-nitrobenzyl |
| C | H | H | H | -CH₂N(benzyl)₂ |
| D | H | H | H | -(CH₂)₂COOH |
| E | H | -(CH₂)₄COOH | H | H |
| F | OH | -(CH₂)₃NH(4-dibenzylcyclooktyn amidobutoxyl) | OH | OH |

The subject of the present invention is also a pharmaceutical preparation, which contains at least one cyclam based compound according to the present invention and/or at least one conjugate according to the present invention and/or at least one coordination compound according to the present invention and/or at least one targeting conjugate according to the present invention, and a pharmaceutically acceptable substance. The dosage form of the pharmaceutical preparation is a form for administration by injection, most often as a bolus or as an infusion, preferably intravenously. Suitable pharmaceutically acceptable auxiliary substances are preferably selected from the group containing solvents (especially aqueous or saline solution), buffers (especially phosphate buffer, HEPES = 2-[4-(2-hydroxyethyl)piperazine-1-yl]ethanesulfonic acid), ionization additives, antioxidants, antimicrobial additives. A person skilled in the art would be able, without exerting inventive activity, to determine which adjuvans to choose.

Subject of the present invention is also the cyclam based compound according to the present invention and/or at least one conjugate according to the present invention and/or at least one coordination compound according to the present invention and/or at least one targeting conjugate according to the present invention and/or the pharmaceutical preparation according to the present invention, for use as a medicament, preferably in the treatment of tumor diseases, inflammations, and/or atherosclerosis. Especially coordination compounds with metal radioisotopes according to the present invention, preferably with copper, lutetium, bismuth and yttrium radioisotopes, are suitable for the use as medicaments in radiotherapy. Cyclam based compounds according to the present invention were successfully tested *in vivo* in rodents. It results from the tests performed that those substances are neither deposited nor decomposed anywhere in the body and they are excreted from the body in a normal manner (urine, faeces). Therefore, they are suitable for medical applications.

The subject of the present invention is also the cyclam based compound according to any of the claims 1 thru 5 and/or at least one conjugate according to the present invention and/or at least one coordination compound according to the present invention and/or at least one targeting conjugate according to the present invention and/or the pharmaceutical preparation according to the present invention, for use as a contrast agent in medical diagnostics, preferably in the diagnostics of tumor diseases, inflammations, and/or atherosclerosis. For luminescence methods in medical diagnostics, coordination compounds according to the present invention containing cations of trivalent lanthanides are especially suitable; for magnetic nuclear resonance (NMR, MRI), coordination compounds of gadolinium are suitable.

The subject of the present invention is the use of cyclam based compounds according to the present invention and/or of the conjugate according to the present invention and/or of the coordination compound according to the present invention and/or of the targeting conjugate according to the present invention and/or of the pharmaceutical preparation according to the present invention, in radiochemistry as markers or precursors of markers when labelling with the use of radioisotopes of copper, lutetium, bismuth and yttrium, preferably using radioisotopes of copper, more preferably ⁶⁴Cu.

The subject of the present invention is the use of cyclam based compounds according to the present invention and/or of the conjugate according to the present invention and/or of the coordination compound according to the present invention and/or of the targeting conjugate according to the present invention, for the complexation and/or purification of copper radioisotopes.

### Examples

### Example 1: Synthesis of compound 1

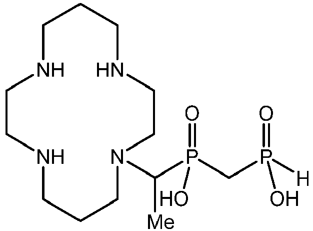

To a glass vial (4 mL), 1,4,8,11-tetraazacyclotetradecane (205 mg; 1.02 mmol; 5.0 equiv.) was added. Methylene-bis(phosphinic acid) (85.6 mg; 594 µmol; 2.9 equiv.), acetaldehyde (9.0 mg; 205 µmol; 1.0 equiv.) and aqueous HCl (6 M; 2 mL) were subsequently added and the resulting suspension was stirred at 80 °C overnight. The mixture was evaporated to dryness and several times co-evaporated with H₂O. Crude product was purified by ion exchange chromatography (DOWEX 50; H⁺-form; H₂O → 10% aqueous pyridine). The pyridine fraction with product was evaporated to dryness and further co-evaporated with H₂O (25 mL). The residue was re-dissolved in H₂O (25 mL) and subsequently lyophilized. Product was obtained as white substance (35.0 mg; 40%; 1 step, based on acetaldehyde).
**NMR** (D₂O): **¹H** *δ* 1.16 (C*H*₃, dd, 3H, ³*J*_{HP} = 15 Hz, ²*J*_{HH} = 7 Hz); 1.73-2.00 (P-CH₂-P, CH₂-CH₂-CH₂, m, 5H); 2.08 (P-C*H*₂-P, dddd, 1H, ²*J*_{HH} = 19 Hz, ²*J*_{HP} = 16 Hz, ²*J*_{HP} = 12 Hz, ³*J*_{HH} = 4 Hz); 2.52-3.24 (CH₂-N, m, 16H); 3.37 (CH-CH₃, dq, 1H, ²*J*_{HP} = 14 Hz, ²*J*_{HH} = 7 Hz); 7.12 (P*H*, dd, ¹*J*_{HP} = 534 Hz, ³*J*_{HH} = 4 Hz); ¹³C{¹H} *δ* 6.3 (*C*H₃, bm); 23.9 (CH₂-*C*H₂-CH₂, s); 26.2 (CH₂-*C*H₂-CH₂, s); 34.2 (P-*C*H₂-P, dd, *¹J*_{CP} = 78 Hz, *¹J*_{CP} = 73 Hz); 44.8 (*cycle,* s); 46.4 *(cycle,* s); 46.5 *(cycle,* s); 46.7 *(cycle,* s); 49.4 *(cycle,* s); 49.6 *(cycle,* d, ³*J_{CP}* = 10 Hz); 50.0 *(cycle,* s); 50.6 *(cycle,* s); 52.0 (N-*C*H-P, d, *¹J*_{CP} = 109 Hz); ³¹**P** *δ* 19.1 (P*H*, dtd, 1P, ¹*J*_{PH} = 534 Hz, ²*J*_{PH} = 16 Hz, ²*J*_{PP} = 4 Hz); 35.5 (N-CH-*P*, m, 1P).
**MS:** (-) 368.8 [M-H⁺]⁻. (+) 371.0 [M+H⁺]⁺; 392.9 [M+Na⁺]⁺; 408.9 [M+K⁺]⁺.
**TLC** (SiO₂, *i*-PrOH-conc. NH₄OH-H₂O 7:3:3): *R*_{f =} 0.7.
**EA** (C₁₃H₃₂N₄O₄P_{2˙}3_{·}5H₂O, *M*_{R} = 433.4): C 36.0 (35.6); H 9.1 (8.9); N 12.9 (13.2).

### Example 2: Synthesis of compound 2

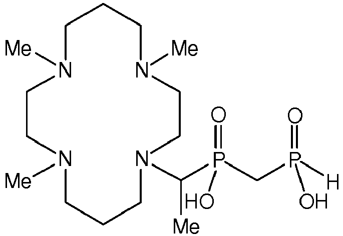

To a glass vial (4 mL), tetrahydrochloride of 1,4,8-trimethyl-1,4,8,11-tetraazacyclotetradecane (65.2 mg; 168 µmol; 1.0 equiv.) was added. Methylene-bis(phosphinic acid) (73.2 mg; 508 µmol; 3.0 equiv.), acetaldehyde (8.1 mg; 184 µmol; 1.1 equiv.) and aqueous HCl (6 M; 2 mL) were subsequently added and the resulting suspension was stirred at 60 °C for two days. The mixture was evaporated to dryness and several times co-evaporated with H₂O. Crude product was purified by ion exchange chromatography (DOWEX 50; H⁺-form; H₂O → 10% aqueous pyridine). The pyridine fraction with product was evaporated to dryness and further co-evaporated with H₂O (25 mL). The residue was re-dissolved in H₂O (55 mL) and subsequently lyophilized. Product was obtained as a white substance (48.7 mg; 63%; 1 step, based on acetaldehyde). **NMR** (D₂O): **¹H** *δ* 1.19 (C*H*₃*-*C*,* dd, 3H, ³*J*_{HP} = 16 Hz, ²*J*_{HH} = 7 Hz); 1.68 (CH₂-CH₂-CH₂, m, 4H); 1.81 (P-C*H*₂-P, m, 1H); 1.98 (P-C*H*₂-P, m, 1H); 2.25 (C*H*₃-N, m, 3H); 2.27 (C*H*₃-N, s, 6H); 2.48-3.16 (C*H*₂-N, m, 16H); 3.41 (C*H-*CH₃, dq, 1H, ²*J*_{HP} = 15 Hz, ²*J*_{HH} = 7 Hz); 7.13 (P*H*, dm, *¹J_{HP}* = 533 Hz); ¹³C{¹H} *δ* 7.1 (*C*H₃-C, s); 21.4 (CH₂-*C*H₂-CH₂, s); 22.7 (CH₂—*C*H₂—CH₂, s); 35.2 (P-*C*H₂-P, dd, *¹J*_{CP} = 77 Hz, ¹*J*_{CP} = 73 Hz); 43.6 (*C*H₃-N, s); 43.7 (*C*H₃-N, s); 43.9 (*C*H₃-N, s); 46.2 *(cycle,* s); 48.2 *(cycle,* s); 50.0 *(cycle,* s); 50.8 *(cycle,* d, ³*J_{CP}* = 8 Hz); 51.3 *(cycle,* d, ³*J*_{CP} = 9 Hz); 51.9 *(cycle,* s); 53.5 *(cycle,* s); 53.6 *(cycle,* s); 56.5 (N-*C*H₂-P, d, *¹J*_{CP} = 109 Hz); **³¹P** *δ* 19.7 (PH, dtd, 1P, ¹*J*_{PH} = 533 Hz, ²*J*_{PH} = 16 Hz, ²*J*_{PP} = 3 Hz); 32.1 (N-CH-*P*, m, 1P).
**MS:** (-) 410.9 [M-H⁺]⁻. (+) 413.1 [M+H⁺]⁺; 435.1 [M+Na⁺]⁺; 451.0 [M+K⁺]⁺.
**TLC** (SiO₂, EtOH-conc. NH₄OH 4:1): *R*_{f =} 0.3.
**EA** (C₁₆H₃₈N₄O₄P₂·2.5H₂O, *M*_{R} = 457.4): C 42.0 (42.3); H 9.5 (9.1); N 12.3 (12.2).

### Example 3: Synthesis of compound 3

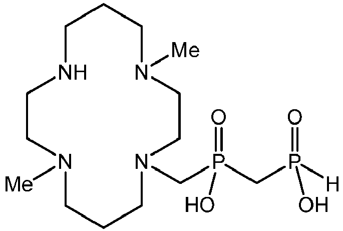

To a glass flask (25 ml), tetrahydrochloride of 1,8-dimethyl-1,4,8,11-tetraazacyclotetradecane (1.56 mg; 4.17 mmol; 2.5 equiv.) was added. Methylene-bis(phosphinic acid) (480 mg; 3.33 mmol; 2.0 equiv.), paraformaldehyde (50 mg; 1.67 mmol; 1.0 equiv.) and aqueous HCl (6 M; 5 mL) were subsequently added and the resulting suspension was stirred at 80 °C overnight. The mixture was evaporated to dryness and several times co-evaporated with H₂O. Crude product was purified by ion exchange chromatography (DOWEX 50; H⁺-form; H₂O → 10% aqueous pyridine). The pyridine fraction with product was evaporated to dryness and further co-evaporated several times with H₂O. The crude product was further purified by column chromatography (SiO₂; EtOH-conc. NH₄OH 4:1; *R*_{f} = 0.4). Fractions with product were combined and evaporated to dryness. The residue was suspended in H₂O (25 mL), treated with small amounts of charcoal and filtered through syringe microfilter (Millipore; 0.22 µm). The filtrate was re-purified by ion exchange chromatography (DOWEX 50; H⁺- form; H₂O → 10% aqueous pyridine). The pyridine fraction with product was evaporated to dryness and further co-evaporated several times with H₂O. The residue was re-dissolved in H₂O (25 mL) and subsequently lyophilized. Product was obtained as a white substance (215 mg; 29%; 1 step, based on paraformaldehyde).
**NMR** (D₂O): **¹H** *δ* 1.70 (CH₂-*C*H₂-CH₂, p, 2H, ³*J*_{HH} = 7 Hz); 1.76 (CH₂-C*H*₂-CH₂, p, 2H, ³*J*_{HH} = 7 Hz); 2.03 (P-C*H*₂-P, ddd, 2H, ²*J*_{HP} = 18 Hz, ²*J*_{HP} = 16 Hz, ³*J*_{HH} = 2 Hz); 2.24 (C*H*₃, s, 3H); 2.30 (C*H*₃, s, 3H); 2.50 *(cycle,* t, 2H, ³*J*_{HH} = 7 Hz); *2.57* (*cycle,* m, 4H); 2.66 (*cycle,* t, 2H, ³*J*_{HH} = 7 Hz); 2.69-2.82 (*cycle,* N*-*C*H*₂*-*P*,* m, 10H); 7.14 (PH, dt, ¹*J*_{HP} = 531 Hz, ³*J*_{HH} = 2 Hz); ¹³C{¹H} *δ* 23.0 (CH₂-*C*H₂-CH₂, s); 23.6 (CH₂-*C*H₂-CH₂, s); 35.9 (P-*C*H₂-P, dd, *¹J*_{CP} = 77 Hz, *¹J*_{CP} = 75 Hz); 43.5 (*cycle,* s); 43.2 (*C*H₃, s); 43.8 (*C*H₃, s); 44.8 (*cycle,* s); 51.1 (*cycle,* d, *³J_{CP}* = 8 Hz); 52.2 (*cycle,* s); 53.1 (*cycle,* s); 53.4 (*cycle,* s); 53.5 (*cycle,* d, ³*J*_{CP} = 6 Hz); 53.7 (*cycle,* s); 56.1 (N-*C*H₂-P, d, *¹J*_{CP} = 109 Hz); **³¹P** *δ* 19.8 (P*H*, dtd, 1P, ¹*J*_{PH} = 531 Hz, ²*J*_{PH} = 18 Hz, ²*J*_{PP} = 3 Hz); 32.5 (N-CH-*P*, m, 1P).
**MS:** (-) 382.6 [M-H⁺]⁻. (+) 384.6 [M+H⁺]⁺; 406.8 [M+Na⁺]⁺.
**TLC** (SiO₂, EtOH-conc. NH₄OH 4:1): *R*_{f} = 0.4.
**EA** (C₁₄H₃₄N₄O₄P_{2˙}3.5H₂O, *M*_{R} = 447.5): C 37.6 (37.5); H 9.2 (9.0); N 12.5 (12.4).

### Example 4: Synthesis of compound 4

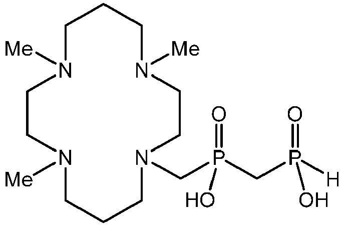

To a glass vial (4 mL), tetrahydrochloride of 1,4,8-trimethyl-1,4,8,11-tetraazacyclotetradecane (37.8 mg; 99.7 µmol; 1.0 equiv.) was added. Methylene-bis(phosphinic acid) (55.6 mg; 386 µmol; 3.9 equiv.), paraformaldehyde (5.6 mg; 210 µmol; 2.1 equiv.) and aqueous HCl (6 M; 2 mL) were subsequently added and the resulting suspension was stirred at 60 °C for two days. The mixture was evaporated to dryness and several times co-evaporated with H₂O. The crude product was purified by ion exchange chromatography (DOWEX 50; H⁺-form; H₂O → 10% aqueous pyridine). The pyridine fraction with product was evaporated to dryness and further co-evaporated with H₂O (25 mL). The residue was re-dissolved in H₂O (25 mL) and subsequently lyophilized. Product was obtained as a white substance (35.4 mg; 82%; 1 step, based on paraformaldehyde).
**NMR** (D₂O): **¹H** *δ* 1.71 (CH₂-C*H*₂-CH₂, m, 4H); 2.04 (P-C*H*₂-P, ddd, 2H, *²J*_{HP} = 18 Hz, ²*J*_{HP} = 16 Hz, ³*J*_{HH} = 2 Hz); 2.24 (C*H*₃, m, 6H); 2.27 (C*H*₃, s, 3H); 2.47-2.64 *(cycle,* m, 12H); 2.75 *(cycle,* m, 2H); 2.81 (N-C*H*₂-P, d, 2H, ³*J*_{HH} = 9 Hz); 2.84 *(cycle,* m, 2H); 7.14 (PH, dt, ¹*J*_{HP} = 531 Hz, ³*J*_{HH} = 2 Hz); ¹³C{¹H} *δ* 20.2 (CH₂-*C*H₂-CH₂, s); 20.6 (CH₂-*C*H₂-CH₂, s); 35.4 (P-*C*H₂-P, t, *¹J*_{CP} = 77 Hz); 43.5 (*C*H₃, s); 43.7 (*C*H₃, s); 43.8 (*C*H₃, s); 49.0 *(cycle,* s); 49.2 *(cycle,* s); 49.6 *(cycle,* s); 50.0 *(cycle,* d, ³*J_{CP}* = 8 Hz); 52.1 *(cycle,* d, ³*J_{CP}* = 8 Hz); 52.2 *(cycle,* s); 53.4 *(cycle,* s); 53.5 *(cycle,* s); 56.7 (N-*C*H₂-P, d, *¹J*_{CP} = 109 Hz); **³¹P** *δ* 19.7 (P*H*, dtd, 1P, ¹*J*_{PH} = 531 Hz, ²*J*_{PH} = 18 Hz, ²*J*_{PP} = 3 Hz); 32.1 (N-CH-*P*, m, 1P).
**MS:** (-) 396.8 [M-H⁺]⁻. (+) 399.0 [M+H⁺]⁺; 421.0 [M+Na⁺]⁺; 437.0 [M+K⁺]⁺.
**TLC** (SiO₂, EtOH-conc. NH₄OH 4:1): *R*_{f} = 0.2.
**EA** (C₁₄H₃₄N₄O₄P_{2˙}2H₂O, *M*_{R} = 434.5): C 41.5 (41.8); H 9.3 (9.1); N 12.9 (12.9).

### Example 5: Synthesis of compound 5

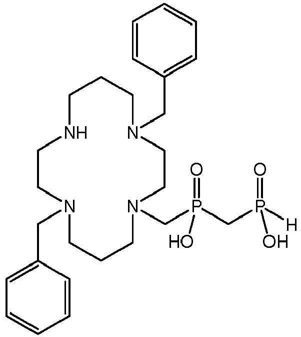

To a glass vial (20 mL), tetrahydrochloride of 1,8-dibenzyl-1,4,8,11-tetraazacyclotetradecane (506 mg; 959 µmol; 2.0 equiv.) was added. Methylene-bis(phosphinic acid) (136 mg; 944 µmol; 2.0 equiv.). CF₃COOH (5 mL), aqueous HCl (12 M; 5 mL) and paraformaldehyde (20.9 mg; 474 µmol; 1.0 equiv.) were subsequently added and the resulting suspension was stirred at 80 °C for two days. The mixture was evaporated to dryness and several times co-evaporated with H₂O. The crude product was further purified by column chromatography (SiO₂; EtOH-conc.aq. NH₄OH 5:1; *R*_{f} = 0.6). The fractions with product were combined and evaporated to dryness. The residue was suspended in H₂O (25 mL), treated with small amounts of charcoal and filtered through syringe microfilter (Millipore; 0.22 µm). The filtrate was re-purified by ion exchange chromatography (DOWEX 50; H⁺-form; H₂O → 10% aqueous pyridine). The pyridine fraction with product was evaporated to dryness and further co-evaporated several times with H₂O. The residue was re-dissolved in H₂O (100 mL) and subsequently lyophilized. Product was obtained as a white substance (146 mg; 54%; 1 step, based on paraformaldehyde).
**NMR** (D₂O): *δ* 1.82 (CH₂-C*H*₂-CH₂, m, 4H); 2.05 (P-C*H*₂-P, dd, 2H, ²*J*_{HP} = 18 Hz, ²*J*_{HP} = 15 Hz); 2.46-2.61 *(cycle,* m, 6H); 2.74-3.12 (*cycle,* N*-*C*H*₂*-*P*,* m, 10H); 3.68 (C*H*₂-Ph, s, 2H); 3.75 (C*H*₂-Ph, s, 2H); 7.14 (PH, d, ¹*J*_{HP} = 529 Hz); 7.22-7.35 *(Ph,* m, 10H); ¹³C{¹H} *δ* 24.1 (CH₂-*C*H₂-CH₂, s); 27.2 (CH₂-*C*H₂-CH₂, s); 37.1 (P-*C*H₂-P, dd, ¹*J*_{CP} = 79 Hz, *¹J*_{CP} = 71 Hz); 41.6 *(cycle,* s); 42.2 *(cycle,* s); 50.1 *(cycle,* d, ³*J*_{CP} = 7 Hz); 52.2 *(cycle,* s); 53.3 *(cycle,* s); 53.4 *(cycle,* s); 53.8 *(cycle,* d, ³*J*_{CP} = 9 Hz); 54,2 *(cycle,* s); 56.2 (*C*H₂-Ph, s); 58.8 (N-*C*H₂-P, d, ¹*J*_{CP} = 105 Hz); 59.1 (*C*H₂-Ph, s); 124.1 *(Ph,* s); 126.4 *(Ph,* s); 128.1 *(Ph,* s); 128.3 *(Ph,* s); 128.9 *(Ph,* s); 130.8 *(Ph,* s); 138.1 *(Ph,* s); 139.1 *(Ph,* s); **³¹P** *δ* 26.4 (P*H*, dm, 1P, ¹*J*_{PH} = 529 Hz); 34.3 (N-CH-*P*, m, 1P).
**MS:** (-) 535.1 [M-H⁺]⁻ . (+) 537.1 [M+H⁺]⁺; 559.2 [M+Na⁺]⁺; 581.4 [M-H⁺+2Na⁺]⁺. **TLC** (SiO₂, EtOH-conc. NH₄OH 5:1): *R*_{f} = 0.6.
**EA** (C₂₆H₄₂N₄O₄P₂·2H₂O, *M*_{R} = 572.6): C 54.5 (54.7); H 8.1 (7.7); N 9.8 (10.0).

### Example 6: Synthesis of compound 6

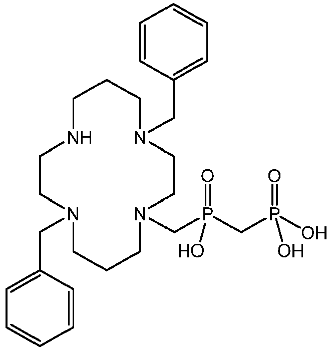

In a glass flask (50 mL), 5·2H₂O (103 mg; 180 µmol; 1.0 equiv.) was dissolved in aqueous HCl (1%; 10 mL). Solution of HgCl₂ (83.1 mg; 306 µmol; 1.7 equiv.) in H₂O (10 mL) was added and the mixture was stirred at 60 °C overnight. Precipitate was filtered off and the mother liquour was saturated with H₂S. Precipitate was filtered off, washed with H₂O and the filtrate was evaporated to dryness. The residue was further dried on vaccum pump and subsequently in vaccum dessicator over P₂O₅. Product was obtained as a white substance (106 mg; 84%; 1 step, based on 5·2H₂O). **NMR** (D₂O): **¹H** *δ* 1.76 (CH₂-C*H*₂-CH₂, p, 2H, ³*J*_{HH} = 4 Hz); 1.88 (CH₂-C*H*₂-CH₂, m, 2H); 2.03 (P-C*H*₂-P, dd, 2H, *²J*_{HP} = 18 Hz, *²J*_{HP} = 14 Hz); 2.44 *(cycle,* m, 2H); 2.51-2.63 *(cycle,* m, 4H); 2.69-3.30 (*cycle,* N*-*C*H*₂*-*P*,* m, 10H); 3.72 (C*H*₂-Ph, s, 2H); 3.74 (C*H*₂-Ph, s, 2H); 7.16 *(Ph,* m, 2H); 7.25-7.34 *(Ph,* m, 8H); ¹³C{¹H} *δ* 24.9 (CH₂-*C*H₂-CH₂, s); 26.9 (CH₂-*C*H₂-CH₂, s); 36.6 (P-*C*H₂-P, dd, *¹J*_{CP} = 114 Hz, *¹J*_{CP} = 70 Hz); 40.8 *(cycle,* s); 42.2 *(cycle,* s); 50.3 *(cycle,* d, ³*J*_{CP} = 7 Hz); 52.8 *(cycle,* s); 53.5 *(cycle,* s); 53.6 *(cycle,* s); 53.8 *(cycle,* d, ³*J*_{CP} = 9 Hz); 54.8 *(cycle,* s); 55.3 (*C*H₂-Ph, s); 58.8 (*C*H₂-Ph, s); 60.1 (N-*C*H₂-P, d, ¹*J*_{CP} = 106 Hz); 125.2 *(Ph,* s); 125.9 *(Ph,* s); 128.1 *(Ph,* s); 128.3 *(Ph,* s); 130.0 *(Ph,* s); 130.2 *(Ph,* s); 138.5 *(Ph,* s); 140.3 (*Ph,* s);³¹P{¹H} *δ* 21.4 (HO-*P*-OH, d, 1P, ²*J*_{PP} = 9 Hz); 33.3 (N-CH*-P,* d, 1P, ²*J*_{PP} = 9 Hz).
**MS:** (-) 551.1 [M-H⁺]⁻.
**TLC** (SiO₂, EtOH-conc. NH₄OH 5:1): *R*_{f} = 0.2.
**EA** (C₂₆H₄₂N₄O₅P₂·3.5HCl·H₂O, *M*_{R} = 698.2): C 44.7 (45.0); H 6.9 (6.7); N 8.0 (8.1).

### Example 7: Synthesis of intermediate product A

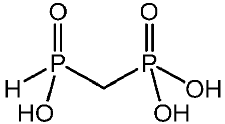

In a glass flask, methylene-bis(phosphinic acid) (2.50 g; 17.4 mmol; 1.0 equiv.) was dissolved in H₂O (50 mL). Solution of HgCl₂ (5.90 g; 21.7 mmol; 1.2 equiv.) in H₂O (50 mL) was added and the mixture was stirred at 60 °C for three days. Precipitate was filtered off, washed with H₂O and the mother liquour was saturated with H₂S. Precipitate was filtered off, washed with H₂O and the filtrate was evaporat to dryness. The crude product was further purified by column chromatography (SiO₂; *i*PrOH-conc. NH₄OH-H₂O 7:3:3; *R*_{f} = 0.2). The fractions with product were combined and evaporated to dryness. The residue was suspended in H₂O (25 mL), treated with small amounts of charcoal and filtered through syringe microfilter (Millipore; 0.22 µm). The filtrate was re-purified by ion exchange chromatography (DOWEX 50; H⁺-form; H₂O). Aqueous fraction with product was evaporated to dryness and the residue was further dried on vaccum pump and subsequently in vaccum dessicator over P₂O₅. Product was obtained as a white substance (1.24 g; 42%; 1 step, based on methylene-bis(phosphinic acid).
**NMR** (D₂O): ¹**H** *δ* 1.93 (C*H*₂-P, tm, 2H, ²*J*_{HP} = 18 Hz); 7.10 (PH, dm, 1H, ¹*J*_{HP} = 526 Hz); ¹³C{¹H} *δ* 34.6 (CH₂, dd, ¹*J*_{CP} = 114 Hz, ¹*J*_{CP} = 78 Hz); ³¹**P** *δ* 12.3 (HO*P*-OH, td, ² *J*_{PH} = 18 Hz, ² *J*_{PP} = 4 Hz); 24.9 (HO-*P*-H, dtd, ¹*J*_{PH} = 526 Hz, ²*J*_{PH} = 18 Hz, ²*J*_{PP} = 4 Hz).
**MS:** (-) 158.6 [M-H⁺]⁻. (+) 160.8 [M+H⁺]⁺.
**TLC** (SiO₂, EtOH-conc. NH₄OH 1:1): *R*_{f} = 0.2.
**EA** (CH₆O₅P₂·0.5H₂O, *M*_{R} = 169.0): C 7.1 (7.1); H 4.2 (4.0).

### Example 8: Synthesis of compound 7

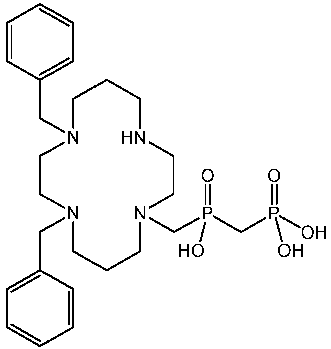

To a glass vial (20 ml), tetrahydrochloride of 1,4-dibenzyl-1,4,8,11-tetraazacyclotetradecane (554 mg; 1.05 µmol; 2.0 equiv.) was added. The intermediate **A**-0.5H₂O (355 mg; 1.05 mmol; 2.0 equiv.). CF₃COOH (5 mL), aqueous HCl (12 M; 5 mL) and paraformaldehyde (23.1 mg; 525 µmol; 1.0 equiv.) were subsequently added and the resulting suspension was stirred at 80 °C for two days. The mixture was evaporated to dryness and several times co-evaporated with H₂O. The crude product was further purified by column chromatography (SiO₂; EtOH-conc. NH₄OH 5:1; *R*_{f} = 0.3). The fractions with product were combined and evaporated to dryness. The residue was suspended in H₂O (25 mL), treated with small amounts of charcoal and filtered through syringe microfilter (Millipore; 0.22 µm). The filtrate was re-purified by ion exchange chromatography (DOWEX 50; H⁺-form; H₂O → 10% aqueous pyridine). The pyridine fraction with product was evaporated to dryness and further co-evaporated several times with H₂O. The residue was re-dissolved in H₂O (100 mL) and subsequently lyophilized. Product was obtained as a white substance (198 mg; 62%; 1 step, based on paraformaldehyde).
**NMR** (D₂O): ¹**H** *δ* 1.74-1.92 (CH₂-*CH*₂-CH2, bm, 4H); 2.09 (P-CH₂-P, t, 2H, ²*J*_{HP} = 16 Hz); 2.44-2.49 *cycle,* bm, 3H); 2.54 (*cycle,* m, 1H); 2.64-2.67 *(cycle,* m, 3H); 2.82-3.16 (*cycle,* N-C*H*₂-P, bm, 11H); 3.84 (C*H*₂-Ph, s, 2H); 3.96 (CH₂-Ph, s, 2H); 7.14-7.33 (Ph, bm, 6H); 7.37 (Ph, m, 2H); 7.44 (Ph, m, 2H); ¹³C{¹H} *δ* 25.5 (CH₂-*C*H₂-CH₂, s); 25.9 (CH₂-*C*H₂-CH₂, s); 36.6 (P-CH₂-P, dd, ¹*J*_{CP} = 118 Hz, *¹J_{CP}* = 83 Hz); 41.3 (*cycle,* s); 41.4 (*cycle,* s); 48.9 (*cycle,* s); 49.7 (*cycle,* d, ³*J*_{CP} = 10 Hz); 52.4 (*cycle,* s); 54.6 (*cycle,* s); 55.5 (*cycle,* d, ³*J*_{CP} = 10 Hz); 55.8 *(cycle,* s); 59.8 (C*H*₂-Ph, s); 60.1 (N-*C*H₂-P, d, ¹*J*_{CP} = 109 Hz); 61.3 (C*H*₂-Ph, s); 126.0 (*Ph,* s); 127.4 (*Ph,* s); 130.2 (*Ph,* s); 130.7 (*Ph,* s); 131.4 *(Ph,* s); 132.2 *(Ph,* s); 134.2 (*Ph,* s); 137.6 (*Ph,* s); ³¹**P**{¹H} *δ* 19.2 (HO-*P*-OH, d, 1P, ²*J*_{PP} = 7 Hz); 36.2 (N-CH-*P*, d, 1P, ²*J*_{PP} = 7 Hz).
**MS:** (-) 535.4 [M-H⁺]⁻. (+) 537.3 [M+H⁺]⁺; 559.5 [M+Na⁺]⁺; 581.2 [M-H⁺+2Na⁺]⁺. **TLC** (SiO₂, EtOH-conc. NH₄OH 5:1): *R*_{f} = 0.3.
**EA** (C₂₆H₄₂N₄O₄P₂·4H₂O,*M*_{R} = 608.6): C 50.9 (51.3); H 8.0 (8.3); N 9.4 (9.2).

### Example 9: Synthesis of intermediate product B

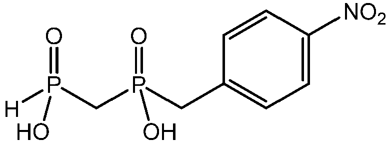

In a glass flask (100 mL), methylene-bis(phosphinic acid) (1.04 g; 7.22 mol; 1.0 equiv.) was suspended in dry CH₂Cl₂ (25 mL). *N*,*N*-diisopropylethylamine (9.90 mL; 57.8 mmol; 8.0 equiv.) was added and the flask was washed by gentle stream of argon gas. Then trimethylsilylchloride was added (5.50 mL; 43.5 mmol; 6.0 equiv.) and the resulting mixture was stirred at room temperature for one hour. Solution of 4-nitrobenzylbromidu (1.87 g; 8.66 mmol; 1.2 equiv.) in dry CH₂Cl₂ (20 mL) was added and the resulting mixture was stirred at room temperature for three days. Reaction was stopped by addition of EtOH (25 mL). The mixture was evaporated to dryness and further co-evaporated several times with EtOH. The residue was suspended in H₂O (100 mL). The solid material was filtered off and the filtrate was evaporated to dryness. Crude product was purified by ion exchange chromatography (DOWEX 1; OH -form; H₂O → 50% AcOH → 3% HCl). The latter fraction was evaporated to dryness and further purified by ion exchange chromatography (Amberlite CG50; H⁺-form; H₂O). Fraction with pure product were combined and evaporated to dryness and the residue was further dried on vaccum pump and subsequently in vaccum dessicator over P₂O₅. Product was obtained as a yellow substance (1.40 g; 69%; 1 step, based on methylene-bis(phosphinic acid).
**NMR** (D₂O): ¹**H** *δ* 2.28 (P-C*H*₂-P, m, 2H); 3.29 (C*H*₂-C, d, 2H, ²*J*_{HP} = 17 Hz); 7.16 (P*H*, d, 1H, ¹*J*_{HP} = 532 Hz); 7.53 (C*H*-C-CH₂, d, 2H, ²*J*_{HH} = 9 Hz); 8.21 (C*H-*C*-*N*,* d, 2H, ²*J*_{HH} = 9 Hz); ¹³**C**{¹H} *δ* 35.3 (P-CH₂-P, t, ¹*J*_{CP} = 78 Hz); 40.4 (P-*C*H₂-C, d, ¹*J*_{CP} = 86 Hz); 124.3 (C*H-*C*-*N*,* s); 131.3 (C*H*-C-CH₂, d, ³*J*_{CP} = 4 Hz); 144.0 (C*H*-C-CH₂, d, ²*J*_{CP} = 8 Hz); 146.7 (C*H-*C*-*N*,* s); ³¹**P** *δ* 18.7 (PH, dt, 1P, 1 *J*_{PH} = 532 Hz; ²*J*_{PH} = 18 Hz); 30.5 (CH₂-*P*-CH₂, p, 1P, ²*J*_{PH} = 17 Hz).
**MS:** (-) 277.5 [M-H⁺]⁻.
**TLC** (SiO₂, EtOH-conc. NH₄OH 5:1): *R*_{f} = 0.5.
**EA** (C₈HᵤNO₆P₂, *M*_{R} = 279.1): C 34.4 (34.2); H 4.0 (3.9); N 5.0 (4.8).

### Example 10: Synthesis of compound 8

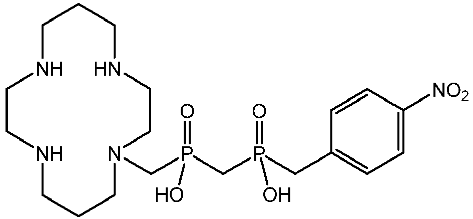

To a glass flask (100 ml), 1,4,8,11-tetraazacyklotetradecane (3.38 g; 16.9 mmol; 3.6 equiv.) was added. Intermediate **B** (1.31 g; 4.69 mmol; 1.0 equiv.), paraformaldehyde (169 mg; 5.63 mmol; 1.2 equiv.) and aqueous HCl (6 M; 60 mL) were subsequently added and the resulting mixture was stirred at 80 °C overnight. The mixture was evaporated to dryness and co-evaporated several times with H₂O. The crude product was purified by ion exchange chromatography (DOWEX 50; H⁺-form; H₂O → 10% aqueous pyridine). Pyridine fraction with product was evaporated to dryness and several times co-evaporated with H₂O. The residue was purified by ion exchange chromatography (DOWEX 1; OH -form; H₂O → 10% aqueous AcOH). Acetate fraction with product was evaporated to dryness and several times co-evaporated with H₂O. The residue was further dried on vaccum pump and subsequently in vaccum dessicator over P₂O₅. Product was obtained as a white substance (1.67 g; 71%; 1 step, based on intermediate **B**).
**NMR** (D₂O): ¹**H** *δ* 1.70 (CH₂-C*H*₂-CH₂, p, 2H, ³*J*_{HH} = 5 Hz); 1.76 (CH₂-C*H*₂-CH₂, p, 2H, ³*J*_{HH} = 5 Hz); 2.01 (P-C*H*₂-P, t, 2H, ²*J*_{HP} = 16 Hz); 2.55-2.83 *(cycle,* N-C*H*₂-P, m, 18H); 3.32 (C*H*₂-C-CH, d, 2H, ²*J*_{HP} = 17 Hz); 7.56 (C*H*-C-CH₂, d, 2H, ²*J*_{HH} = 9 Hz); 8.21 (C*H-*C*-*N*,* d, 2H, ²*J*_{HH} = 9 Hz); ¹³C{¹H} *δ* 25.4 (CH₂-*C*H₂-CH₂, s); 27.0 (CH₂-*C*H₂-CH₂, s); 33.2 (P-*C*H₂-P, t, ¹*J*_{CP} = 79 Hz); 40.8 (P-*C*H₂-C, d, *¹J_{CP}* = 86 Hz); 46.0 *(cycle,* s); 46.2 *(cycle,* s); 46.7 *(cycle,* s); 46.8 *(cycle,* s); 47.9 *(cycle,* s); 48.9 *(cycle,* s); 54.8 *(cycle,* s); 54.5 *(cycle,* s); 55.2 (P-*C*H₂-N, d, ¹*J*_{CP} = 110 Hz); 124.3 (C*H-*C*-*N*,* s); 131.3 (C*H*-C-CH₂, d, ³*J*_{CP} = 4 Hz); 144.6 (C*H*-C-CH₂, s); 146.6 (C*H-*C*-*N*,* s); ³¹**P**{¹H} *δ* 31.6 (CH₂-*P-*CH₂, d, 1P, ²*J*_{PP} = 9 Hz); 32.9 (CH₂-*P*-CH₂, d, 1P, ²*J*_{PP} = 9 Hz).
**MS:** (-) 489.7 [M-H⁺]⁻. (+) 491.9 [M+H⁺]⁺; 513.9 [M+Na⁺]⁺; 529.8 [M+K⁺]⁺.
**TLC** (SiO₂, EtOH-conc. NH₄OH 1:1): *R*_{f} = 0.7.
**EA** (C₁₉H₃₅N₅O₆P_{2·}0.5H₂O, *M*_{R} = 500.5): C 45.6 (45.2); H 7.3 (7.6); N 14.0 (13.8).

### Example 11: Synthesis of compound 9

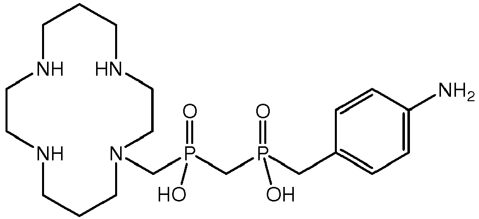

In a glass flask (100 ml), **8·**0.5H₂O (512 mg; 1.02 mmol; 1.0 equiv.) was dissolved in H₂O and the resulting mixture was briefly washed with gaseous argon. Ammonium formate (1.98 g; 31.4 mmol; 31 equiv.) and Pd@C catalyst (10%; 54 mg) were subsequently added. Resulting suspension was stirred at 60 °C for three hours. Another portion of ammonium formate (658 mg; 10.4 mmol; 10 equiv.) and Pd@C catalyst (10%; 52 mg) was added and the mixture was further stirred at 60 °C for two days. The mixture was then evaporated to dryness and several times evaporated with H₂O. The residue was dissolved in H₂O, catalyst was filtered off and the filtrate was purified by ion exchange chromatography (DOWEX 1; OH⁻-forma; H₂O → 10% aqueous AcOH). Acetate fraction with product was evaporated to dryness and then several times co-evaporated with H₂O. The residue was purified by ion exchange chromatography (DOWEX 50; H⁺-forma; H₂O → 10% aqueous pyridine). Pyridine fraction with product was evaporated to dryness and further co-evaporated several times with H₂O. The residue was re-dissolved in H₂O (150 ml) and lyophilized. Product was obtained as an off-white substance (443 mg; 84%; 1 step; based on **8·**0.5H₂O).
**NMR** (D₂O): ¹**H** *δ* 1.72 (CH₂-C*H*₂-CH₂, p, 2H, ³*J*_{HH} = 5 Hz); 1.79 (CH₂-C*H*₂-CH₂, p, 2H, ³*J*_{HH} = 5 Hz); 1.96 (P-C*H*₂-P, t, 2H, ²*J*_{HP} = 15 Hz); 2.62-2.88 *(cycle,* N-C*H*₂-P, m, 18H); 3.02 (C*H*₂-C-CH, d, 2H, ²*J*_{HP} = 17 Hz); 6.82 (*CH-*C-CH₂, d, 2H, ²*J*_{HH} = 8 Hz); 7.18 (C*H-*C*-*N*,* d, 2H, ²*J*_{HH} = 8 Hz); ¹³**C**{¹H} 25.0 (CH₂-CH₂-CH₂, s); 26.7 (CH₂-CH₂-CH₂, s); 32.7 (P-*C*H₂-P, t, ¹*J*_{CP} = 78 Hz); 39.4 (P-*C*H₂-C, d, ¹*J*_{CP} = 91 Hz); 45.7 (*cycle,* s); 46.2 (*cycle,* s); 46.7 *(cycle,* s); 46.8 (*cycle,* s); 48.7 (*cycle,* s); 49.4 (*cycle,* s); 54.8 (*cycle,* s); 54.9 (*cycle,* s); 55.0 (P-*C*H₂-N, d, ¹*J*_{CP} = 109 Hz); 117.3 (C*H-*C*-*N*,* s); 126.4 (C*H*-C-CH₂, d, ³*J*_{CP} = 8 Hz); 131.4 (C*H*-C-CH₂, s); 144.8 (C*H-*C*-*N*,* s); ³¹**P**{¹H} *δ* 33.7 (CH₂-*P-*CH₂, d, 1P, ² *J*_{PP} = 9 Hz); 34.6 (CH₂-*P*-CH₂, d, 1P, ²*J*_{PP} = 9 Hz).
**MS:** (-) 459.8 [M-H⁺]⁻. (+) 461.9 [M+H⁺]⁺; 483.9 [M+Na⁺]⁺; 499.9 [M+K⁺]⁺.
**TLC** (SiO₂, EtOH-conc. NH₄OH 1:1): *R*_{f} = 0.6.
**EA** (C₁₉H₃₇N₅O₄P_{2·}3H₂O, *M*_{R} = 515.5): C 44.3 (44.4); H 8.4 (8.6); N 13.6 (13.6).

### Example 12: Synthesis of compound 10

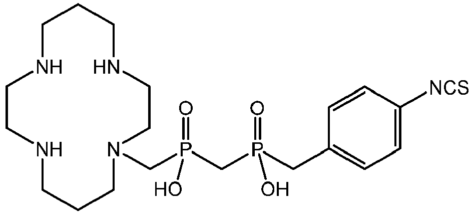

In a glass vial (20 mL), **9·**3H₂O (53.4 mg; 104 µmol; 1.0 equiv.) was dissolved in H₂O. The solution was acidified by aqueous HCl (1.12 M; 185 µL; 207 µmol; 2.0 equiv.). Freshly prepared solution of CSCl₂ (16 µL; 209 µmol; 2.0 equiv.) in CCl₄ (5 mL) was then added and the resulting biphasic mixture was vigorously stirred at room temperature overnight. Organic layer was separated and the aqueous layer was extracted with CCl₄ (2×20 mL) and Et₂O (1×20 mL). Aqueous layer was then separated and evaporated to dryness. The crude product was purified by preparative HPLC (C8; gradient elution H₂O-0,1% TFA-MeCN). Fraction with product was directly lyophilized. Product was obtained as a white substance (70.9 mg; 79%; 1 step; based on 9-3H₂O).
NMR (D₂O): ¹**H** *δ* 2.19 (CH₂-CH₂-CH₂, m, 4H); 2.40 (P-CH₂-P, t, 2H, ²*J*_{HP} = 16 Hz); 3.13-3.60 *(cycle,* N-C*H*₂-P, P-C*H*₂-C, m, 20H); 7.36 (CH, m, 4H); ¹³**C**{¹H} *δ* 21.1 (CH₂-CH₂-CH₂, s); 21.7 (CH₂-CH₂-CH₂, s); 32.8 (P-CH₂-P, t, ¹*J*_{CP} = 81 Hz); 38.8 (P-*C*H₂-C, d, ¹*J*_{CP} = 91 Hz); 40.5 *(cycle,* s); 41.4 *(cycle,* s); 42.8 *(cycle,* s); 43.1 *(cycle,* s); 43.9 *(cycle,* s); 44.3 *(cycle,* s); 52.9 *(cycle,* s); 54.3 (P-*C*H₂-N, d, ¹*J_{CP}* = 100 Hz); 54.4 *(cycle,* s); 126.7 (C*H-*C*-*N*,* s); 130.1 (CH-*C*-N, s); 131.6 (C*H*-C-CH₂, d, ³*J*_{CP} = 5 Hz); 132.9 (C*H*-C-CH₂, d, ²*J*_{CP} = 8 Hz); 134.8 (NCS, s); ³¹**P**{¹H} *δ* 27.0 (CH₂-*P*-CH₂, bm, 1P); 39.1 (CH₂-*P*-CH₂, d, 1P, ²*J*_{PP} = 7 Hz).
**MS:** (-) 501.8 [M-H⁺]⁻. (+) 504.0 [M+H⁺]⁺.
**EA** (C₂₀H₃₅N₅O₄P₂S·TFA·3.5H₂O, *M*_{R} = 863.3): C 30.6 (30.8); H 5.0 (4.8); N 8.1 (8.0).

### Example 13: Synthesis of compound 11

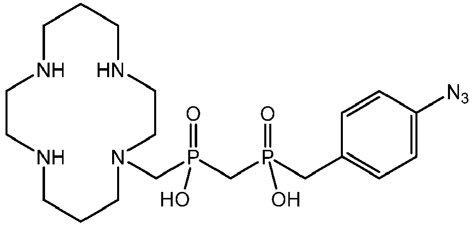

In a glass vial (4 mL), **9·**3H₂O (40.2 mg; 78.0 µmol; 1.3 equiv.) was dissolved in aqueous HCl (243.8 mM; 962 µL; 235 µmol; 4.0 equiv.) and the resulting mixture was cooled in an ice bath (1 °C). Freshly prepared aqueous solution of NaNO₂ was then gradually added (290 mM; 200 µL; 58.0 µmol; 1.0 equiv.; 20 µL each three minutes). Freshly prepared aqueous solution of NaN₃ (580 mM; 200 µL; 116 µmol; 2.0 equiv.) was then added. The reaction mixture was then stirred at room temperature for three hours. The crude product was purified by preparative HPLC (C8; gradient elution H₂O-0,1% TFA-MeCN). Fraction with product was directly lyophilized. Product was obtained as a white substance (29.5 mg; 57%; 1 step; based on **9**·3H₂O).
NMR (D₂O): ¹**H** *δ* 2.07 (CH₂-C*H*₂-CH₂, p, 2H, ³*J*_{HH} = 6 Hz); 2.13 (CH₂-C*H*₂-CH₂, p, 2H, ³*J*_{HH} = 7 Hz); 2.20 (P-C*H*₂-P, t, 2H, ²*J*_{HP} = 16 Hz); 2.80-3.57 *(cycle,* N-C*H*₂-P, P-C*H*₂-C, bm, 20H); 7.11 (C*H-*C*-*N*,* d, 2H, ³*J*_{HH} = 8 Hz); 7.34 (C*H*-C-CH₂, d, 2H, ³*J*_{HH} = 8 Hz); ¹³C{¹H} *δ* 22.4 (CH₂-*C*H₂-CH₂, s); 22.7 (CH₂-*C*H₂-CH₂, s); 32.3 (P-*C*H₂-P, t, ¹*J*_{CP} = 80 Hz); 39.3 (P-*C*H₂-C, d, ¹*J*_{CP} *=* 91 Hz); 42.4 (*cycle,* s); 43.5 *(cycle,* s); 44.3 (*cycle,* s); 45.1 *(cycle,* s); 45.4 (*cycle,* s); 46.0 (*cycle,* s); 53.7 (P-*C*H₂-N, d, ¹*J*_{CP} = 105 Hz); 54.6 (*cycle,* s); 55.6 *(cycle,* s); 119.9 (C*H-*C*-*N*,* s); 130.9 (C*H*-C-CH₂, d, ²*J*_{CP} = 8 Hz); 131.8 (C*H*-C-CH₂, d, ³*J*_{CP} = 5 Hz); 139.0 (C*H-*C*-*N*,* s); ³¹**P**{¹H} *δ* 29.6 (CH₂-*P*-CH₂, bm, 1P); 37.1 (CH₂-*P*-CH₂, d, 1P, ²*J*_{PP} = 9 Hz).
**MS:** (-) 485.8 [M-H⁺]⁻. (+) 488.0 [M+H⁺]⁺; 509.9 [M+Na⁺]⁺; 531.9 [M+K⁺]⁺.
**TLC** (SiO₂, EtOH-conc. NH₄OH 2:1): *R*_{f} = 0.7.
**EA** (C₁₉H₃₅N₇O₄P₂·0.5TFA·1.5H₂O, *M*_{R} = 662.9): C 36.2 (36.4); H 5.9 (5.6); N 14.8 (15.0).

### Example 14: Synthesis of intermediate product C

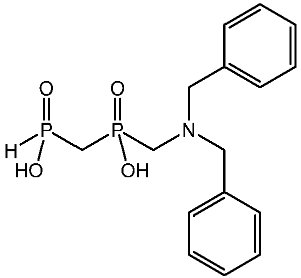

In a glass flask (100 mL), methylene-bis(phosphinic acid) (2.32 g; 16.1 mol; 1.0 equiv.) and dibenzylamine (4.03 mL; 21.0 mmol; 1.3 equiv.) were suspended in a mixture of 6 M aqueous HCl-THF (1:1; 50 mL). Paraformaldehyde (726 mg; 24.2 mmol; 1.5 equiv.) was then added, the flask was quickly sealed with stopper and the resulting mixture was stirred at 80 °C overnight. After cooling to room temperature, the mixture was evaporated to dryness. The residue was dissolved in H₂O and washed with CH₂Cl₂. Aqueous layer was separated, evaporated to dryness and the residue was purified was purified by flash chromatography (C18; gradient elution H₂O-0,1% TFA-MeCN). Fraction with product was evaporated to dryness and the residue was re-dissolved in small volume of MeOH. Et₂O was subsequently added until cloudiness. Next day, the formed precipitate was collected on glass frit S3 and washed with Et₂O. Product was obtained as a white substance (2.27 mg; 37%; 1 step; based on methylene-bis(phosphinic acid)).
**NMR** (D₂O): ¹**H** *δ* 2.02 (P-C*H*₂-P, t, 2H, ²*J*_{HP} = 17 Hz); 3.25 (P-*C*H₂-N, d, 2H, ²*J*_{HP} = 11 Hz); 4.46 (C*H*₂-Ph, m, 4H); 7.15 (P*H*, d, 1H, *¹J*_{HP} = 562 Hz); 7.52 *(Ph,* m, 10H); ¹³C{¹H} δ 35.8 (P-*C*H₂-P, t, *¹J_{CP}* = 81 Hz); 51.7 (P-*C*H₂-N, d, ¹*J*_{CP} = 92 Hz); 59.4 (C*H*₂-Ph, s); 129.8 (*C*-CH₂, s); 129.9 *(Ph,* s); 130.8 *(Ph,* s); 132.1 *(Ph,* s); ³¹**P** *δ* 20.9 (P-*C*H₂-N, m, 1P); 22.1 (*P*H, dm, 1P, 1 *J*_{PH} = 562 Hz).
**MS:** (-) 351.6 [M-H⁺]⁻. (+) 353.7 [M+H⁺]⁺; 391.7 [M+K⁺]⁺.
**TLC** (SiO₂, MeOH): *R*_{f} *=* 0.6.
**EA** (C₁₆H₂₁NO₄P_{2·}MeOH, *M*_{R} = 385.3): C 53.0 (53.1); H 6.5 (6.2); N 3.6 (3.9).

### Example 15: Synthesis of compound 12

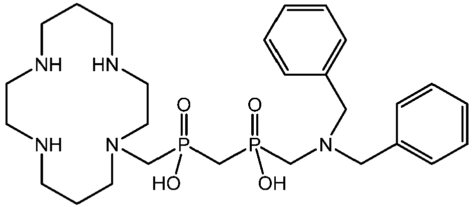

To a glass flask (100 ml), 1,4,8,11-tetraazacyklotetradecane (3.26 g; 16.3 mmol; 5.5 equiv.) was added. Intermediate C·MeOH (1.15 g; 2.99 mmol; 1.0 equiv.), paraformaldehyde (160 mg; 5.33 mmol; 1.8 equiv.) and aqueous HCl (6 M; 60 mL) were subsequently added and the resulting mixture was stirred at 80 °C overnight. Mixture was evaporated to dryness and co-evaporated several times with H₂O. Crude product was purified by ion exchange chromatography (DOWEX 50; H⁺-form; H₂O → 5% aqueous ammonia). Ammonia fraction with product was evaporated to dryness and several times co-evaporated with H₂O. The residue was purified by ion exchange chromatography (DOWEX 1; OH⁻-form; H₂O → 10% aqueous AcOH). Acetate fraction with product was evaporated to dryness and several times co-evaporated with H₂O. The residue was further dried on vaccum pump and subsequently in vaccum dessicator over P₂O₅. Product was obtained as a white substance (1.46 g; 74%; 1 step, based on intermediate C·MeOH).
**NMR** (D₂O): ¹**H** *δ* 1.67 (CH₂-C*H*₂-CH₂, p, 2H, ³*J*_{HH} = 5 Hz); 1.71 (CH₂-C*H*₂-CH₂, p, 2H, ³*J*_{HH} = 5 Hz); 2.07 (P-C*H*₂-P, t, 2H, ²*J*_{HP} = 15 Hz); 2.55-2.73 (N-*CH₂-*P*, cycle,* m, 18H); 2.76 (N-*C*H₂-P, d, 2H, ²*J*_{HP} = 9 Hz); 3.79 (C*H*₂-Ph, s, 4H); 7.32-7.49 (*Ph,* m, 10H); ¹³**C**{¹H} *δ* 25.1 (CH₂-*C*H₂-CH₂, s); 27.0 (CH₂-*C*H₂-CH₂, s); 33.5 (P-*C*H₂-P, t, ¹*J*_{CP} = 77 Hz); 45.7 (*cycle,* s); 45.9 (*cycle,* s); 46.3 (*cycle,* s); 46.4 (*cycle,* s); 53.7 (*cycle,* s); 54.6 (*cycle,* s); 54.9 (P-*C*H₂-N, d, ¹*J*_{CP} = 107 Hz); 55.2 (P-*C*H₂-N, d, ¹*J*_{CP} = 109 Hz); 58.9 (C*H*₂-Ph, d, ³*J*_{CP} = 6 Hz); 127.8 *(Ph,* s); 128.9 *(Ph,* s); 130.3 *(Ph,* s); 139.0 *(Ph,* s); ³¹**P**{¹H} *δ* 33.1 (P-*C*H₂-N, d, 1P, ² *J*_{PP} = 12 Hz) 33.6 (P-*C*H₂-N, d, 1P, ²*J*_{PP} = 12 Hz).
**MS:** (-) 563.8 [M-H⁺]⁻. (+) 566.0 [M+H⁺]⁺; 588.0 [M+Na⁺]⁺; 604.0 [M+K⁺]⁺.
**TLC** (SiO₂, EtOH-conc. NH₄OH 2:1): *R*_{f} = 0.8.
**EA** (C₂₇H₄₅N₅O₄P₂·AcOH·1.5H₂O, *M*_{R} = 657.2): C 53.4 (53.4); H 8.0 (7.7); N 10.7 (10.6).

### Example 16: Synthesis of compound 13

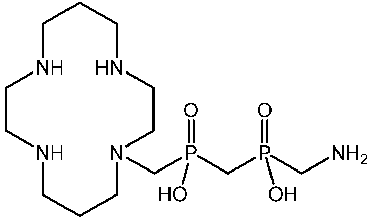

In a glass flask (250 mL), **12·**AcOH·1.5H₂O (1.55 g; 2.36 mmol) was dissolved in H₂O and the resulting solution was briefly washed with argon gas. HCOONH₄ (4.45 g; 70.5 mmol; 30 equiv.) and catalyst (10% Pd@C; 113 mg) were subsequently added and the resulting suspension was stirred at 60 °C for three hours. Another portion of HCOONH₄ (1.48 g; 23.5 mmol; 10 equiv.) and catalyst (10% Pd@C; 41 mg) was added and the mixture was further stirred for two days. The mixture was evaporated to dryness and further co-evaporated several times with H₂O. The residue was dissolved in H₂O, catalyst was filtered off and the filtrate was purified by ion exchange chromatography (DOWEX 50; H⁺-form; H₂O → 5% aqueous ammonia). Ammonia fraction with product was evaporated to dryness and further co-evaporated several times with H₂O. The residue was re-dissolved in H₂O (150 ml) and lyophilized. Product was obtained as an off-white substance (720 mg; 74%; 1 step; based on **13**·AcOH·1.5H₂O).
**NMR** (D₂O): ¹**H** *δ* 1.69 (CH₂-CH₂-C*H*₂, p, 2H, ³*J*_{HH} = 6 Hz); 1.81 (CH₂-C*H*₂-CH₂, m, 2H); 2.11 (P-C*H*₂-P, dd, 2H, ²*J*_{HP} = 17 Hz, ²*J*_{HP} =15 Hz); 2.61-3.32 (N-*CH₂-*P*, cycle,* m, 20H); ¹³**C**{¹H} *δ* 29.2 (CH₂-*C*H₂-CH₂, s); 30.1 (CH₂-*C*H₂-CH₂, s); 36.4 (P-*C*H₂-P, t, ¹*J*_{CP} = 78 Hz); 39.9 (*cycle,* s); 42.2 (*cycle,* s); 43.3 (*cycle,* s); 44.3 (*cycle,* s); 45.8 (*cycle,* s); 51.1 (*cycle,* s); 51.8 (P-*C*H₂-N, d, ¹*J*_{CP} = 105 Hz); 57.8 (P-*C*H₂-N, d, *¹J*_{CP} = 102 Hz); ³¹**P**{¹H} *δ* 24.0 (*P*-CH₂-NH₂, d, 1P, ²*J*_{PP} = 5 Hz); 31.3 (C-N-CH₂-*P,* d, 1P, ²*J*_{PP} = 5 Hz).
**MS:** (-) 383.9 [M-H⁺]⁻. (+) 385.7 [M+H⁺]⁺; 407.9 [M+Na⁺]⁺.
**TLC** (SiO₂, EtOH-conc. NH₄OH 1:1): *R*_{f} = 0.6.
**EA** (C₁₃H₃₃N₅O₄P₂·2.5H₂O, *M*_{R} = 412.4): C 40.5 (40.3); H 8.6 (8.9); N 18.2 (18.4).

### Example 17: Synthesis of intermediate D

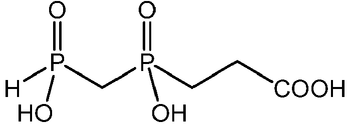

In a argon-washed three-necked flask (1000 mL), methylene-bis(phosphinic acid) (2.50 g; 17.4 mol; 1.0 equiv.) was suspended in hexamethyldisilizane (150 mL; 716 mmol; 41 equiv.) and the resulting mixture was stirred at 110 °C under gentle flow of argon overnight. The mixture was then cooled to room temperature and dry CH₂Cl₂ (100 mL) was subsequenty added followed by addition of *tert*-butyl acrylate (5.00 mL; 34.2 mmol; 2.0 equiv.) in dry CH₂Cl₂ (100 mL). The resulting mixture was further stirred at room temperature overnight. Reaction was quenched with EtOH (150 mL) and the resulting mixture was evaporated to dryness. The crude product was purified by flash chromatography (C18; gradient elution H₂O-0,1% TFA-MeCN). Fractions with pure product were combined and evaporated to dryness. The residue was further purified by ion exchange chromatography (Amberlite CG50; H⁺-form; H₂O). Aqueous fraction was evaporated to dryness and further co-evaporated several times with CH₂Cl₂. Residue was dissolved in CH₂Cl₂-TFA (1:1; 200 mL) and the resulting mixture was stirred overnight at RT in the absence of light. Reaction mixture was then evaporated to dryness and the residue was purified by ion exchange chromatography (DOWEX 50; H+-form; H₂O). Fractions with product were combined and evaporated to dryness. The residue was further purified on vacuum pump and subsequently in vacuum dessicator over P₂O₅. Product was obtained as a white substance (1.41 g; 38%; 2 steps; based on methylene-bis(phosphinic acid)).
**NMR** (D₂O): ¹**H** *δ* 1.97 (P-C*H*₂-CH₂, m, 2H); 2.21 (P-C*H*₂-P, t, 2H, ²*J*_{HP} = 17 Hz); 2.46 (P-C*H*₂-C*H*₂, m, 2H); 7.01 (P*H*, 1H, d, ¹ *J*_{HP} = 560 Hz); ¹³**C**{¹H} 26.2 (P-C*H*₂-CH₂, d, ¹*J*_{CP} = 98 Hz); 27.1 (P-C*H*₂-*C*H₂, s); 33.0 (P-C*H*₂₋P, t, ¹*J*_{CP} = 79 Hz); 177.2 (CO, d, ³*J*_{CP} = 16 Hz); ³¹**P** *δ* 19.2 (*P*H, d, 1P, ¹*J* _{PH} = 544 Hz); 44.5 (*P-*CH₂-CH₂, m, 1P).
**MS:** (-) 196.6 [M-H₃O⁺]⁻; 214.5 [M-H⁺]⁻. (+) 216.7 [M+H⁺]⁺.
**TLC** (SiO₂, EtOH-conc. NH₄OH 1:1): *R*_{f} = 0.6.
**EA** (C₄H₁₀O₆P₂, *M*_{R} = 216.1): C 22.2 (21.9); H 4.7 (5.0).

### Example 18: Synthesis of compound 14

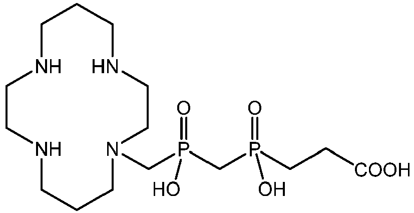

To a glass vial (20 ml), 1,4,8,11-tetraazacyklotetradecane (1.19 g; 5.95 mmol; 3.8 equiv.) was added. Intermediate **D** (340 mg; 1.57 mmol; 1,0 equiv.), paraformaldehyde (58 mg; 1.3 mmol; 1.2 equiv.) and aqueous HCl (6 M; 10 mL) were subsequently added and the resulting mixture was stirred at 60 °C overnight. Next day, mixture was evaporated to dryness and co-evaporated several times with H₂O. Crude product was purified by ion exchange chromatography (IRA 402; OH⁻-form; H₂O → 10% aqueous AcOH). Acetate fraction with product was evaporated to dryness and several times co-evaporated with H₂O. The residue was purified by ion exchange chromatography (DOWEX 50; H⁺-form; H₂O → 10% aqueous pyridine). Pyridine fraction with product was evaporated to dryness and several times co-evaporated with H₂O. The residue was re-dissolved in H₂O (50 mL) and subsequently lyophilized. Product was obtained as a white substance (459 mg; 61%; 1 step, based on intermediate **D).**
**NMR** (D₂O): **¹H** *δ* 1.71 (CH₂-C*H*₂-CH₂, p, 2H, ³*J*_{HH} = 5 Hz); 1.76 (CH₂-C*H*₂-CH₂, p, 2H, ³*J*_{HH} = 5 Hz); 1.91 (P-C*H*₂-C, m, 2H); 2.05 (P-C*H*₂-P, t, 2H, ²*J*_{HP} = 16 Hz); 2.37 (C*H*₂-CO, m, 2H); 2.65 *(cycle,* t, 2H, ³*J*_{HH} = 5 Hz); 2.69-2.79 *(cycle,* N-C*H*₂-P, m, 16H); ¹³**C**{¹H} *δ* 25.6 (*C*H₂-CH₂-CH₂, s); 27.2 (CH₂-*C*H₂-CH₂, s); 29.7 (P-*C*H₂-C, d, ¹*J_{CP}* = 96 Hz); 31.2 (*C*H₂-CO, s); 33.5 (P-*C*H₂-P, t, ¹*J*_{CP} = 77 Hz); 46.2 (*cycle,* s); 46.3 (*cycle,* s); 46.8 (*cycle,* s); 46.9 (*cycle,* s); 47.6 (*cycle,* s); 48.8 (*cycle,* s); 54.4 (*cycle,* s); 54.8 (*cycle,* s); 55.2 (N-*C*H₂-P, d, *¹J_{CP}* = 109 Hz); 183.2 (CO, d, ³*J*_{CP} = 19 Hz); ³¹**P**{¹H} *δ* 33.4 (P-*C*H₂-N, d, 1P, ²*J*_{PP} = 10 Hz); 36.6 (*P*-C*H*₂-CH₂, d, 1P, ²*J*_{PP} = 10 Hz).
**MS:** (-) 402.8 [M-H₃O⁺]⁻ ; 426.7 [M-H⁺]⁻. (+) 428.9 [M+H⁺]⁺; 450.8 [M+Na⁺]⁺; 466.8 [M+K⁺]⁺.
**TLC** (SiO₂, *i*PrOH-conc.NH₄OH-H₂O7:1:1): *R*_{f} = 0.5.
**EA** (C₁₅H₃₄N₄O₆P₂·4H₂O, *M*_{R} = 500.5): C 36.0 (36.0); H 8.5 (8.8); N 11.2 (11.2).

### Example 19: Synthesis of intermediate product E

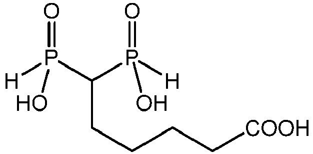

In a glass beaker (1000 mL), NaH₂PO₂·H₂O (28.6 g; 270 mmol; 6.0 equiv.) was dissolved in a mixture of MeOH (500 mL) and unstabilized dioxane (75 mL). 5-hexynoic acid (5.00 mL; 45.3 mmol; 1.0 equiv.) and solution of Et₃B in hexanes (1.0 M; 45 mL; 45.0 mmol; 1.0 equiv.) were subsequently added and the resulting mixture was vigorously stirred in presence of air for four hours. The resulting precipitate was collected on glass frit S3 and several times washed with cold MeOH and air dried. The crude product was purified by column chromatography (SiO₂; EtOH-conc. NH₄OH 2:1; *R*_{f} = 0.4). Fractions with product were combined and evaporated to dryness. The residue was re-dissolved in H₂O (150 mL) and treated with charcoal. The resulting suspension was filterred through syringe microfilter (Millipore; 0.22 µm) and the filtrate was further purified by ion exchange resin (DOWEX 50; H⁺-form; H₂O). Aqueous fraction was evaporated to dryness, re-dissolved in H₂O and subsequently lyophilized (4.31 g; 32%; 1 step; based on Et₃B).
NMR (D₂O): ¹**H** *δ* 1.57 (C*H*₂, m, 2H); 1.64 (C*H*₂, m, 2H); 1.74 (*C*H₂, m, 2H); 1.87 (P-C*H*-P, m, 1H); 2.38 (C*H*₂-CO, t, 2H, 3 *J*_{HH} = 8 Hz); 7.04 (P*H*, dm, 2H, ¹*J*_{HP} = 526 Hz); ¹³C{¹H} *δ* 21.7 (P-CH-CH₂-*C*H₂, t, ³*J*_{CP} = 3 Hz); 25.6 (CH₂-CH₂-CO, s); 29.4 (P-CH-*C*H₂, t, ²*J*_{CP} = 7 Hz); 35.2 (*C*H₂-CO, s); 44.8 (P-C*H*-P, t, ¹*J*_{CP} = 78 Hz); 180.9 (CO, s); ³¹**P** *δ* 25.8 (dm, ¹*J*_{PH} = 526 Hz).
**MS:** (-) 242.5 [M-H⁺]⁻; 264.5 [M-2H⁺+Na⁺]⁻; 286.5 [M-3H⁺+2Na⁺]⁻. (+) 310.8 [M-2H⁺+3Na⁺]⁺.
**TLC** (SiO₂, EtOH-conc. NH₄OH 2:1): *R*_{f} = 0.4.
**EA** (C₆H₁₄O₆P₂·3H₂O, *M*_{R} = 298.2): C 24.2 (24.1); H 6.8 (6.5).

### Example 20: Synthesis of compound 15

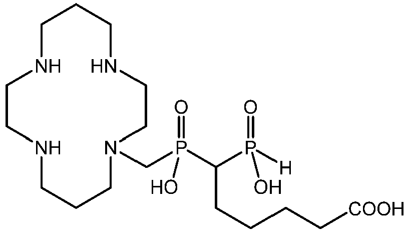

To a glass vial (20 ml), 1,4,8,11-tetraazacyklotetradecane (1.50 g; 7.50 mmol; 3.8 equiv.) was added. Intermediate **E·**3H₂O (1.51 g; 5.08 mmol; 2.6 equiv.), paraformaldehyde (59.2 mg; 1.97 mmol; 1.0 equiv.) and aqueous HCl (6 M; 10 mL) were subsequently added and the resulting mixture was stirred at 70 °C overnight. Next day, the mixture was evaporated to dryness and co-evaporated several times with H₂O. The crude product was purified by ion exchange chromatography (DOWEX 50; H⁺-form; H₂O → 10% aqueous pyridine). Pyridine fraction with product was evaporated to dryness and several times co-evaporated with H₂O. The crude producted was purified by column chromatography (SiO₂; *i*PrOH-konc. NH₄OH-H₂O 7:3:3; *R*_{f} = 0.5) Fractions with product were combined and evaporated to dryness. The residue was suspended in H₂O (50 mL) and treated with charcoal. Suspension was then filtered throught syringe microfilter (Millipore; 0.22 µm). The filtrate was re-purified by ion exchange chromatography (IRA 402; OH⁻ -form; H₂O → 10% aqueous AcOH). Acetate fraction with product was evaporated to dryness and several times co-evaporated with H₂O. The residue was re-dissolved in H₂O (50 mL) and subsequently lyophilized. Product was obtained as a white substance (621 mg; 61%; 1 step, based on paraformaldehyde).
**NMR** (D₂O): ¹**H** *δ* 1.39 (CH₂-C*H*₂-CH₂, m, 1H); 1.50 (CH₂-C*H*₂-CH₂, m, 3H); 1.57-1.77 (CH₂-C*H*₂-CH₂, C*H*₂-CH-P, P-C*H*-P, m, 7H); 2.12 (C*H*₂-COOH, t, 2H, ³*J*_{HH} = 7 Hz); 2.51-2.79 (*cycle,* N-C*H*₂-P, m, 18H); 7.05 (P*H*, d, ¹*J*_{HP} = 525 Hz); ¹³**C**{¹H} *δ* 23.8 (*C*H₂-CH₂-CH-P, t, ³*J*_{CP} = 3 Hz); 25.6 (CH₂-*C*H₂-CH₂, s); 26.9 (CH₂-CH₂-CH₂, s); 27.2 (CH₂-*C*H₂-CH₂, s); 30.8 (*C*H₂-CH-P, dd, ²*J*_{CP} = 8 Hz, ²*J*_{CP} = 4 Hz); 38.3 (*C*H₂-CO, s); 45.0 (P-C*H*-P, dd, ¹*J*_{CP} = 79 Hz, ¹*J*_{CP} = 73 Hz); 45.8 (*cycle,* s); 46.1 (*cycle,* s); 46.4 (*cycle,* s); 46.7 (*cycle,* s); 47.3 (*cycle,* s); 48.2 (*cycle,* s); 53.3 (N-*C*H₂-P, d, ¹*J*_{CP} = 103 Hz); 54.1 (*cycle,* d, ³*J*_{CP} = 7 Hz); 54,7 (*cycle,* d, ³*J*_{CP} = 6 Hz); 184.6 (CO, s); ³¹**P** *δ* 25.7 (*P*H, dm, 1P, ¹*J*_{HP} = 525 Hz); 36.3 (*P*-*C*H₂-N, m, 1P).
**MS:** (-) 454.7 [M-H⁺]⁻. (+) 457.0 [M+H⁺]⁺.
**TLC** (SiO₂, *i*PrOH-conc. NH₄OH-H₂O 7:1:1): *R*_{f} = 0.5.
**EA** (C₁₄H₃₂N₄O₅P₅·AcOH, *M*_{R} = 516.5): C 44.2 (44.1); H 8.2 (8.5); N 10.9 (10.7).

### Example 21: Synthesis of compound 16

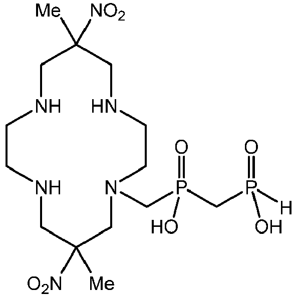

To a glass flask (25 ml), 6,13-dimethyl-6,13-dinitro-1,4,8,1 1-tetraazacyklotetradecane (411 mg; 885 mmol; 4.7 equiv.) was added. Methylene-bis(phosphinic acid) (61.3 mg; 426 µmol; 2.3 equiv.) and paraformaldehyde (5.6 mg; 187 µmol; 1.0 equiv.) and aqueous HCl (6 M; 10 mL) were subsequently added and the resulting mixture was stirred at 60 °C for two days. The mixture was then evaporated to dryness and several times co-evaporated with H₂O. The crude product was purified by ion exchange chromatography (DOWEX 50; 200 mL; H⁺-form; H₂O → 10% aqueous pyridine). Pyridine fraction with product was evaporated to dryness and several times co-evaporated with H₂O. The residue was further dried on vaccuum pump and in vaccum dessicator over P₂O₅. Product was obtained as a yellowish substance (92.2 mg; 82%; 1 step, based on methylene-bis(phosphinic acid)).
**NMR** (D₂O): ¹**H** *δ* 1.65 (C*H₃,* s, 3H); 1.74 (C*H₃,* s, 3H); 2.12 (P-C*H*₂-P, dd, 2H, ²*J*_{HP} = 18 Hz, ²*J*_{HP} = 16 Hz); 2.69-2.94 (*cycle,* N-C*H*₂-P m, 8H); 3.03-3.41 *(cycle,* m, 10H); 7.14 (P*H*, d, 1H, ¹*J*_{HP} = 530 Hz); ¹³C{¹H} *δ* 24.2 (C*H₃,* s); 25.2 (C*H₃,* s); 35.5 (P-*C*H₂-P, dd, ¹*J*_{CP} = 79 Hz, ¹*J*_{CP} = 76 Hz); 48.2 (*cycle,* s); 48.8 (*cycle,* s); 49.2 (*cycle,* s); 50.1 (*cycle,* s); 52.3 (*cycle,* s); 52.4 (*cycle,* s); 55.2 (*cycle,* s); 57.6 (N-*C*H₂-P, d, ¹*J*_{CP} = 104 Hz); 58.2 (*cycle,* d, *³J*_{CP} = 10 Hz); 89.9 (*C*-NO₂, s); 91.8 (*C*-NO₂, s); ³¹**P** *δ* 16.2 (*P*H, dt, 1P, ¹*J*_{PH} = 530 Hz, ²*J*_{PH} = 18 Hz); 36.9 (N-*C*H₂-P, m, 1P).
**MS:** (-) 473.3 [M-H⁺]⁻. (+) 475.1 [M+H⁺]⁺; 513.4 [M+K⁺]⁺.
**TLC** (SiO₂, EtOH-konc. NH₄OH 1:1): R_{f} = 0.8.
**EA** (C₁₄H₃₂N₆O₈P₂·3HCl·H₂O, *M*_{R} = 601.8): C 27.9 (27.7); H 6.2 (6.5); N 14.0 (14.3).

### Example 22: Synthesis of conjugate 17

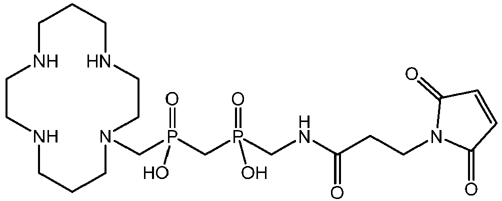

In a glass vial (4 ml), **13**·1.5H₂O (14.1 mg; 34.2 µmol; 1.0 equiv.) and *N-*hydroxysuccinimide ester of 3-(maleimido)propanoic acid (10.0 mg; 37.6 µmol; 1.1 equiv.) were dissolved in aqueous buffer H₃PO₄-NaOH (1.0 M pH = 8.1; 1.00 mL; 1.0 mmol; 29 equiv.). The resulting mixture was stirred at room temperature overnight. The crude product was purified by preparative HPLC (C8; H₂O-MeCN). Fraction with product was lyophilized. Product was obtained as a white substance (13.4 mg; 45%; 1 step, based on **13**.1.5H₂O).
**NMR** (D₂O): ¹**H** *δ* 1.99 (CH₂-CH₂-C*H*₂, p, 2H, ³*J*_{HH} = 7 Hz); 2.12 (CH₂-C*H*₂-CH₂, m, 2H); 2.18 (P-C*H*₂-P, t, 2H, ²*J*_{HP} = 16 Hz); 2.48-3.11 (C*H*₂-CO, N-C*H₂-*P*, cycle,* m, 20H); 4.21 (C*H*₂-N-CO, t, ³*J*_{HH} = 9 Hz); 6.82 (C*H,* s, 2H); ¹³**C**{¹H} *δ* 22.1 (CH₂-CH₂-CH₂, s); 23.4 (CH₂-CH₂-CH₂, s); 29.8 (*C*H₂-CO, s); 34.1 (P-CH₂-P, t, ¹*J*_{CP} = 79 Hz); 43.1 (*C*H₂-N-CO, s); 41.9 (*cycle,* s); 43.6 (*cycle,* s); 43.9 (*cycle,* s); 44.3 (*cycle,* s); 46.8 (*cycle,* s); 47.2 (*cycle,* s); 53.4 (P-*C*H₂-N, d, ¹*J*_{CP} = 96 Hz); 53.6 (*cycle,* s); 55.1 (*cycle,* s); 55.2 (P-*C*H₂-N, d, ¹*J*_{CP} = 108 Hz); 133.7 (CH, s); 169.8 (*C*O-N-CO, s); 174.5 (CH₂₋*C*O-N, d, ³*J*_{CP} = 5 Hz). ³¹**P**{¹H}*δ* 32.8 (P-*C*H₂-N, d, 1P, ² *J*_{PP} = 11 Hz); 35.2 (*P*-*C*H₂-N, d, 1P, ²*J*_{PP} = 11 Hz).
**MS:** (-) 535.0 [M-H⁺]⁻. (+) 537.1 [M+H⁺]⁺; 559.1 [M+Na⁺]⁺; 575.1 [M+K⁺]⁺.
**TLC** (SiO₂, EtOH-conc. NH₄OH 2:1): *R*_{f} = 0.5.
**EA** (C_{2O}H₃₈N₆O₇P₂·TFA-2H₂O, *M*_{R} = 869.3): C 30.4 (30.7); H 5.0 (4.7); N 9.7 (9.5).

### Example 23: Synthesis of conjugate 18

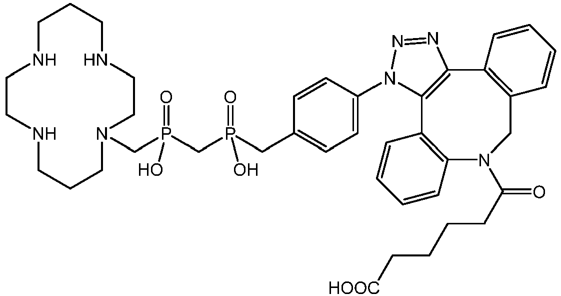

To a glass vial (4 mL), solution of **11**.0.5TFA·1.5H₂O (20.4 mM in D₂O; 420 µL; 8.57 µmol; 1.0 equiv.) was pipetted and, then, freshly prepared solution of 6-dibenzocyclooctynamidehexanoic acid (3.0 mg; 9.0 µmol; 1.1 equiv.) in a mixture of dry DMSO-dry MeCN (1:1; 500 µL) was added. The resulting mixture was stirred at room temperature for 2 hours. The crude product was purified by preparative HPLC (C8; H₂O--MeCN). Fractions with product were lyophilized. Product as two regioisomers was obtained as a white substance (2 × ∼1 mg).
**NMR** (D₂O): ¹**H** *δ* 1.18 (C*H*₂, m, 2H); 1.32 (C*H*₂, m, 2H); 2.04 (*cycle,* p, ³*J*_{HH} = 6 Hz); 2.15 (*cycle,* P*-CH*₂*-*P*,* CH₂, m, 6H); 2.36 (*C*H₂, m, 1H); 2.56 (C*H*₂, m, 1H); 2.67-3.63 (*cycle,* N-C*H*₂-P, P-C*H*₂-C, m, 20H); 5.07 (C*H*₂-N-CO, d, 1H, ²*J*_{HH} = 18 Hz); 5.67 (C*H₂*-N-CO, d, 1H, ²*J*_{HH} = 18 Hz); 6.89 (C*H*, d, 1H, ³*J*_{HH} = 8 Hz); 7.21 (C*H,* t, 1H, ³*J*_{HH} = 8 Hz); 7.29 *(CH,* d, 1H, ³*J*_{HH} = 8 Hz); 7.39 (C*H*, m, 1H); 7.42 (C*H*, m, 1H); 7.45 (C*H*-C-CH₂-P, d, 2H, ³*J*_{HH} = 8 Hz); 7.50 (C*H*-C-N₃, *CH,* m, 3H); 7.55 (*CH,* m, 2H); ¹³C{¹H} *δ* 23.0 (*cycle,* s); 23.3 (*cycle,* s); 24.3 (CH₂, s); 24.6 (CH₂, s); 32.4 (P-*C*H₂-P, t, ¹*J*_{CP} = 80 Hz); 33.3 (*C*H₂, s); 34.1 (CH₂, s); 40.2 (P-*C*H₂-P, d, ¹*J*_{CP} = 89 Hz); 42.9 (*cycle,* s); 44.2 (*cycle,* s); 44.9 (*cycle,* s); 45.4 (*cycle,* s); 46.0 (*cycle,* s); 46.4 (*cycle,* s); 53.6 (N-*C*H₂-P, d, ¹*J*_{CP} = 106 Hz); 55.0 (*cycle,* s); 55.9 (*cycle,* d, ³*J*_{CP} = 11 Hz); 55.9 (C*H*₂-N-CO, s); 116.1-145.2 (4 × *arom.,* 14 × *arom.*)*;* 177.1 (*C*O-N, s); 178.9 (*C*O-O, s); ³¹**P**{**¹**H} *δ* 30.9 (CH₂-*P*-CH₂, m, 1P); 34.5 (CH₂-*P*-CH₂, d, 1P, ²*J*_{PP} = 10 Hz).
**MS:** (-) 818.8 [M-H⁺]⁻. (+) 821.1 [M+H⁺]⁺; 843.0 [M+Na⁺]⁺; 859.0 [M+K⁺]⁺.

### Example 24: Synthesis of targeting conjugate 19

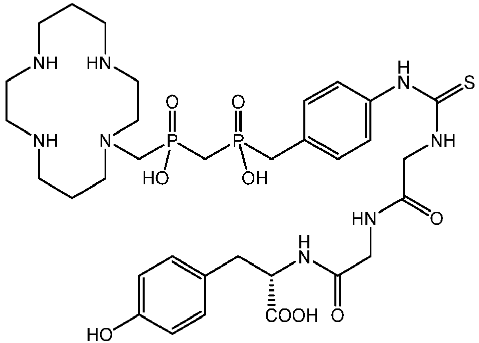

In an NMR tube, **10·**TFA·3.5H₂O (3.4 mg; 3.9 µmol; 1.0 equiv.) was dissolved in D₂O (500 µL). The HGlyGlyTyrOH (1.9 mg; 6.2 µmol; 1.6 equiv.) and aqueous buffer H₃BO₃-LiOH (750 mM in D₂O; pD = 9.3; 413 µL; 310 µmol; 80 equiv.) were then added and the mixture was stirred at room temperature overnight. The crude product was purified by preparative HPLC (C8; H₂O-MeCN). Fraction with product was lyophilized. Product was obtained as a white substance (∼1 mg).
NMR (D₂O): **¹H** *δ* 2.01 (CH₂-C*H*₂-CH₂, m, 2H); 2.06 (CH₂-CH₂-CH₂, m, 2H); 2.12 (P-C*H*₂-P, t, 2H, ²*J*_{HP} = 15 Hz); 2.58-3.56 (*cycle,* N-C*H*₂-P, P-C*H*₂-C, C*H*₂, m, 22H); 3.90 (C*H*₂, m, 2H); 4.21 (C*H*₂, m, 2H); 4.64 (C*H*-N, dd, 1H, ³*J*_{HH} = 9 Hz, ³*J*_{HH} = 5 Hz); 6.85 (C*H*-C-OH, d, 2H, ³*J*_{HH} = 8 Hz); 7.16 (CH-C*H*-CH₂, d, 2H, ³*J*_{HH} = 8 Hz); 7.32 (C*H*-C-NH, d, 2H, ³*J*_{HH} = 8 Hz); 7.38 (C*H*-C-CH₂, d, 2H, ³*J*_{HH} = 8 Hz); ¹³C{¹H} *δ* 23.0 (CH₂-CH₂-CH₂, s); 23.8 (CH₂-CH₂-CH₂, s); 32.4 (P-CH₂-P, t, ¹*J*_{CP} = 80 Hz); 36.5 (*C*H₂, s); 40.1 (P-*C*H₂-C, d, ¹*J*_{CP} = 89 Hz); 42.9 (*C*H₂, s); 43.3 *(cycle,* s); 44.8 *(cycle,* s); 45.6 *(cycle,* s); 46.0 *(cycle,* s); 46.4 (*cycle,* s); 47.0 *(cycle,* s); 48.2 (*C*H₂, s); 53.5 (P-*C*H₂-N, d, ¹*J*_{CP} = 107 Hz); 54.9 (*cycle,* s); 55.1 (*C*H-N, s); 55.8 (*cycle,* d, ³*J*_{CP} = 11 Hz); 116.1 (*C*H-C-OH, s); 126.8 (*C*H-C-NH, s); 129.2 (*C*-CH₂, s); 131.3 (*C*H-C-CH₂, s); 131.5 (*C*H-C-CH₂, s); 134.3 (*C*-CH₂-P, d, ²*J*_{CP} = 6 Hz); 135.8 (C-NH, s); 155.1 (*C*—OH, s); 171.9 (*C*O, s); 173.1 (*C*O, s); 175.7 (*C*O, s); 182.3 (*C*S, s); **³¹P**{¹H} *δ* 31.2 (N-CH₂-*P*, m, 1P); 34.2 (*P*-CH₂-C, d, 1P, ²*J*_{PP} = 8 Hz).
**MS:** (-) 796.8 [M-H⁺]⁻. (+) 799.0 [M+H⁺]⁺.

### Example 25: Synthesis of targeting conjugate 20

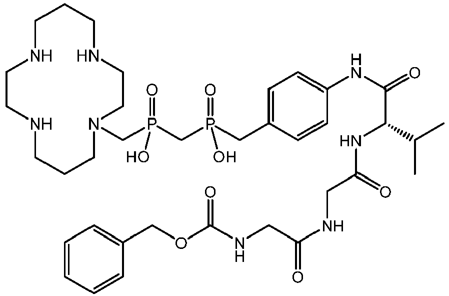

In a glass vial (4 mL), HOValGlyGlyCbz (9.6 mg; 26 µmol; 3.0 equiv.) was dissolved in H₂O-MeCN (2:3; 1.0 mL). Trichlortriazine (1.6 mg; 8.7 µmol; 1.0 equiv.) and pyridine (21.2 µL; 0.26 mmol; 30 equiv.) were subsequently added and the resulting mixture was stirred at room temperature for one hour. Solution of **9**·3H₂O (17.4 mg; 33.8 µmol; 3.9 equiv.) in a mixture of H₂O-MeCN (2:3; 1.0 mL) was then added and the resulting mixture was further stirred at room temperature overnight. The crude product was purified by preparative HPLC (C8; H₂O-MeCN). Fraction with product was lyophilized. Product was obtained as a white substance (∼1 mg).
**NMR** (D₂O): **¹H** *δ* 1.02 (C*H*₃, m, 6H); 2.02 (CH₂-CH₂-CH₂, p, 2H, ³*J*_{HH} = 6 Hz); 2.07 (CH₂-C*H*₂-CH₂, p, 2H, ³*J*_{HH} = 6 Hz); 2.16 (P-C*H*₂-P, t, 2H, ²*J*_{HP} = 16 Hz); 2.20 (CH₃-C*H*-CH₃, m, 1H); 2.34-3.52 (*cycle,* N-C*H*₂-P, P-C*H*₂-C, bm, 20H); 3.91 (C*H*₂, s, 2H); 4.02 (C*H*₂, s, 2H); 4.23 (C*H-*CO*,* d, 1H, ³*J*_{HH} = 8 Hz); 5.15 (Ph-C*H*₂-O, s, 2H); 7.29-7.51 (C*H*-C-N, C*H-*C*-*CH₂*, Ph,* bm, 9H); ¹³C{¹H} *δ* 18.5 (*C*H₃, s); 19.1 (*C*H₃, s); 23.0 (CH₂-CH₂-CH₂, s); 23.6 (CH₂-CH₂-CH₂, s); 31.0 (CH₃-*C*H-CH₃, s); 32.5 (P-CH₂-P, t, ¹*J*_{CP} = 80 Hz); 40.0 (P-CH₂-C, d, ¹*J*_{CP} = 90 Hz); 43.1 (*C*H₂, s); 43.2 *(cycle,* s); 44.5 (*C*H₂, s); 44.6 *(cycle,* s); 45.4 *(cycle,* s); 45.6 *(cycle,* s); 46.3 *(cycle,* s); 46.6 *(cycle,* s); 53.5 (P-CH₂-N, d, ¹*J*_{CP} = 107 Hz); 54.8 *(cycle,* s); 55.8 *(cycle,* d, ³*J*_{CP} = 10 Hz); 61.1 (*C*H-CO, s); 68.1 (Ph-*C*H₂-O, s); 123.3 (*Ph,* s); 128.5 (*C*H-C-NH, s); 129.2 *(Ph,* s); 129.6 *(Ph,* s); 131.2 (*C*H-C-CH₂, d, ³*J*_{CP} = 6 Hz); 132.5 (*C*-CH₂-P, d, ²*J*_{CP} = 8 Hz); 135.4 (C-NH, s); 137.0 *(Ph,* s); 159.4 (*C*O-O, s); 172.3 (*C*O, s); 173.0 (*C*O, s); 173.9 (*C*O, s); **³¹P**{¹H} *δ* 30.1 (N-CH₂-*P*, m, 1P); 34.4 (*P*-CH₂-C, d, 1P, ²*J*_{PP} = 9 Hz).
**MS:** (-) 806.9 [M-H⁺]⁻. (+) 809.1 [M+H⁺]⁺; 831.0 [M+Na⁺]⁺.

### Example 26: Synthesis of targeting conjugate 21

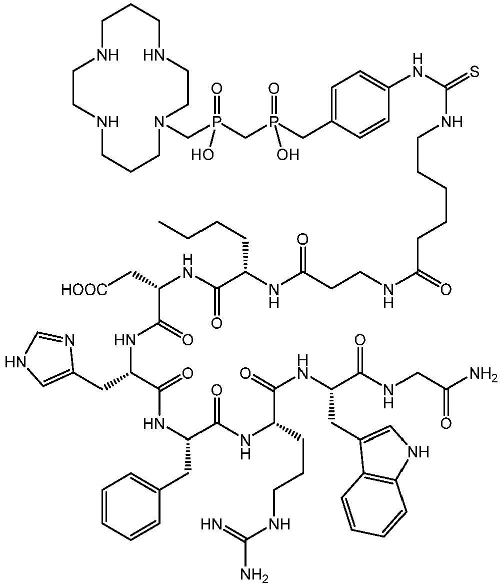

To a glass vial (4 mL), **10**·TFA·3.5H₂O (13.2 mg; 18.5 µmol; 1.7 equiv.) was added. Peptide NH₂(CH₂)₅CONH(CH₂)₂CONleAspHisD-PheArgTrpGlyNH₂ (12.0 mg; 10.8 µmol; 1.0 equiv.) and aqueous buffer H₃BO₃-LiOH (750 mM in D₂O; pD = 9.3; 3.5 mL; 2.63 mmol; 240 equiv.) were subsequently added and the resulting mixture was stirred at room temperature overnight. The crude product was purified by preparative HPLC (C8; H₂O-MeCN). Fraction with product was lyophilized. Product was obtained as a white substance (∼2 mg).
**NMR** (D₂O): **¹H** *δ* 0.83 (C*H*₃, t, 3H, ³*J*_{HH} = 7 Hz); 1.04 (CH₂, bm, 2H); 1.21-1.72 (CH₂, bm, 10H); 1.82-2.23 (P-*CH*₂-P, C*H*₂, *cycle,* bm, 8H); 2.35-3.54 (*cycle,* C*H*₂, P-C*H*₂-N, P-C*H*₂-C, bm, 38H); 3.80 (C*H*₂, m, 2H); 4.12 (*CH,* m, 1H); 4.16 (C*H*, m, 1H); 4.51 (C*H*, m, 1H); 4.55 (C*H*, m, 1H); 4.59 (C*H*, m, 1H); 4.69 (C*H*, m, 1H); 7.06 (CH₂-C-C*H*-N, s, 1H); 7.10-7.27 (*arom.,* m, 7H); 7.28-7.39 (*arom.,* m, 5H); 7.46 *(CH,* d, 1H, ³*J*_{HH} = 7 Hz); 7.63 *(CH,* d, 1H, ³*J*_{HH} = 8 Hz); 8.53 (N-C*H*-N, s, 1H); **³¹P**{¹H} *δ* 32.5 (CH₂-*P*-CH₂, m, 1P); 35.4 (CH₂-*P*-CH₂, m, 1P).
**MS:** (-) 1614.5 [M-H⁺]⁻. (+) 1616.4 [M+H⁺]⁺.

### Example 27: Synthesis of compound 22

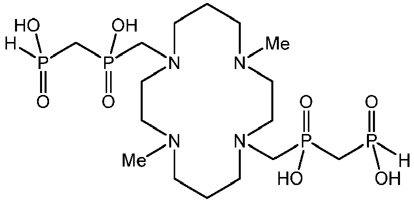

To a glass vial (4 mL), **3**·3.5H₂O (35.6 mg; 79.6 µmol; 1.0 equiv.) was added. Methylene-bis(phosphinic acid) (27.6 mg; 192 µmol; 2.4 equiv.), paraformaldehyde (5.8 mg; 193 µmol; 2.4 equiv.) and aqueous HCl (6 M; 1 mL) were subsequently added and the resulting suspension was stirred overnight at 80 °C. The mixture was evaporated to dryness and the residue was several times co-evaporated with H₂O. The crude product was purified by ion exchange chromatography (DOWEX 50; H⁺-form; H₂O). Fractions with product were combined, evaporated to dryness and further co-evaporated with H₂O. Residue was re-dissolved in H₂O (50 mL) and subsequently lyophilized. Product was obtained as a white substance (21.2 mg; 47%; 1 step, based on **3**·3.5H₂O).
**NMR** (D₂O): **¹H** *δ* 1.71 (CH₂-C*H*₂-CH₂, p, 4H, ³*J*_{HH} = 7 Hz); 2.04 (P-C*H*₂-P, dd, 2H, ²*J*_{HP} = 18 Hz, ²*J*_{HP} = 16 Hz); 2.23 (C*H*₃, s, 6H); 2.51 *(cycle,* t, 4H, ³*J*_{HH} = 7 Hz); 2.57 *(cycle,* m, 4H); 2.75 *(cycle,* m, 4H); 2.81 (N-C*H*₂-P, d, 2H, ²*J*_{HP} = 9 Hz); 2.86 (*cycle,* m, 4H); 7.13 (P*H*, d, ¹*J*_{HP} = 531 Hz); **¹³C**{¹H} *δ* 20.2 (CH₂-*C*H₂-CH₂, s); 35.7 (P-*C*H₂-P, t, ¹*J*_{CP} = 77 Hz); 44.0 (*C*H₃, s); 48.7 *(cycle,* s); 49.9 *(cycle,* d, ³*J*_{CP} = 7 Hz); 51.9 (*cycle,* d, ³*J*_{CP} = 7 Hz); 53.8 *(cycle,* s); 57.0 (N-*C*H₂-P, d, ¹*J*_{CP} = 110 Hz); **³¹P** *δ* 19.8 (*P*H, dtd, 2P, ¹*J*_{PH} = 531 Hz, ²*J*_{PH} = 18 Hz, ²*J*_{PP} = 3 Hz); 32.0 (N-CH-*P*, m, 2P).
**MS:** (-) 539.3 [M-H⁺]⁻. (+) 541.4 [M+H⁺]⁺; 563.4 [M+Na⁺]⁺; 585.4 [M-H⁺+2Na⁺]⁺; 607.4 [M-2H⁺+3Na⁺]⁺.
**TLC** (SiO₂, *i*-PrOH-conc. NH₄OH-H₂O 7:3:3): *R*_{f} = 0.7.
**EA** (C₁₆H₄₀N₄O₈P₄·1.5H₂O, *M*_{R} = 567.4): C 33.9 (33.8); H 7.6 (7.2); N 9.9 (9.6).

### Example 28: Synthesis of compound 23

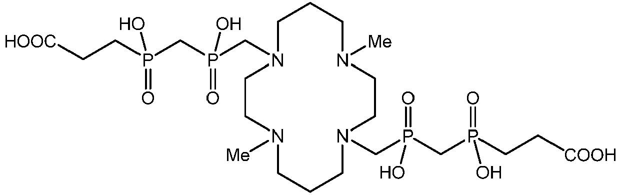

To a glass vial (20 ml), 1,8-dimethyl-1,4,8,11-tetraazacyclotetradecane tetrahydrochloride (295 mg; 788 µmol; 1.0 equiv.) was added. Intermediate **D** (792 mg; 3,66 mmol; 4,6 equiv.), paraformaldehyde (49.7 mg; 1.66 mmol; 2.1 equiv.) and aqueous HCl (6 M; 15 mL) were subsequently added and the resulting suspension was stirred at 80 °C for three days. Mixture was then evaporated to dryness and several times co-evaporated with H₂O. The crude product was purified by column chromatography (SiO₂; EtOH-conc. aq. NH₄OH 1:1; *R*_{f} = 0.5). Fractions with product were joined and evaporated to dryness. Residue was re-dissolved in H₂O (25 mL), treated with charcoal and resulting suspension was filtered through syringe microfilter (Millipore; 0.22 µm). Filtrate was purified by ion exchange chromatography (DOWEX 50; H⁺-form; H₂O). Fractions with product were combined and evaporated to dryness. The residue was re-dissolved in H₂O (500 mL) and subsequently lyophilized. Product was obtained as a white substance (392 mg; 62 %; 1 step, based on 1,8-dimethyl-1,4,8,11-tetraazacyclotetradecane tetrahydrochloride).
**NMR** (D₂O): **¹H** *δ* 1.80 (CH₂-C*H*₂-CH₂, p, 4H, ³*J*_{HH} = 6 Hz); 1.95 (P-C*H*₂-C, m, 4H); 2.10 (P-C*H*₂-P, dd, 4H, ²*J*_{HP} = 17 Hz, ²*J*_{HP} = 15 Hz); 2.25 (C*H*₃, s, 6H); 2.35 (C*H*₂-CO, tm, 4H, ³*J*_{HH} = 8 Hz); 2.49 *(cycle,* t, 4H, ³*J*_{HH} = 7 Hz); 2.51 *(cycle,* m, 2H); 2.58 *(cycle,* m, 4H); 2.63-2.79 (*cycle,* N-C*H*₂-P, m, 10H); ¹³**C**{¹H} *δ* 23.2 (CH₂-CH₂-CH₂, s); 29.2 (P-CH₂-C, d, ¹*J*_{CP} = 97 Hz); 30.8 (*C*H₂-CO, s); 31.0 (P-*C*H₂-P, dd, ¹*J*_{CP} = 79; ¹*J*_{CP} = 76 Hz); 43.8 (*C*H₃, s); 50.2 (*cycle,* d, ³*J*_{CP} = 6 Hz); 50.6 *(cycle,* s); 52.6 *(cycle,* s); 54.2 *(cycle,* s); 55.5 (N-*C*H₂-P, d, ¹*J*_{CP} = 110 Hz); 184.1 (CO, d, ³*J*_{CP} = 17 Hz); **³¹P**{¹H} *δ* 33.8 (CH₂-*P*-CH₂, d, 1P, ²*J*_{PP} = 9 Hz); 34.8 (CH₂-*P*-CH₂, d, 1P, ²*J*_{PP} = 9 Hz).
**MS:** (-) 665.2 [M-H₃O⁺]⁻; 683.3 [M-H⁺]⁻. (+) 685.2 [M+H⁺]⁺; 707.2 [M+Na⁺]⁺; 729.2 [M-H⁺+2Na⁺]⁺; 751.1 [M-2H⁺+3Na⁺]⁺.
**TLC** (SiO₂, EtOH-conc. NH₄OH 1:1): *R*_{f} = 0.5.
**EA** (C₂₂H₄₈N₄O₁₂P₄·3H₂O, *M*_{R} = 738.6): C 35.8 (35.5); H 7.6 (8.0); N 7.6 (7.7).

### Example 29: Synthesis of compound 24

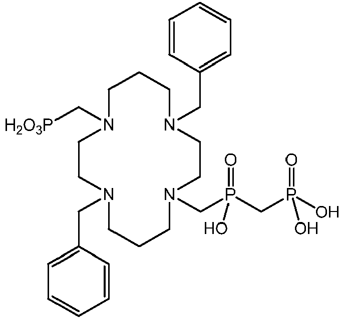

To a glass vial (4 ml), **6**·3.5HCl·H₂O (81.3 mg; 116 µmol; 1.0 equiv.) was added. Phosphorous acid (90.2 mg; 1.10 mmol; 9.5 equiv.), paraformaldehyde (8.2 mg; 273 µmol; 2.4 equiv.) and aqueous HCl (12 M; 1 mL) were subsequently added and the resulting suspension was stirred at 40 °C for five days. The mixture was then evaporated to dryness and several times co-evaporated with H₂O. The residue was purified by ion exchange chromatography (DOWEX 50; H⁺-form; H₂O). Fractions with product were combined and evaporated to dryness. The residue was several times co-evaporated with H₂O and re-dissolved in H₂O (50 mL). The resulting solution was subsequently lyophilized. Product was obtained as a white substance (40.4 mg; 51 %; 1 step, based on **6**·3.5HCl·H₂O).
**NMR** (D₂O): **¹H** *δ* 1.78-1.94 (CH₂-C*H*₂-CH₂, bm, 4H); 2.12 (P-C*H*₂-P, dd, 2H, ²*J*_{HP} = 19 Hz, ²*J*_{HP} = 17 Hz); 2.51-2.60 *(cycle,* m, 2H); 2.63-2.81 *(cycle,* bm, 4H); 2.88-2.98 (*cycle,* N-C*H*₂-P, m, 4H); 3.03-3.44 (*cycle,* N-C*H*₂-P, m, 10H); 3.94 (C*H*₂-Ph, bm, 2H); 4.02 (C*H*₂-Ph, bm, 2H); 6.99-7.30 (*Ph,* m, 8H); **¹³C**{¹H} *δ* 22.6 (CH₂-CH₂-CH₂, s); 27.6 (CH₂-*C*H₂-CH₂, s); 34.8 (P-*C*H₂-P, dd, ¹*J*_{CP} = 117 Hz, ¹*J*_{CP} = 75 Hz); 41.5 (*cycle,* s); 42.4 (*cycle,* s); 50.4 (*cycle,* s); 51.3 (*cycle,* s); 52.3 (*cycle,* s); 52.9 (N-*C*H₂-P, d, ¹*J*_{CP} = 104 Hz); 53.3 (*cycle,* s); 54.8 (*cycle,* s); 56.2 (*C*H₂-Ph, s); 56.9 (*cycle,* d, ³*J*_{CP} = 8 Hz); 58.9 (*C*H₂-Ph, s); 59.0 (N-*C*H₂-P, d, ¹*J*_{CP} = 110 Hz); 123.3 (*Ph,* s); 124.5 (*Ph,* s); 129.0 (*Ph,* s); 129.2 (*Ph,* s); 130.3 (*Ph,* s); 131.4 *(Ph,* s); 137.2 *(Ph,* s); 142.8 *(Ph,* s);
**³¹P**{¹H} 19.2 (HO-*P*-OH, m, 1P); 26.9 (HO-*P*-OH, d, 1P, ²*J*_{PP} = 9 Hz); 34.5 (N-CH-*P*, bm, 1P).
**MS:** (-) 645.0 [M-H⁺]⁻. (+) 647.1 [M+H⁺]⁺; 669.2 [M+Na⁺]⁺; 685.2 [M+K⁺]⁺.
**TLC** (SiO₂, *i*PrOH-conc. NH₄OH-H₂O 7:3:3): *R*_{f} = 0.8.
**EA** (C₂₇H₄₅N₄O₈P₃·2H₂O, *M*_{R} = 682.6): C 47.5 (47.6); H 7.2 (7.2); N 8.2 (8.2).

### Example 30: Synthesis of compound 25

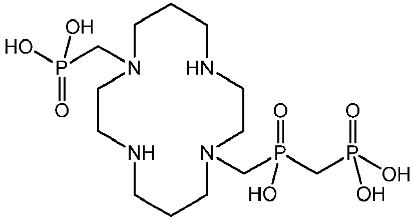

In a glass flask (50 ml), **24**·2H₂O (26.3 mg; 38.5 µmol; 1.0 equiv.) was dissolved in H₂O (25 mL) and the resulting mixture was briefly washed with gaseous argon. Ammonium formate (98.2 mg; 1.56 mmol; 41 equiv.) and Pd@C catalyst (10 %; 9.1 mg) were subsequently added. Resulting suspension was stirred at 60 °C overnight. The mixture was then evaporated to dryness and several times co-evaporated with H₂O. The residue was dissolved in H₂O, the catalyst was filtered off and the filtrate was evaporated to dryness. The residue was purified by ion exchange chromatography (DOWEX 50; H+-form; H₂O). Aqueous fraction with product was evaporated to dryness and several times co-evaporated with H₂O. The residue was re-dissolved in H₂O (50 mL) and lyophilized. Product was obtained as a white substance (15.4 mg; 76%; 1 step, based on **24**·2H₂O).
**NMR** (D₂O): **¹H** *δ* 1.85-1.98 (CH₂-C*H*₂-CH₂, bm, 4H); 2.11 (P-C*H*₂-P, dd, 2H, ²*J*_{HP} = 18 Hz, ²*J*_{HP} = 15 Hz); 2.48-2.84 *(cycle,* bm, 6H); 2.88 *(cycle,* m, 2H); 3.01 (N-C*H*₂-P, d, 2H, ²*J*_{HP} = 12 Hz); 3.08-3.38 (*cycle,* N-C*H*₂-P, m, 10H); **¹³C**{¹H} *δ* 23.1 (CH₂-*C*H₂-CH₂, s); 27.4 (CH₂-*C*H₂-CH₂, s); 34.2 (P-*C*H₂-P, dd, ¹*J*_{CP} = 117 Hz, ¹*J*_{CP} = 76 Hz); 43.8 *(cycle,* s); 42.4 *(cycle,* s); 50.4 *(cycle,* s); 51.3 *(cycle,* s); 52.6 *(cycle,* s); 52.8 (N-*C*H₂-P, d, ¹*J*_{CP} = 107 Hz); 54.1 *(cycle,* s); 55.2 *(cycle,* s); 57.6 (N-*C*H₂-P, d, ¹*J*_{CP} = 111 Hz); 58.2 *(cycle,* d, ³*J*_{CP} = 8 Hz); **³¹P**{¹H} 28.3 (HO-*P*-OH, d, 1P, ²*J*_{PP} = 10 Hz); 32.6 (N-CH-*P*, d, 1P, ²*J*_{PP} = 10 Hz).

**MS:** (-) 465.2 [M-H⁺]⁻. (+) 467.3 [M+H⁺]⁺; 489.2 [M+Na⁺]⁺; 505.2 [M+K⁺]⁺; 511.2 [M-H⁺+2Na⁺]⁺.
**TLC** (SiO₂, *i*PrOH-conc. aq. NH₄OH-H₂O 7:3:3): *R*_{f} = 0.4.
**EA** (C₁₃H₃₃N₄O₈P₃·3.5H₂O, *M*_{R} = 529.4): C 29.5 (29.3); H 7.6 (7.9); N 10.6 (10.7).

### Example 31: Synthesis of compound 26

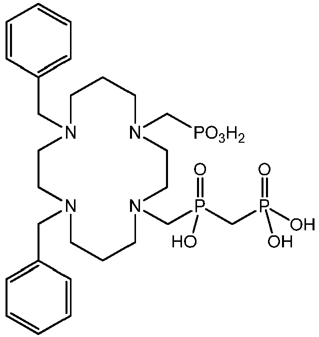

To a glass vial (4 ml), **7**·4H₂O (91.3 mg; 150 µmol; 1.0 equiv.) was added. Phosphorous acid (123 mg; 1.5 mmol; 10 equiv.), paraformaldehyde (10.8 mg; 360 µmol; 2.4 equiv.) and aqueous HCl (12 M; 1 mL) were subsequently added and the resulting suspension was stirred at 40 °C for five days. The mixture was then evaporated to dryness and several times co-evaporated with H₂O. The residue was purified by ion exchange chromatography (DOWEX 50; H⁺-form; H₂O). Fractions with product were joined and evaporated to dryness. The residue was several times co-evaporated with H₂O and re-dissolved in H₂O (50 mL). Resulting solution was subsequently lyophilized. Product was obtained as a white substance (59.4 mg; 58 %; 1 step, based on **7**·4H₂O).
**NMR** (D₂O): 1.72-1.84 (CH₂-C*H*₂-CH₂, bm, 2H); 1.92 (CH₂-C*H*₂-CH₂, m, 1H); 2.05-2.33 (CH₂-C*H*₂-CH₂, P-C*H*₂-P bm, 3H); 2.49-3.24 (*cycle,* N-C*H*₂-P, bm, 20H); 3.82 (C*H*₂-Ph, m, 2H); 4.07 (C*H*₂-Ph, m, 2H); 6.83-6.92 (*Ph,* bm, 3H); 6.98-7.22 *(Ph,* bm, 8H); **³¹P**{¹H} 18.3 (HO-*P*-OH, s, 1P); 22.9 (HO-*P*-OH, d, 1P, ²*J*_{PP} = 9 Hz); 32.4 (N-CH-*P*, bm, 1P).
**MS:** (-) 645.1 [M-H⁺]⁻. (+) 647.4 [M+H⁺]⁺; 669.5 [M+Na⁺]⁺; 685.4 [M+K⁺]⁺.
**TLC** (SiO₂, *i*PrOH-conc. NH₄OH-H₂O 7:3:3): *R*_{f} = 0.8.
**EA** (C₂₇H₄₅N₄O₈P₃·2H₂O, *M*_{R} = 682.6): C 47.2 (47.6); H 7.3 (7.2); N 8.0 (8.2).

### Example 32: Synthesis of compound 27

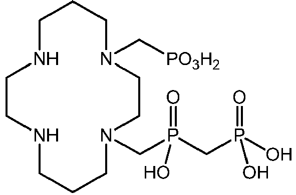

In a glass flask (50 ml), **26**·2H₂O (28 mg; 41 µmol; 1.0 equiv.) was dissolved in H₂O (25 mL) and the resulting mixture was briefly washed with gaseous argon. Ammonium formate (107.8 mg; 1.71 mmol; 45 equiv.) and Pd@C catalyst (10 %; 9.1 mg) were subsequently added. The resulting suspension was stirred at 60 °C overnight. The mixture was then evaporated to dryness and several times evaporated with H₂O. The residue was dissolved in H₂O, the catalyst was filtered off and the filtrate was evaporated to dryness. The residue was purified by ion exchange chromatography (DOWEX 50; H⁺-form; H₂O). Aqueous fraction with product was evaporated to dryness and several times co-evaporated with H₂O. The residue was re-dissolved in H₂O (50 mL) and lyophilized. Product was obtained as a white substance (15.4 mg; 82%; 1 step, based on **26**·2H₂O).
**NMR** (D₂O): **NMR** (D₂O): **¹H** *δ* 1.88 (CH₂-C*H*₂-CH₂, m, 1H); 1.97-2.08 (CH₂-C*H*₂-CH₂, bm, 2H); 2.13-2.35 (P-*CH*₂-P, CH₂-C*H*₂-CH₂, bm, 3H); 2.44-3.38 (*cycle,* N-C*H*₂-P, m, 20H); ¹³C{¹H} *δ* 22.8 (CH₂-*C*H₂-CH₂, s); 25.2 (CH₂-*C*H₂-CH₂, s); 33.1 (P-CH₂-P, dd, ¹*J*_{CP} = 114 Hz, ¹*J*_{CP} = 80 Hz); 42.1 *(cycle,* s); 45.5 *(cycle,* s); 49.9 *(cycle,* s); 51.1 *(cycle,* s); 51.5 *(cycle,* s); 53.9 (N-*C*H₂-P, d, ¹*J*_{CP} = 109 Hz); 54.6 *(cycle,* s); 54.8 *(cycle,* s); 57.6 *(cycle,* s); 59.2 (N-*C*H₂-P, d, ¹*J*_{CP} = 108 Hz); **³¹P**{¹H} 21.3 (HO-*P*-OH, m, 1P); 28.3 (HO-*P*-OH, d, 1P, ²*J*_{PP} = 10 Hz); 32.2 (N-CH-*P*, d, 1P, ²*J*_{PP} = 10 Hz).
**MS:** (-) 465.4 [M-H⁺]⁻. (+) 467.2 [M+H⁺]⁺; 489.5 [M+Na⁺]⁺; 505.5 [M+K⁺]⁺; 511.6 [M-H⁺+2Na⁺]⁺.
**TLC** (SiO₂, *i*PrOH-conc. NH₄OH-H₂O 7:3:3): *R*_{f} = 0.4.
**EA** (C₁₃H₃₃N₄O₈P₃·5H₂O, *M*_{R} = 556.4): C 28.3 (28.1); H 8.6 (8.8); N 9.8 (10.1).

### Example 33: Synthesis of compound 28 - not forming part of the claimed invention

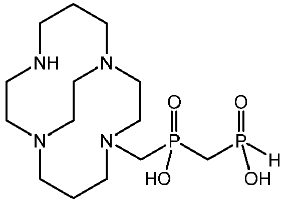

To a glass vial (20 ml), 1,4,8,11-tetraazabicyklo[6.6.2]hexadecane (1.17 g; 5.16 mmol; 2.4 equiv.) was added. Methylene-bis(phosphinic acid) (633 mg; 4.40 mmol; 2.0 equiv.), paraformaldehyde (65.3 mg; 2.17 mmol; 1.0 equiv.) and aqueous HCl (5 M; 20 mL) were subsequently added and the resulting suspension was stirred 80 °C overnight. The mixture was then evaporated to dryness and several times co-evaporated with H₂O. The residue was purified by ion exchange chromatography (Amberlite IRA 402; OH⁻ -form; H₂O → 10 % aqueous AcOH). Acetate fraction with product was evaporated to dryness and several times co-evaporated with H₂O. The residue was dissolved in aqueous HCl (5 M; 10 mL) and the resulting solution was evaporated to dryness. The residue was further dried on vacuum pump and then in vacuum desiccator over P₂O₅. The final product was obtained as a white substance (668 mg; 63%; 1 step; based on paraformaldehyde).
**NMR** (D₂O): **¹H** *δ* 1.70 (CH₂-C*H*₂-CH₂, dm, 1H, ¹*J*_{HH} = 17 Hz); 1.76 (CH₂-C*H*₂-CH₂, dm, 1H, ¹*J*_{HH} = 17 Hz); 2.12 (P-C*H*₂-P, m, 2H); 2.30 (CH₂-C*H*₂-CH₂, m, 1H); 2.30 (CH₂-C*H*₂-CH₂, m, 1H); 2.56 *(cycle,* m, 2H); 2.63 (*cycle,* m, 2H); 2.92 (*cycle,* m, 3H); 3.05 (*cycle,* m, 2H); 3.14 (*cycle,* m, 2H); 3.17 (N-C*H*₂-P, 1H); 3.22 (*cycle,* m, 1H); 3.30 (*cycle,* m, 3H); 3.44 (*cycle,* m, 2H); 3.62 (*cycle,* m, 1H); 3.73 (N-C*H*₂-P, m, 1H); 3.78 (*cycle,* m, 2H); 7.13 (P*H*, d, 1H, ¹*J*_{HP} = 533 Hz); **¹³C**{¹H} *δ* 18.9 (CH₂-*C*H₂-CH₂, s); 20.1 (CH₂-*C*H₂-CH₂, s); 36.0 (P-*C*H₂-P, dd, ¹*J*_{CP} = 84 Hz; ¹*J*_{CP} = 77 Hz); 42.2 *(cycle,* s); 47.7 *(cycle,* s); 49.5 *(cycle,* s); 49.9 *(cycle,* s); 51.7 (*C*H₂-N-CH₂-P, d, ³*J*_{CP} = 6 Hz); 54.0 (N-*C*H₂-P, d, ¹*J*_{CP} = 91 Hz); 54.4 (*cycle,* s); 56.2 (*cycle,* s); 58.2 (*cycle,* s); 58.4 (*cycle,* s); 59.5 (*cycle,* s); **³¹P** *δ* 20.6 (*P*H, dtd, 1P, ¹*J*_{PH} = 533 Hz, ²*J*_{PH} = 17 Hz, ²*J*_{PP} = 6 Hz); 25.7 (*P*-CH₂-N, m, 1P).
**MS:** (-) 381.2 [M-H⁺]⁻; 402.9 [M-2H⁺+Na⁺]⁻. (+) 383.3 [M+H⁺]⁺; 405.3 [M+Na⁺]⁺; 421.2 [M+K⁺]⁺.
**TLC** (SiO₂, *i*PrOH-conc. aq. NH₄OH-H₂O 7:3:3): *R*_{f} = 0.7.
**EA** (C₁₄H₃₂N₄O₄P₂·2.5HCl·H₂O, *M*_{R} = 491.5): C 34.2 (34.4); H 7.5 (7.7); N 11.4 (11.0).

### Example 34: Synthesis of compound 29 - not forming part of the claimed invention

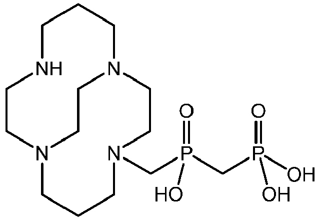

To a glass vial (50 ml), 1,4,8,11-tetraazabicyclo[6.6.2]hexadecane (954 mg; 4.22 mmol; 2.0 equiv.) was added. Intermediate **A**·0.5H₂O (360 mg; 2.13 mmol; 1.0 equiv.), paraformaldehyde (81.9 mg; 2.73 mmol; 1.3 equiv.) and aqueous HCl (5 M; 20 mL) were subsequently added and the resulting suspension was stirred 80 °C overnight. The mixture was then evaporated to dryness and several times co-evaporated with H₂O. The residue was purified by ion exchange chromatography (Amberlite IRA 402; OH⁻-form; H₂O → 10 % aqueous AcOH). Acetate fraction with product was evaporated to dryness and several times co-evaporated with H₂O. The residue was dissolved in aqueous HCl (6 M; 10 mL) and the resulting solution was evaporated to dryness. The residue was further dried on vacuum pump and in later in vacuum desiccator over P₂O₅. The final product was obtained as a white substance (571 mg; 58%; 1 step; based on **A**·0.5H₂O).
**NMR** (D₂O): **¹H** *δ* 1.66 (CH₂-C*H*₂-CH₂, dm, 1H, ¹*J*_{HH} = 17 Hz); 1.75 (CH₂-C*H*₂-CH₂, dm, 1H, ¹*J*_{HH} = 17 Hz); 1.92 (P-C*H*₂-P, m, 2H); 2.20 (CH₂-C*H*₂-CH₂, m, 1H); 2.33 (CH₂-C*H*₂-CH₂, m, 1H); 2.60 *(cycle,* m, 2H); 2.71 *(cycle,* m, 2H); 2.91 *(cycle,* m, 2H); 3.01 *(cycle,* m, 6H); 3.03 (N-C*H*₂-P, 1H); 3.15 *(cycle,* m, 1H); 3.29 *(cycle,* m, 4H); 3.79 *(cycle,* m, 3H); 4.24 (N-C*H*₂-P, m, 1H); ¹³C{¹H} *δ* 19.5 (CH₂-*C*H₂-CH₂, s); 20.6 (CH₂-CH₂-CH₂, s); 33.7 (P-*C*H₂-P, dd, ¹*J*_{CP} = 115 Hz; ¹*J*_{CP} = 88 Hz); 42.3 (*cycle,* s,); 48.8 *(cycle,* s,); 50.0 *(cycle,* s); 50.1 *(cycle,* s); 52.3 (*C*H₂-N-CH₂-P, d, ³*J*_{CP} = 7 Hz); 53.2 *(cycle,* s); 53.4 (N-*C*H₂-P, d, ¹*J*_{CP} = 87 Hz); 55.0 *(cycle,* s); 58.7 *(cycle,* s); 59.1 *(cycle,* s); 59.4 *(cycle,* s); **³¹P**{¹H} *δ* 11.3 (HO-*P*-OH, m, 1P); 27.6 (*P*-CH₂-N, m, 1P).
**MS:** (+) 399.1 [M+H⁺]⁺; 421.0 [M+Na⁺]⁺; 437.2 [M+K⁺]⁺.
**TLC** (SiO₂, *i*PrOH-conc. aq. NH₄OH-H₂O 7:3:3): *R*_{f} = 0.2.
**EA** (C₁₄H₃₂N₄O₅P₂·1.5HCl·0.5H₂O, *M*_{R} = 462.1): C 36.4 (36.2); H 7.5 (7.5); N 12.1 (12.0).

### Example 35: Synthesis of compound 30

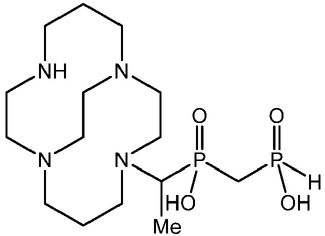

To a glass vial (4 mL), 1,4,8,11-tetraazabicyclo[6.6.2]hexadecane (298 mg; 1.32 mmol; 4.5 equiv.) was added. Methylene-bis(phosphinic acid) (93.5 mg; 649 µmol; 2.2 equiv.), acetaldehyde (13.0 mg; 295 µmol; 1.0 equiv.) and aqueous HCl (6 M; 20 mL) were subsequently added and the resulting suspension was stirred 60 °C overnight. The mixture was then evaporated to dryness and several times co-evaporated with H₂O. The residue was purified by ion exchange chromatography (DOWEX 50; H⁺-form; H₂O → 10 % aqueous pyridine). Pyridine fraction with product was evaporated to dryness and several times co-evaporated with H₂O. The residue was re-dissolved in H₂O (50 mL) and subsequently lyophilized. The final product was obtained as a white substance (62.3 mg; 51 %; 1 step; based on acetaldehyde).
**NMR** (D₂O): **¹H** *δ* 1.19 (C*H*₃, dd, 3H, ³*J*_{HP} = 15 Hz, ²*J*_{HH} = 6 Hz); 1.72 (CH₂-C*H*₂-CH₂, dm, 1H, ¹*J*_{HH} = 16 Hz); 1.80 (CH₂-C*H*₂-CH₂, dm, 1H, ¹*J*_{HH} = 17 Hz); 2.15 (P-C*H*₂-P, t, 2H, ²*J*_{HP} = 16 Hz); 2.27-2.39 (CH₂-C*H*₂-CH₂, m, 2H); 2.48 *(cycle,* m, 2H); 2.68 (*cycle,* m, 2H); 2.95-3.04 *(cycle,* m, 4H); 3.21-3.37 *(cycle,* bm, 11H) 3.51 (C*H*-CH₃, dq, 1H, ²*J*_{HP} = 14 Hz, ²*J*_{HH} = 6 Hz); 3.62 *(cycle,* m, 2H); 3.78 *(cycle,* m, 1H); 7.15 (P*H*, d, 1H, ¹*J*_{HP} = 533 Hz); **¹³C**{¹H} 8.9 (*C*H₃, s); *δ* 22.1 (CH₂-*C*H₂-CH₂, s); 23.5 (CH₂-*C*H₂-CH₂, s); 36.7 (P-*C*H₂-P, dd, ¹*J*_{CP} = 81 Hz; ¹*J*_{CP} = 75 Hz); 41.6 *(cycle,* s); 46.2 *(cycle,* s); 49.7 *(cycle,* s); 50.9 (cycle, d, ³*J*_{CP} = 6 Hz); 51.2 *(cycle,* s); 53.5 (N-*C*H₂-P, d, ¹*J*_{CP} = 92 Hz); 54.1 (*cycle,* s); 55.5 (*cycle,* s); 56.8 *(cycle,* s); 57.2 *(cycle,* s); 59.9 *(cycle,* s); **³¹P** *δ* 19.8 (*P*H, dtm, 1P, ¹*J*_{PH} = 533 Hz, ²*J*_{PH} = 16 Hz); 29.2 (*P*-CH₂-N, m, 1P).
**MS:** (-) 394.9 [M-H⁺]⁻. (+) 396.9 [M+H⁺]⁺; 419.0 [M+Na⁺]⁺; 441.0 [M-H⁺+2Na⁺]⁺.
**TLC** (SiO₂, EtOH-conc. aq. NH₄OH 2:1): *R*_{f} = 0.7.
**EA** (C₁₅H₃₄N₄O₄P₂·H₂O, *M*_{R} = 414.2): C 43.5 (43.8); H 8.8 (8.5); N 13.5 (13.5).

### Example 36: Synthesis of compound 31

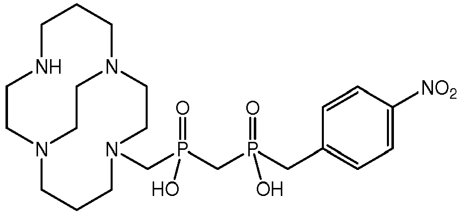

To a glass vial (20 mL), 1,4,8,11-tetraazabicyclo[6.6.2]hexadecane (1.12 g; 4.97 µmol; 1.6 equiv.) was added. Intermediate **B** (856 mg; 3.07 mmol; 1.0 equiv.), paraformaldehyde (92.4 mg; 3.08 mmol; 1.0 equiv.) and aqueous HCl (6 M; 15 mL) were subsequently added and the resulting suspension was stirred 80 °C overnight. The mixture was then evaporated to dryness and several times co-evaporated with H₂O. The residue was purified by ion exchange chromatography (DOWEX 50; H⁺-form; H₂O → 10% aqueous pyridine). Pyridine fraction with product was evaporated to dryness and several times co-evaporated with H₂O. The crude product was purified by flash chromatography (C18; gradient elution H₂O--MeCN). Fractions with pure product were combined and evaporated to dryness. The residue was re-dissolved in H₂O (250 mL) and subsequently lyophilized. The final product was obtained as a white substance (1.04 g; 55 %; 1 step; based on intermediate **B**).
**NMR** (D₂O): **¹H** *δ* 1.69 (CH₂-C*H*₂-CH₂, m, 1H); 1.77 (CH₂-C*H*₂-CH₂, m, 1H); 2.10 (P-C*H*₂-P, m, 2H); 2.27 (CH₂-C*H*₂-CH₂, m, 1H); 2.39 (CH₂-C*H*₂-CH₂, m, 1H); 2.59 *(cycle,* m, 2H); 2.64 *(cycle,* m, 2H); 2.85-3.07 *(cycle,* m, 5H); 3.07-3.35 (c*ycle,* P-C*H*₂-C, N-C*H*₂-P, m, 9H); 3.38 *(cycle,* m, 1H); 3.38 (*cycle,* m, 1H); 3.68 *(cycle,* m, 1H); 3.74-3.88 (*cycle,* N-C*H*₂-P, m, 3H); 7.51 (C*H*-C-CH₂, dd, 2H, ²*J*_{HH} = 9 Hz, ⁴*J*_{HP} = 2 Hz); 8.19 (C*H*-C-N, d, 2H, ²*J*_{HH} = 9 Hz); ¹³**C**{¹H} *δ* 19.0 (CH₂-*C*H₂-CH₂, s); 20.2 (CH₂-CH₂-CH₂, s); 33.3 (P-CH₂-P, dd, ¹*J*_{CP} = 85 Hz, ¹*J*_{CP} = 82 Hz); 41.1 (P-CH₂-C, dd, ¹*J*_{CP} = 86 Hz, ³*J*_{CP} = 3 Hz); 42.2 (*cycle,* s); 47.9 (*cycle,* s); 49.6 (*cycle,* s); 49.9 (*cycle,* s); 51.8 (*cycle,* d, ³*J*_{CP} = 7 Hz); 54.1 (*cycle,* s); 55.2 (P-*C*H₂-N, d, ¹*J*_{CP} = 90 Hz); 58.4 (*cycle,* s); 58.7 (*cycle,* s); 59.5 (*cycle,* s); 124.4 (*C*H-C-N, s); 131.3 (*C*H-C-CH₂, d, ³*J*_{CP} = 5 Hz); 143.9 (CH-*C*-CH₂, d, ²*J*_{CP} = 8 Hz); 146.8 (CH-*C*-N, s); **³¹P**{¹H} *δ* 23.3 (CH₂-*P*-CH₂, d, 1P, ²*J*_{PP} = 11 Hz); 31.1 (CH₂-*P*-CH₂, d, 1P, ²*J*_{PP} = 11 Hz).
**MS:** (-) 516.2 [M-H⁺]⁻.
**TLC** (SiO₂, EtOH-conc. aq. NH₄OH 4:1): *R*_{f} = 0.6.
**EA** (C₂₁H₃₇N₅O₆P₂·5.5H₂O, *M*_{R} = 616.5): C 40.9 (40.8); H 7.9 (7.6); N 11.4 (11.7).

### Example 37: Synthesis of compound 32

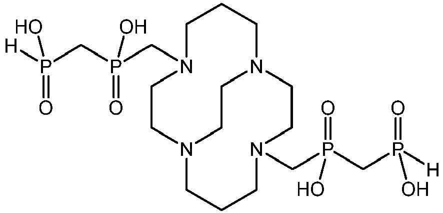

To a glass flask (25 mL), 1,4,8,11-tetraazabicyclo[6.6.2]hexadecane (214 mg; 945 µmol; 1.0 equiv.) was added. Methylene-bis(phosphinic acid) (835 mg; 5.80 mmol; 5.5 equiv.), paraformaldehyde (63.5 mg; 2.12 mmol; 2.0 equiv.) and aqueous HCl (6 M; 5 mL) were subsequently added and the resulting suspension was stirred at 80 °C for two days. The mixture was then evaporated to dryness and several times co-evaporated with H₂O. The residue was purified by ion exchange chromatography (DOWEX 50; H⁺-form; H₂O). Fractions with product were joined and evaporated to dryness. The residue was re-dissolved in H₂O (200 mL) and subsequently lyophilized. The final product was obtained as a white substance (392 mg; 68 %; 1 step; based on 1,4,8,11-tetraazabicyclo[6.6.2]hexadecane).
**NMR** (D₂O): **¹H** *δ* 1.67 (CH₂-C*H*₂-CH₂, bm, 2H); 1.82 (CH₂-C*H*₂-CH₂, bm, 2H); 1.99 (P-C*H*₂-P, t, 4H, ²*J*_{HP} = 17 Hz); 2.78-3.19 (N-C*H*₂, bm, 20H); 3.26 (N-C*H*₂, bm, 2H); 3.62 (N-C*H*₂, bm, 2H); 7.14 (P*H*, d, 2H, ¹*J*_{HP} = 530 Hz); **¹³C**{¹H} *δ* 24.4 (CH₂-CH₂-CH₂, s); 37.1 (P-CH₂-P, dd, ¹*J*_{CP} = 76 Hz, ¹*J*_{CP} = 72 Hz); 52.4 *(cycle,* s); 52.8 *(cycle,* s); 53.8 (*C*H₂-N-CH₂-P, d, ³*J*_{CP} = 4 Hz); 53.8 (N-*C*H₂-P, d, ¹*J*_{CP} = 100 Hz); 56.5 *(cycle,* s); 57.1 (*cycle,* s); **³¹P**{¹H} *δ* 19.8 (*P*H, dt, 2P, ¹*J*_{PH} = 530 Hz, ²*J*_{PH} = 18 Hz); 32.5 (*P*-CH₂-N, t, 2P, ²*J*_{PH} = 15 Hz).
**MS:** (-) 536.7 [M-H⁺]⁻.
**TLC** (SiO₂, *i*PrOH-conc. aq. NH₄OH-H₂O 7:3:3): *R*_{f} = 0.4.
**EA** (C₁₆H₃₈N₄O₈P₄·4H₂O, *M*_{R} = 610.5): C 31.5 (31.3); H 7.6 (7.4); N 9.2 (9.2).

### Example 38: Synthesis of compound 33

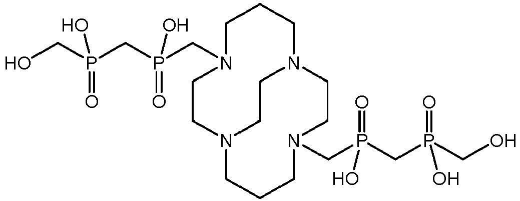

To a glass vial (4 mL), **32**·4H₂O (59.8 mg; 98 µmol; 1.0 equiv.) was added and it was followed by addition of paraformaldehyde (13.6 mg; 453 µmol; 4.6 equiv.) and aqueous HCl (6 M; 10 mL). The resulting suspension was stirred at 80 °C overnight. On the next day, the mixture was evaporated to dryness and several times co-evaporated with H₂O. The crude product was purified by ion exchange chromatography (DOWEX 50; H⁺-form; H₂O). Fractions with product were combined and evaporated to dryness. The residue was re-dissolved in H₂O (25 mL) and subsequently lyophilized. The final product was obtained as a white substance (51.3 mg; 80 %; 1 step; based on **32**·4H₂O).
**NMR** (D₂O): **¹H** *δ* 1.82 (CH₂-C*H*₂-CH₂, m, 2H); 2.06 (CH₂-C*H*₂-CH₂, m, 2H); 2.30 (P-C*H*₂-P, t, 4H, ²*J*_{HP} = 16 Hz); 2.67-3.63 (*cycle,* N-C*H*₂-P, bm, 24H); 3.67 (O-C*H*₂-P, d, 4H, ²*J*_{HP} = 5 Hz); **¹³C**{¹H} *δ* 22.1 (CH₂-CH₂-CH₂, s); 34.2 (P-CH₂-P, dd, ¹*J*_{CP} = 84 Hz, ¹*J*_{CP} = 73 Hz); 51.4 *(cycle,* s); 52.8 *(cycle,* s); 53.6 (*C*H₂-N-CH₂-P, d, ³*J*_{CP} = 4 Hz); 54.9 (*cycle,* s); 55.2 (N-*C*H₂-P, d, ¹*J*_{CP} = 98 Hz); 57.8 (*cycle,* s); 61.3 (P-*C*H₂-O, d, ³*J*_{CP} = 108 Hz) **³¹P**{¹H} *δ* 27.6 (*P*-CH₂-N, bm, 2P); 40.6 (*P*-CH₂-O, m, 2P).
**MS:** (-) 596.8 [M-H⁺]⁻.
**TLC** (SiO₂, *i*PrOH-conc. aq. NH₄OH-H₂O 7:3:3): *R*_{f} = 0.5.
**EA** (C₁₈H₄₂N₄O₁₀P₄·3H₂O, *M*_{R} = 652.5): C 33.1 (33.4); H 7.4 (7.5); N 8.6 (8.3).

### Example 39: Synthesis of compound 34

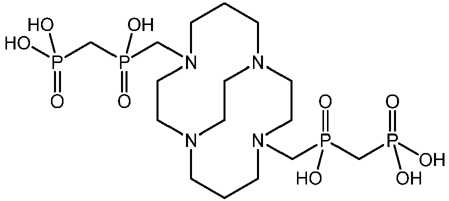

To a glass flask (20 mL), **32**·4H₂O (91.6 mg; 150 µmol; 1.0 equiv.) was added. Solution of HgCl₂ (173 mg; 638 µmol; 4.3 equiv.) in aqueous HCl (2 M; 5 mL) was added and the mixture was stirred at 60 °C for two days. Precipitate was filtered off and the mother liquor was saturated with H₂S. Precipitate was centrifuged off, washed with H₂O and the supernatant was evaporated to dryness. The residue was re-dissolved in aqueous HCl (3 %; 300 µL) followed by addition of excess of *i*PrOH (25 mL). On the next day, mother liquor was decanted off and the oily residue was several times co-evaporated with H₂O. The residue was re-dissolved in H₂O (100 mL) and subsequently lyophilized. Product was obtained as a white substance (68.4 mg; 68 %; 1 step, based on **32**·4H₂O).
**NMR** (D₂O): **¹H** *δ* 1.68 (CH₂-C*H*₂-CH₂, bm, 2H); 1.81 (CH₂-C*H*₂-CH₂, bm, 2H); 1.92 (P-C*H*₂-P, t, 4H, ²*J*_{HP} = 18 Hz); 2.75-3.21 (N-C*H*₂, bm, 20H); 3.27 (N-C*H*₂, bm, 2H); 3.66 (N-C*H*₂, bm, 2H); ¹³**C**{¹H} *δ* 24.5 (CH₂-CH₂-CH₂, s); 35.1 (P-*C*H₂-P, dd, ¹*J*_{CP} = 117 Hz, ¹*J*_{CP} = 75 Hz); 52.5 *(cycle,* s); 52.9 *(cycle,* s); 53.3 (N-*C*H₂-P, bm); 53.7 (*C*H₂-N-CH₂-P, bm); 56.7 *(cycle,* s); 57.0 *(cycle,* s); **³¹P**{¹H} *δ* 12.4 (HO-*P*-OH, d, 2P, ²*J*_{PP} = 7 Hz); 36.7 (*P*-CH₂-N, d, 2P, ²*J*_{PP} = 7 Hz).
**TLC** (SiO₂, *i*PrOH-conc. aq. NH₄OH-H₂O 7:3:3): *R*_{f} = 0.1.
**MS:** (-) 569.2 [M-H⁺]⁻. (+) 571.4 [M+H⁺]⁺; 593.4 [M+Na⁺]⁺; 609.4 [M+K⁺]⁺.
**EA** (C₁₆H₃₈N₄O₁₀P₄·2.5HCl·H₂O, *M*_{R} = 679,6): C 28.3 (28.4); H 6.3 (6.2); N 8.2 (8.3).

### Example 40: Synthesis of compound 35

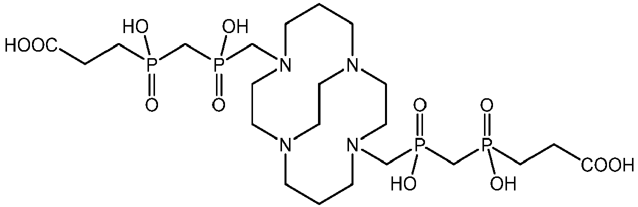

To a glass vial (20 mL), 1,4,8,11-tetraazabicyclo[6.6.2]hexadecane (148 mg; 653 µmol; 1.0 equiv.) was added. Intermediate **D** (688 mg; 3.18 mmol; 4.9 equiv.), paraformaldehyde (48.6 mg; 1.62 mmol; 2.5 equiv.) and aqueous HCl (12 M; 10 mL) were subsequently added and the resulting suspension was stirred at 80 °C for two days. The mixture was then evaporated to dryness and several times co-evaporated with H₂O. The residue was purified by column chromatography (SiO₂; EtOH-conc. aq. NH₄OH 1:1; *R*_{f} = 0.4). Fractions with product were combined and evaporated to dryness. The residue was re-dissolved in H₂O (25 mL), treated with small amounts of charcoal and filtered through syringe microfilter (Millipore; 0.22 µm). The filtrate was further purified by ion exchange chromatography (DOWEX 50; H⁺-form; H₂O). Fractions with product were combined and evaporated to dryness. The residue was re-dissolved in H₂O (125 mL) and subsequently lyophilized. The final product was obtained as a white substance (272 mg; 58 %; 1 step; based on 1,4,8,11-tetraazabicyclo [6.6.2] hexadecane).
**NMR** (D₂O): **¹H** *δ* 1.72 (CH₂-C*H*₂-CH₂, m, 2H); 1.74 (CH₂-C*H*₂-CH₂, m, 2H); 1.94 (P-C*H*₂-P, P-C*H*₂-C, m, 8H); 2.48 (C*H*₂-CO, t, 4H, ³*J*_{HH} = 7 Hz); 2.92-3.31 (*cycle,* N-C*H*₂, bm, 22H); 3.52 (N-C*H*₂, bm, 2H); ¹³**C**{¹H} *δ* 24.4 (CH₂-*C*H₂-CH₂, s); 26.8 (P-CH₂-C, d, ¹*J*_{CP} = 94 Hz); 31.0 (*C*H₂—CO, s); 34.9 (P-*C*H₂-P, dd, ¹*J*_{CP} = 76 Hz, ¹*J*_{CP} = 74 Hz); 52.4 *(cycle,* s); 52.8 *(cycle,* s); 54.2 (N-*C*H₂-P, d, ¹*J*_{CP} = 102 Hz); 54.4 (*cyclus,* d, ³*J*_{CP} = 5 Hz); 57.1 *(cycle,* s); 57.3 *(cycle,* s); 182.8 (CO, d, ³*J*_{CP} = 15 Hz); **³¹P**{¹H} *δ* 30.4 (CH₂-*P*-CH₂, d, 2P, ²*J*_{PP} = 10 Hz); 33.7 (CH₂-*P*-CH₂, d, 2P, ²*J*_{PP} = 10 Hz).
**MS:** (-) 663.3 [M-H₃O⁺]⁻; 681.3 [M-H⁺]⁻. (+) 683.2 [M+H⁺]⁺; 727.2 [M-H⁺ +2Na⁺]⁺.
**TLC** (SiO₂, EtOH-conc. NH₄OH 1:1): *R*_{f} = 0.3.
**EA** (C₂₂H₄₆N₄O₁₂P₄·2H₂O, *M*_{R} = 718.6): C 36.8 (36.9); H 7.0 (7.3); N 7.8 (7.5).

### Example 41: Synthesis of compound 36

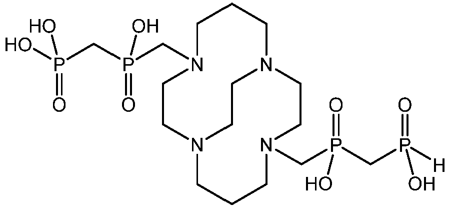

To a glass vial (4 mL), **29**·1.5HCl·0.5H₂O (51.3 mg; 111 µmol; 1.0 equiv.) was added. Methylene-bis(phosphinic acid) (59.2 mg; 411 µmol; 3.7 equiv.), paraformaldehyde (4.9 mg; 163 µmol; 1.5 equiv.) and aqueous HCl (12 M; 1 mL) were subsequently added and the resulting suspension was stirred at 80 °C for two days. The mixture was then evaporated to dryness and several times co-evaporated with H₂O. The residue was purified by ion exchange chromatography (DOWEX 50; H⁺-form; H₂O). Fractions with product were combined and evaporated to dryness. The residue was dried in vacuum pump and subsequently in vacuum desiccator over P₂O₅. Resulting product was obtained as a white substance (35.0 mg; 55 %; 1 step; based on **29**·1.5HCl·0.5H₂O).
**NMR** (D₂O): **¹H** *δ* 1.83-2.11 (CH₂-C*H₂*-CH₂, bm, 4H); 2.24 (P-*CH*₂-P, t, 2H, ²*J*_{HP} = 15 Hz); 2.37 (CH₂-C*H₂*-CH₂, dd, 2H, ²*J*_{HP} = 18 Hz, ²*J*_{HP} = 16 Hz); 2.64-3.44 (*cycle,* N-C*H*₂-P, bm, 24H); **¹³C**{¹H} *δ* 22.2 (CH₂-CH₂-CH₂, s); 23.7 (CH₂-*C*H₂-CH₂, s); 34.2 (P-*C*H₂-P, dd, ¹*J*_{CP} = 122 Hz, ¹*J*_{CP} = 82 Hz); 36.3 (P-CH₂-P, t, ¹*J*_{CP} = 82 Hz); 49.3-57.4 (12 × N-*C*H₂, bm); **³¹P** *δ* 19.0 (HO-*P*-OH, m, 1P); 24.3 (*P*H, dm, 1P, ¹*J*_{PH} = 533 Hz); 26.7 (*P*-CH₂-N, bm, 1P); 27.8 (*P*-CH₂-N, bm, 1P).
**MS:** (-) 553.1 [M-H⁺]⁻. (+) 555.2 [M+H⁺]⁺; 577.2 [M+Na⁺]⁺.
**TLC** (SiO₂, EtOH-conc. aq. NH₄OH 1:1): *R*_{f} = 0.3.
**EA** (C₁₆H₃₈N₄O₉P₄·H₂O, *M*_{R} = 572.4): C 33.6 (33.4); H 7.0 (6.7); N 9.8 (9.9).

### Example 42: Synthesis of compound 37

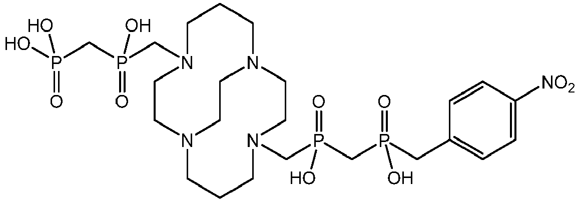

To a glass vial (4 mL), **29**·1.5HCl·0.5H₂O (403 mg; 872 µmol; 1.0 equiv.) was added. Intermediate **B** (294 mg; 1.05 mmol; 1.2 equiv.), paraformaldehyde (39.3 mg; 1.33 mmol; 1.5 equiv.) and aqueous HCl (12 M; 5 mL) were subsequently added and the resulting suspension was stirred at 80 °C for three days. The mixture was then evaporated to dryness and several times co-evaporated with H₂O. The residue was purified was purified by flash chromatography (C18; gradient elution H₂O-0.1 % TFA-MeCN). Fractions with pure product were combined and evaporated to dryness. The residue was re-dissolved in H₂O (250 mL) and subsequently lyophilized. Resulting product was obtained as a yellow substance (417 mg; 62 %; 1 step; based on **29**·1.5HCl·0.5H₂O).
**NMR** (D₂O): **¹H** *δ* 2.02 (CH₂-*CH₂*-CH₂, bm, 2H); 2.32 (CH₂-*CH₂*-CH₂, bm, 2H); 2.40 (P-*CH*₂-P, t, 2H, ²*J*_{HP} = 16 Hz); 2.48 (P-*CH*₂-P, dd, 2H, ²*J*_{HP} = 20 Hz, ²*J*_{HP} = 17 Hz); 2.79-3.98 (*cycle,* N-C*H*₂-P, P-C*H*₂-C, bm, 26H); 7.57 (C*H,* dd, 2H, ³*J*_{HH} = 9 Hz, ⁴*J*_{HP} = 2 Hz); 8.24 (C*H*, d, 2H, ³*J*_{HH} = 9 Hz); ¹³C{¹H} *δ* 20.5 (CH₂-CH₂-CH₂, s); 21.0 (CH₂-(*C*H₂-CH₂, s); 32.2 (P-*C*H₂-P, dd, ¹*J*_{CP} = 124 Hz, ¹*J*_{CP} = 84 Hz); 33.2 (P-*C*H₂-P, t, ¹*J*_{CP} = 82 Hz); 39.3 (P-*C*H₂-C, d, ¹*J*_{CP} = 89 Hz); 48.0-59.6 (12 × N-*C*H₂, bm); 124.5 (*C*H-C-N, d, ⁴*J*_{CP} = 3 Hz); 131.5 (*C*H-C-CH₂, d, ³*J*_{CP} = 6 Hz); 141.8 (CH-*C*-CH₂, d, ²*J*_{CP} = 9 Hz); 147.2 (CH-*C*-N, s); ³¹**P**{¹H} *δ* 18.0 (HO-*P*-OH, m, 1P); 24.0 (*P*-CH₂-N, bm, 1P); 25.6 (*P*-CH₂-N, bm, 1P); 39.0 (*P*-CH₂-C, m, 1P).
**MS:** (-) 687.8 [M-H⁺]⁻; 709.7 [M-2H⁺+Na⁺]⁻; 725.7 [M-2H⁺+K⁺]⁻. (+) 689.9 [M+H⁺]⁺; 711.9 [M+Na⁺]⁺; 727.8 [M+K⁺]⁺; 749.8 [M-H⁺+Na⁺+K⁺]⁺.
**TLC** (SiO₂, *i*PrOH-conc. aq. NH₄OH-H₂O 7:3:3): *R*_{f} = 0.3.
**EA** (C₂₃H₄₃N₅O₁₁P₄·4.5H₂O, *M*_{R} = 770.6): C 28.3 (28.4); H 6.3 (6.2); N 8.2 (8.3).

### Example 43: Synthesis of compound 38

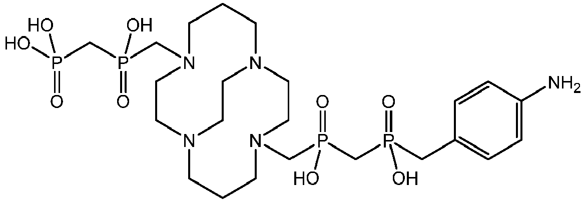

In a glass flask (250 ml), **37**·4.5H₂O (288 mg; 374 µmol) was dissolved in H₂O (100 mL) and the resulting mixture was briefly washed with gaseous argon. Pd@C catalyst (10 %; 44 mg) was subsequently added and the resulting suspension was then treated with gaseous H₂ at 60 °C overnight. The catalyst was then filtered off and the filtrate was evaporated to dryness. The residue was re-dissolved in H₂O (250 mL) and subsequently lyophilized. Resulting product was obtained as a yellowish substance (257 mg; 94 %; 1 step; based on **37**·4.5H₂O.
**NMR** (D₂O): ¹**H** *δ* 1.80 (CH₂-C*H*₂-CH₂, bm, 2H); 1.86 (CH₂-C*H*₂-CH₂, bm, 2H); 2.10 (P-C*H*₂-P, m, 2H); 2.21 (P-C*H*₂-P, m, 2H); 2.65-3.77 (*cycle,* N-C*H*₂-P, P-C*H*₂-C, bm, 26H); 6.97 (C*H,* d, 2H, ³*J*_{HH} = 8 Hz); 7.25 (C*H,* d, 2H, ³*J*_{HH} = 8 Hz); ¹³C{¹H} *δ* 20.1 (CH₂-*C*H₂-CH₂, s); 20.5 (CH₂-*C*H₂-CH₂, s); 32.7 (P-CH₂-P, m); 33.4 (P-*C*H₂-P, m; 33.6 (P-*C*H₂-C, d, ¹*J*_{CP} = 91 Hz); 46.7-57.2 (12 × N-*C*H₂, bm); 123.0 (CH-*C*-N, s); 118.2 (*C*H-C-N, m); 130.8 (*C*H-C-CH₂, d, ³*J*_{CP} = 5 Hz); 140.3 (CH-*C*-CH₂, m); ³¹**P**{¹H} *δ* 13.5 (HO-*P*-OH, bm, 1P); 24.6 (*P*-CH₂-N, bm, 1P); 23.6 (*P*-CH₂-N, bm, 1P); 32.6 (*P*-CH₂-C, bm, 1P). **MS:** (-) 658.0 [M-H⁺]⁻; 679.9 [M-2H⁺+Na⁺]⁻. (+) 659.9 [M+H⁺]⁺; 981.9 [M+Na⁺]⁺; 697.8 [M+K⁺]⁺.
**TLC** (SiO₂, *i*PrOH-conc. aq. NH₄OH-H₂O 7:3:3): *R*_{f} = 0.3.
**EA** (C₂₃H₄₅N₅O₉P₄·4H₂O, *M*_{R} = 731.6): C 37.8 (37.6); H 7.3 (7.2); N 9.6 (9.5).

### Example 44: Synthesis of compound 39

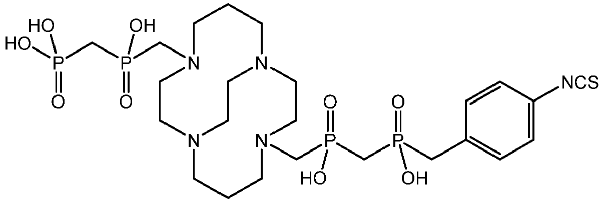

In a glass flask (100 mL), **38**·4H₂O (48.3 mg; 66,0 µmol, 1.0 equiv.) was dissolved in H₂O (20 mL). Freshly prepared solution of CSCl2 (15 mM; 20 mL; 300 µmol; 4.5 equiv.) in CCl₄ was then added and the resulting biphasic mixture was vigorously stirred at room temperature overnight. The organic layer was separated off and the aqueous layer was evaporated to dryness. The crude product was purified by preparative HPLC (C8; gradient elution H₂O-0.1% TFA-MeCN). Fraction with product was directly lyophilized. Product was obtained as a white substance (35.2 mg; 67 %; 1 step; based on **38**·4H₂O).
**NMR** (D₂O): ¹**H** *δ* 2.02 (CH₂-C*H*₂-CH₂, bm, 2H); 2.29 (P-C*H*₂-P, t, 2H, ²*J*_{HP} = 16 Hz); 2.32 (CH₂-C*H*₂-CH₂, bm, 2H); 2.37 (P-C*H*₂-P, t, 2H, ²*J*_{HP} = 19 Hz); 2.86-3.92 (*cycle,* N-C*H*₂-P, P-C*H*₂-C, bm, 26H); 7.37 (*CH,* d, 2H, ³*J*_{HH} = 8 Hz); 7.39 (C*H*, m, 2H); ¹³C{¹H} 20.8 (CH₂-*C*H₂-CH₂, s); 21.3 (CH₂-*C*H₂-CH₂, s); 31.7 (P-*C*H₂-P, dd, ¹*J*_{CP} = 120 Hz, ¹*J*_{CP} = 83 Hz); 32.3 (P-*C*H₂-P, t, ¹*J*_{CP} = 82 Hz); 38.4 (P-*C*H₂-C, d, ¹*J*_{CP} = 91 Hz); 46.6-59.1 (12 × N-*C*H₂, bm); 126.7 (*C*H-C-N, s); 130.1 (CH-*C*-N, s); 131.8 (*C*H-C-CH₂, s); 132.2 (CH-*C*-CH₂, d, ²*J*_{CP} = 8 Hz); 134.8 (CS, s); ³¹**P**{¹H} *δ* 16.3 (HO-*P*-OH, m, 1P); 23.4 (*P*-CH₂-N, bm, 1P); 23.6 (*P*-CH₂-N, bm, 1P); 38.3 (*P*-CH₂-C, m, 1P).
**MS:** (-) 699.6 [M-H⁺]⁻; 721.6 [M-2H⁺+Na⁺]⁻; 737.6 [M-2H⁺+K⁺]⁻. (+) 701.8 [M+H⁺]⁺; 723.8 [M+Na⁺]⁺; 739.8 [M+K⁺]⁺.
**EA** (C₂₄H₄₃N₅O₉P₄S·3.5H₂O, *M*_{R} = 796.1): C 36.2 (36.2); H 6.3 (6.3); N 8.8 (8.7).

### Example 45: Synthesis of compound 40

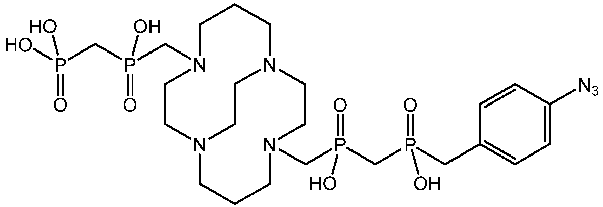

In a glass vial (4 mL), **37**·4H₂O (161 mg; 220 µmol; 1.0 equiv.) was dissolved in aqueous HCl (243.8 mM; 1800 µL; 440 µmol; 2.0 equiv.) and the resulting mixture was cooled in an ice bath (1 °C). Freshly prepared aqueous solution of NaNO₂ was then gradually added (290 mM; 1.0 mL; 290 µmol; 1.3 equiv.; 100 µL each three minutes). Freshly prepared aqueous solution of NaN₃ (580 mM; 760 µL; 441 µmol; 2.0 equiv.) was then added. The reaction mixture was then stirred at room temperature for three hours. The crude product was divided into two halves which were successively purified by preparative HPLC (C8; gradient elution H₂O-0.1% TFA-MeCN). Fraction with product were combined and directly lyophilized. Product was obtained as a white substance (29.5 mg; 57 %; 1 step; based on **37**·4H₂O).
**NMR** (D₂O): ¹**H** *δ* 2.07 (CH₂-C*H*₂-CH₂, m, 2H); 2.18 (CH₂-C*H*₂-CH₂, m); 2.28-2.41 (P-C*H*₂-P, m, 4H); 2.70-3.82 (*cycle,* N-C*H*₂-P, P-C*H*₂-C, bm, 26H); 7.17 (C*H*-C-N, d, 2H, ³*J*_{HH} = 7 Hz); 7.31 (C*H*-C-CH₂, dd, 2H, ³*J*_{HH} = 7 Hz, ^{H}*J*_{HP} = 2 Hz); ¹³C{¹H} *δ* 20.5 (CH₂-*C*H₂-CH₂, s); 22,5 (CH₂-*C*H₂-CH₂, s); 32.2 (P-*C*H₂-P, dd, ¹*J*_{CP} = 121 Hz, ¹*J*_{CP} = 82 Hz); 32.3 (P-*C*H₂-P, dd, ¹*J*_{CP} = 82 Hz); 36.6 (P-*C*H₂-C, d, ¹*J*_{CP} = 93 Hz); 47.2-60.1 (12 × N-*C*H₂, bm); 123.5 (*C*H-C-N, s); 131.7 (*C*H-C-CH₂, d, ³*J*_{CP} = 4 Hz); 132.2 (CH-*C*-CH₂, d, ²*J*_{CP} = 7 Hz); 141.0 (CH-*C*-N, s); ³¹**P**{¹H} *δ* 17.5 (HO-*P*-OH, m, 1P); 22.2 (*P*-CH₂-*N*, bm, 1P); 24.0 (*P*-CH₂-N, bm, 1P); 36.8 (*P*-CH₂-C, m, 1P).
MS: (-) 666.0 [M-H₃O⁺]⁻ ; 684.1 [M-H⁺]⁻. (+) 688.1 [M+H⁺]⁺; 708.1 [M+Na⁺]⁺; 724.0 [M+K⁺]⁺; 730.1 [M-H⁺+2Na⁺]⁺.
**TLC** (SiO₂, EtOH-conc. aq. NH₄OH 1:1): *R*_{f} = 0.2.
**EA** (C₂₃H₄₃N₇O₉P₄·3H₂O, *M*_{R} = 739.6): C 37.4 (37.2); H 6.7 (6.6); N 13.3 (13.3).

### Example 46: Synthesis of compound 41

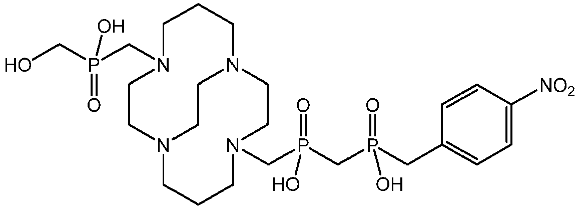

To a glass vial (20 mL), **31**·5.5H₂O (50.9 mg; 82.6 µmol; 1.0 equiv.) was added. Paraformaldehyde (10.1 mg; 343 µmol; 4.2 equiv.), and hydroxymethylphosphinic acid (50.8 mg; 529 µmol; 6.4 equiv.), and aqueous HCl (6 M; 2 mL) were subsequently added and the resulting suspension was stirred at 80 °C overnight. The mixture was then evaporated to dryness and several times co-evaporated with H₂O. The residue was purified by ion exchange chromatography (DOWEX 50; H+-form; H₂O). Fractions with product were combined, evaporated to dryness and several times co-evaporated with H₂O. The crude product was purified by preparative HPLC (C8; gradient elution H₂O-0,1% TFA-MeCN). Fraction with product was directly lyophilized. Resulting product was obtained as a yellowish substance (25.8 mg; 46 %; 1 step; based on **31**·5.5H₂O).
**NMR** (D₂O): ¹**H** *δ* 1.84 (CH₂-C*H*₂-CH₂, m, 2H); 2.15-2.55 (CH₂-C*H*₂-CH₂, P-C*H*₂-P, m, 4H); 2.76 (*cycle,* m, 4H); 2.98 (*cycle,* m, 2H); 3.09 (*cycle,* m, 2H); 3.18-3.84 (*cycle,* N-C*H*₂-P, P-C*H*₂-C, O-C*H*₂-P, m, 20H); 7.57 (C*H*-C-CH₂, d, 2H, ²*J*_{HH} = 8 Hz,); 8.25 (C*H*-C-N, d, 2H, ²*J*_{HH} = 8 Hz); ¹³C{¹H} *δ* 20.7 (CH₂— *C*H₂-CH₂, s); 33.5 (P-*C*H₂-P, t, ¹*J*_{CP} = 81 Hz); 39.5 (P-*C*H₂-C, dd, ¹*J*_{CP} = 88 Hz); 49.1 (*cycle,* s); 49.2 (*cycle,* s); 50.2 (*cycle,* s); 51.7 (P-*C*H₂-N, d, ¹*J*_{CP} = 84 Hz); 54.6 (*cycle,* s); 54.9 (*cycle,* s); 58.3 (*cycle,* s); 58.9 (P-*C*H₂-N, d, ¹*J*_{CP} = 83 Hz); 58.9 (P-*C*H₂-O, d, ¹*J*_{CP} = 116 Hz); 124.6 (C*H*-C-N, s); 131.6 (*C*H-C-CH₂, d, ³*J*_{CP} = 5 Hz); 142.3 (CH-*C*-CH₂, d, ²*J*_{CP} = 8 Hz); 147.2 (CH-*C*-N, s); ³¹**P**{¹H} *δ* 23.6 (*P*-CH₂-*P*, bm, 1P); 29.7 (*P*-*C*H₂-*P*, bm, 1P); 37.3 (*P*-CH₂-O, m, 1P).
**MS:** (-) 623.5 [M-H⁺]⁻. (+) 625.4 [M+H⁺]⁺; 647.7 [M+Na⁺]⁺; 663.5 [M+K⁺]⁺.
**TLC** (SiO₂, EtOH-conc. aq. NH₄OH 2:1): *R*_{f} = 0.6.
**EA** (C₂₃H₄₂N₅O₉P₃·3H₂O, *M*_{R} = 679.6): C 40.7 (40.6); H 7.1 (7.1); N 10.3 (10.5).

### Example 47: Synthesis of compound 42

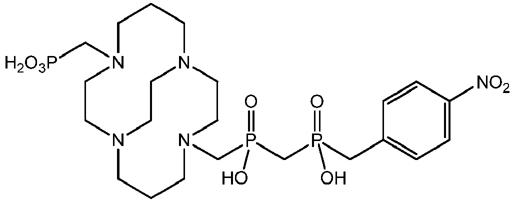

To a glass vial (20 mL), **31**·5.5H₂O (336 mg; 545 µmol; 1.0 equiv.) was added. Paraformaldehyde (44.2 mg; 1.47 mmol; 2.7 equiv.), and phosphorous acid (380 mg; 4.63 mmol; 8.5 equiv.), and aqueous HCl (12 M; 10 mL) were subsequently added and the resulting suspension was stirred at 40 °C for four days. The mixture was then evaporated to dryness and several times co-evaporated with H₂O. The residue was purified by ion exchange chromatography (DOWEX 50; H+-form; H₂O). Fractions with product were combined, evaporated to dryness and several times co-evaporated with H₂O. The crude product was purified by flash chromatography (C18; gradient elution H₂O-0.1% TFA-MeCN). Fraction with product was directly lyophilized. Resulting product was obtained as a yellow substance (194 mg; 54 %; 1 step; based on **31**·5.5H₂O).
**NMR** (D₂O): ¹**H** *δ* 1.85 (CH₂-C*H*₂-CH₂, m, 2H); 2.12 (CH₂-C*H*₂-CH₂, m, 1H); 2.27 (P-C*H*₂-P, m, 4H); 2.35 (CH₂-C*H*₂-CH₂, m, 1H); 2.66-2.72 (*cycle,* m, 3H); 2.89 (*cycle,* m, 2H); 2.98-3.12 (*cycle,* m, 3H); 3.14-3.60 (*cycle,* N-C*H*₂-P, P-C*H*₂-C, m, 18H); 7.42 (C*H*-C-CH₂, d, 2H, ²*J*_{HH} = 8 Hz,); 8.20 (C*H*-C-N, d, 2H, ²*J*_{HH} = 8 Hz); ³¹**P**{¹H} *δ* 21.2 (HO-*P*-OH, s, 1P); 23.6 (*P*-CH₂-*P*, d, 1P, ²*J*_{PP} = 8 Hz); 29.7 (*P*-*C*H₂-P, d, 1P, ²*J*_{PP} = 8 Hz).
**MS:** (-) 609.9 [M-H⁺]⁻. (+) 612.1 [M+H⁺]⁺; 634.2 [M+Na⁺]⁺; 650.2 [M+K⁺]⁺.
**TLC** (SiO₂, EtOH-conc. aq. NH₄OH 2:1): *R*_{f} = 0.4.
**EA** (C₂₂H₄₀N₅O₉P₃·2.5H₂O, *M*_{R} = 656.5): C 40.3 (40.5); H 6.9 (7.0); N 10.7 (10.4).

### Example 48: Synthesis of compound 43

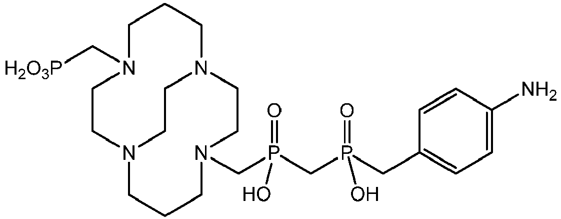

In a glass flask (100 mL), **42·**2.5H₂O (185 mg; 0.28 mmol; 1.0 equiv.) was dissolved in H₂O (10 ml) and the flask was briefly washed with gaseous argon. Catalyst (10 % Pd@C; 54 mg) was then added and the resulting suspension was saturated with gaseous H₂ at 60 °C overnight. The catalyst was then filtered off and the filtrate was evaporated to dryness. The residue was re-dissolved in H₂O (200 mL) and subsequently lyophilized. Resulting product was obtained as a white substance (158 mg; 89 %; 1 step; based on **42·**2.5H₂O).
**NMR** (D₂O): ¹**H** *δ* 1.77 (CH₂-C*H*₂-CH₂, m, 1H); 1.87 (CH₂-C*H*₂-CH₂, m, 1H); 2.12 (CH₂-C*H*₂-CH₂, m, 1H); 2.22-2.40 (CH₂-C*H*₂-CH₂, P-C*H*₂-P, bm, 5H); 2.62-2.79 (*cycle,* m, 2H); 2.91-3.02 (*cycle,* m, 4H); 3.07-3.48 (*cycle,* N-C*H*₂-P, P-C*H*₂-C, m, 20H); 6.89 (C*H*-C-N, d, 2H, ²*J*_{HH} = 8 Hz); 7.33 (C*H*-C-CH₂, d, 2H, ²*J*_{HH} = 8 Hz,); **¹³C**{¹H} *δ* 18.9 (CH₂-*C*H₂-CH₂, s); 22.3 (CH₂-*C*H₂-CH₂, s); 34.1 (P-*C*H₂-P, t, ¹*J*_{CP} = 83 Hz); 37.5 (P-*C*H₂-C, d, ¹*J*_{CP} = 90 Hz); 41.2-59.5 (12 × N-*C*H₂, bm); 120.1 (CH-*C*-N, s); 127.1 (*C*H-C-N, m); 129.3 (*C*H-C-CH₂, d, ³*J*_{CP} = 5 Hz); 134.5 (CH-*C*-CH₂, m); **³¹P**{¹H} *δ* 19.3 (HO-*P*-OH, m, 1P); 22.4 (*P*-*C*H₂-*P*, d, 1P, ²*J*_{PP} = 9 Hz); 31.5 (*P*-CH₂-*P*, bm, 1P).
**MS:** (-) 580.2 [M-H⁺]⁻. (+) 582.8 [M+H⁺]⁺; 603.2 [M+Na⁺]⁺; 619.8 [M+K⁺]⁺.
**TLC** (SiO₂, EtOH-conc. aq. NH₄OH 2:1): *R*_{f} = 0.3.
**EA** (C₂₂H₄₂N₅O₇P₃·3H₂O, *M*_{R} = 635.6): C 41.2 (41.6); H 6.8 (6.6); N 10.7 (11.0).

### Example 49: Synthesis of targeting conjugate 44

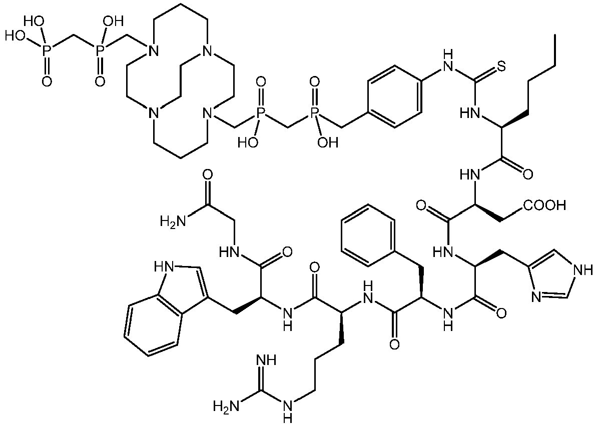

To a glass vial (4 mL), **39**·3.5H₂O (5.3 mg; 6.7 µmol; 1.1 equiv.) was added. Peptide HNleAspHisD-PheArgTrpGlyNH₂ (5.9 mg; 6.4 µmol; 1.0 equiv.), aqueous buffer H₃BO₃-LiOH (750 mM v D₂O; pD = 10.1; 400 µL; 300 µmol; 50 equiv.) and H₂O (600 µL) were then added and the resulting mixture was stirred at room temperature overnight. The crude product was purified by preparative HPLC (C8; gradient elution H₂O-0.1% TFA-MeCN). Fraction with product was lyophilized. Resulting product was obtained as a white substance (5.7 mg; 55 %; 1 step; based on **39·**3.5H₂O).
**NMR** (D₂O): **¹H** *δ* 0.86 (C*H*₃*,* t, 3H, ³*J*_{HH} = 7 Hz); 1.01-1.53 (C*H*₂, bm, 10H); 1.68-2.29 (C*H*₂, P*-*C*H*₂-P, *cycle,* bm, 8 H); 2.47-3.73 (*cycle,* C*H*₂*,* P-C*H*₂-N, P-C*H*₂-C, bm, 36H); 3.80 (C*H*₂, m, 2H); 4.11 *(CH,* m, 1H); 4.45 *(CH,* m, 1H); 4.51 (C*H*, m, 1H); 4.55 (C*H*, m, 1H); 4.59 (C*H*, m, 1H); 4.69 (CH, m, 1H); 6.90 (CH₂— C*-*C*H-*N*,* s, 1H); 7.14 (*CH,* t, 1H, ³*J*_{HH} = 7 Hz); 7.14-7.27 (*arom.,* m, 6H); 7.29-7.38 (*arom.,* m, 5H); 7.46 (*CH,* d, 1H, ³*J*_{HH} = 8 Hz); 7.66 (C*H,* d, 1H, ³*J*_{HH} = 8 Hz); 8.03 (N-C*H*-N, s, 1H); **³¹P**{¹H} *δ* 13.2 (HO-*P*-OH, bm, 1P); 25.3 (*P*-CH₂-N, bm, 1P); 27.4 (*P*-CH₂-N, bm, 1P); 32.3 (*P*-CH₂-C, bm, 1P).
**MS:** (-) 1628.2 [M-H⁺]⁻. (+) 1630.4 [M+H⁺]⁺.
**TLC** (SiO₂, EtOH-conc. aq. NH₄OH 1:1): *R*_{f} = 0.2.

### Example 50: Synthesis of conjugate 45

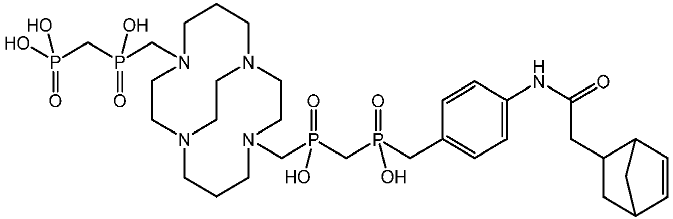

In a glass vial (4 mL), **38·**4H₂O (5.3 mg; 6.7 µmol; 1.1 equiv.) and *N-*hydroxysuccinimidester of 5-norbornen-2-acetic acid (21.1 mg; 84.6 µmol; 2.2 equiv.) were dissolved in a mixture of aqueous buffer H₃PO₄-NaOH (1.0 M pH = 8.1; 1.00 mL; 1.0 mmol; 26 equiv.) and MeCN (0.5 mL). The resulting mixture was stirred at room temperature overnight. The crude product was purified by preparative HPLC (C8; gradient elution H₂O-0.1% TFA-MeCN). Fraction with product was lyophilized. Resulting product was obtained as a white substance (26.1 mg; 71 %; 1 step; based on **38·**4H₂O).
**NMR** (D₂O): **¹H** *δ* 1.41-1.56 (CH-C*H*₂-CH, C*H*₂ bm, 2H); 1.62-1.75 (C*H*₂, CH-C*H*₂-CH, bm, 2H); 1.94-2.32 (P-C*H*₂-P, *cycle,* bm, 6H); 2.44 (P-C*H*₂-P, t, 2H, ²*J*_{HP} = 17 Hz); 2.84-4.16 (*cycle, CH,* N-C*H*₂-P, P-C*H*₂-C, bm, 31H); 5.96 (C*H*, dd, ³*J*_{HH} = 12 Hz, ³*J*_{HH} = 5 Hz); 6.32 (C*H,* dd, ³*J*_{HH} = 12 Hz, ³*J*_{HH} = 6 Hz); 6.98 (C*H*, d, 2H, ³*J*_{HH} = 8 Hz); 7.63 *(CH,* d, 2H, ³*J*_{HH} = 8 Hz); ³¹**P**{¹H} *δ* 21.1 (HO-*P*-OH, bm, 1P); 23.5 (*P*-CH₂-N, bm, 1P); 24.9 (*P*-CH₂-N, bm, 1P); 29.3 (*P*-CH₂-C, bm, 1P).
**MS**: (-) 792.5 [M-H⁺]⁻.
**TLC** (SiO₂, EtOH-conc. aq. NH₄OH 1:1): *R*_{f} = 0.3.
**EA** (C₃₂H₅₅N₅O₁₀P₄·TFA·2H₂O, *M*_{R} = 943.3): C 43.3 (43.4); H 6.4 (6.2); N 7.4 (7.7).

### Example 51: Synthesis of intermediate product F

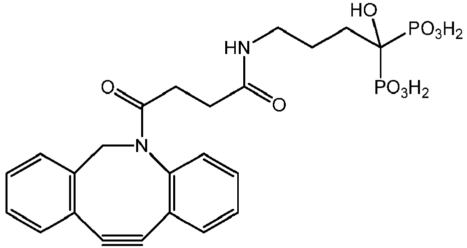

In a glass vial (4 mL), sodium aldendronate trihydrate (21.0 mg; 64.6 µmol; 1.4 equiv.) was dissolved in aqueous NaOH (250.6 mM; 773 µL; 194 µmol; 4.2 equiv.) and the resulting mixture was cooled in an ice bath (5 °C). Solution of *N-*hydroxysuccinimidester of 4-dibenzocyclooctynamidobutanoic acid (18.4 mg; 45.7 µmol; 1.0 equiv.) in dry DMSO (1.25 mL) was then added dropwise and the resulting mixture was stirred in the absence of light overnight. On the next day, the mixture was purified by preparative HPLC (C8; gradient elution H₂O-0.1% TFA-MeCN). Fraction with product was lyophilized. Resulting product was obtained as a white substance (16.3 mg; 66 %; 1 step; based on *N*-hydroxysuccinimidester of 4-dibenzocyclooctynamidobutanoic acid).
**NMR** (D₂O): ¹**H** *δ* 1.42 (CH₂-C*H*₂-CH₂, m, 2H); 1.77 (C*H*₂, m, 1H); 1.85 (C*H*₂-C-P, tm, ³*J*_{HP} = 13 Hz); 1.96 (C*H*₂, t, 2H, ³*J*_{HH} = 7 Hz); 2.16 (C*H*₂, m, 1H); 2.88 (C*H*₂-NH, t, ³*J*_{HH} = 9 Hz); 3.61 (C*H*₂-N-CO, d, 1H, ²*J*_{HH} = 14 Hz); 5.04 (C*H*₂-N-CO, d, 1H, ²*J*_{HH} = 14 Hz); 7.29 (C*H*, d, 1H, ³*J*_{HH} = 7 Hz); 7.35 (C*H*, t, 1H, ³*J*_{HH} = 7 Hz); 7.39 (C*H,* t, 1H, ³*J*_{HH} = 7 Hz); 7.42-7.53 (C*H*, m, 3H); 7.58 (C*H,* d, 1H, ³*J*_{HH} = 7 Hz); 7.63 (C*H,* t, 1H, ³*J*_{HH} = 7 Hz); **³¹P**{¹H} *δ* 21.2 (m, 1P).
MS: (-) 516.9 [M-H₃O⁺]⁻; 534.8 [M-H⁺]⁻. (+) 536.2 [M+H⁺]⁺; 559.2 [M+Na⁺]⁺; 581.1 [M-H⁺+2Na⁺]⁺.
TLC (SiO₂, EtOH-conc. aq. NH₄OH 1:1): *R*_{f} = 0.

### Example 52: Synthesis of conjugate 46

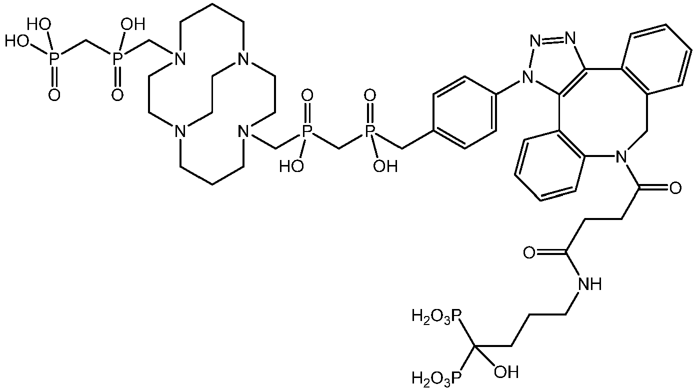

In a glass vial (4 mL), **40**·3H₂O (14.1 mg; 19.1 µmol; 1.0 equiv.) was dissolved in aqueous buffer AcOH-NH₃ (500 mM; 700 µL; 350 µmol; 18 equiv.). Freshly prepared solution of intermediate **F** (11.6 mg; 21.6 µmol; 1.1 equiv.) in a mixture of DMSO-MeCN-H₂O (1:1:1; 800 µL) was then added and the resulting solution was stirred at room temperature for three hours. The crude product was purified by preparative HPLC (C8; gradient elution H₂O-0.1% TFA-MeCN). Fraction with product was lyophilized. Resulting product (mixture of two regioisomers) was obtained as a white substance (14.1 mg; 56 %; 1 step; based on **40·**3H₂O).
**NMR** (D₂O): ¹**H** *δ* 1.22-1.41 (C*H*₂, bm, 4H); 1.83-1.99 (C*H*₂-C-P, C*H*₂*,* bm, 6H); 2.05 (C*H*₂, m, 2H); 2.14-2.45 (P-C*H*₂-P, C*H*₂*,* bm, 6H); 2.63-3.68 (*cycle,* P-C*H*₂-C, P-C*H*₂-N, bm, 26H); 4.52-5.07 (C*H*₂-N-CO, m, 1H); 5.63-5.79 (C*H*₂-N-CO, m, 1H); 6.94 (C*H,* d, 1H, ³*J*_{HH} = 8 Hz); 7.02-7.22 (C*H*, m, 1H); 7.02-7.22 (C*H*, m, 1H); 7.30 (C*H*, bm, 2H); 7.34-7.61 (C*H*, bm, 7H); **³¹P**{¹H} *δ* 14.4 (HO-*P*-OH, bm, 1P); 18.4 (*P*O₃H₂, bm, 2P); 24.4-28.9 (*P*-CH₂-N, bm, 2P); 33.6-34.7 (*P*-CH₂-C, bm, 1P).
**MS:** (-) 1202.5 [M-H₃O⁺]⁻; 1220.5 [M-H⁺]⁻. (+) 1222.7 [M+H⁺]⁺.
**TLC** (SiO₂, EtOH-conc. aq. NH₄OH 1:1): *R*_{f} = start.
**EA** (C₄₆H₆₉N₉O₁₈P₆·5.5H₂O, *M*_{R} = 1321.0): C 41.8 (41.8); H 6.1 (6.0); N 9.5 (9.5).

### Example 53: Synthesis of targeting conjugate 47

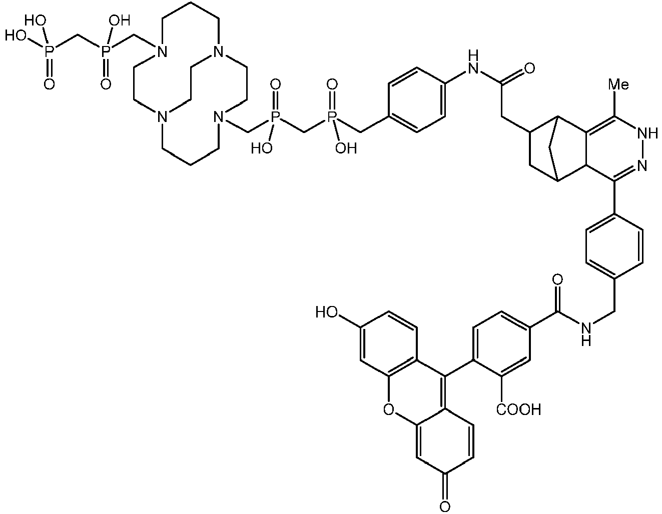

In a glass vial (4 mL), fluorescein-6-methyl-tetrazine conjugate (1.0 mg; 1.8 µmol; 1.0 equiv.) was dissolved in dry DMSO (500 µL) followed by addition of freshly prepared solution of **45·**TFA·2H₂O (2.5 mg; 2.7 µmol; 1.0 equiv.) in aqueous buffer AcOH-NH₃ (100 mM; 500 µL; 50 µmol; 28 equiv.). The resulting mixture was stirred at room temperature overnight. The crude product was purified by preparative HPLC (C8; gradient elution H₂O-0.1% TFA-MeCN). Fraction with product was lyophilized. Resulting product (mixture of two regioisomers) was obtained as a white substance (≈2 mg).
**NMR** (D₂O): **¹H** *δ* 1.46 (CH-C*H*₂-CH, m, 1H); 1.62 (C*H*₂, bm, 1H); 1.88-1.96 (*cycle,* CH-C*H*₂-CH, bm, 2H); 1.98-2.16 (P-C*H*₂-P, *cycle,* bm, 3H); 2.25 (P-C*H*₂-P, dd, 2H, ²*J*_{HP} = 17 Hz, ²*J*_{HP} = 15 Hz); 2.32 (C*H*₃*,* s, 3H); 2.63-4.03 (*cycle, CH,* N-C*H*₂-P, P-C*H*₂-C, bm, 33H); 6.66 (C*H*, m, 1H); 6.82-6.84 (C*H*, bm, 3H); 6.97 (C*H,* d, 2H, ³*J*_{HH} = 7 Hz); 7.12-7.23 (C*H*, bm, 3H); 7.29 (C*H,* d, 2H, ³*J*_{HH} = 9 Hz); 7.34 (C*H,* d, 1H, ³*J*_{HH} = 7 Hz); 8.03 (d, 2H, ³*J*_{HH} = 8 Hz); **³¹P**{¹H} *δ* 14.8 (HO-*P*-OH, d, 1P, ²*J*_{PP} = 8 Hz); 28.9 (*P*-CH₂-N, m, 1P); 32.1 (*P*-CH₂-N, m, 1P); 36.2 (*P*-CH₂-C, d, 1P, ²*J*_{PP} = 10 Hz).
**MS:** (-) 1323.8 [M-H⁺]⁻. (+) 1369.7 [M-H⁺+2Na⁺]⁺.
**TLC** (SiO₂, *i*PrOH-conc. aq. NH₄OH-H₂O 7:3:3): *R*_{f} = 0.3.

### Example 54: Synthesis of conjugate 48

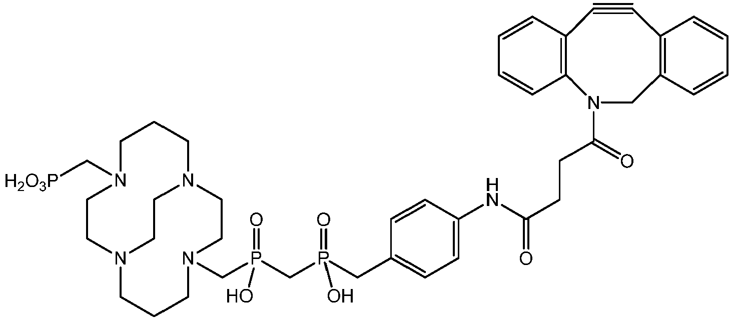

In a glass vial (4 mL), **43·**3H₂O (28.9 mg; 45.5 µmol; 1.0 equiv.) was dissolved in a mixture of aqueous buffer H₃PO₄-NaOH (1.0 M, pH = 8.1; 1.00 mL) and MeCN (0.5 mL) and the resulting solution was cooled in an ice bath (5 °C). Solution of *N-*hydroxysuccinimidester of 4-dibenzocyclooctynamidobutanoic acid (36.2 mg; 90.0 µmol; 2.0 equiv.) in dry DMSO (1.7 mL) was then added dropwise and the resulting solution was stirred in the absence of light overnight. On the next day, the mixture was purified by preparative HPLC (C8; gradient elution H₂O-0,1% TFA-MeCN). Fraction with product was directly lyophilized. Product was obtained as a violet substance (24.6 mg; 63 %; 1 step; based on **43·**3H₂O).
**NMR** (D₂O): **³¹P**{¹H} *δ* 12.2 (HO-*P*-OH, s, 1P); 19.4 (*P*-*C*H₂-P, d, 1P, ²*J*_{PP} = 10 Hz); 38.2 (*P*-*C*H₂-*P*, d, 1P, , ²*J*_{PP} = 10 Hz).
**MS:** (-) 867.6 [M-H⁺]⁻. (+) 870.2 [M+H⁺]⁺; 892.1 [M+Na⁺]⁺; 907.7 [M+K⁺]⁺.
**TLC** (SiO₂, EtOH-conc. aq. NH₄OH 2:1): *R*_{f} = 0.4.
**EA** (C₄₁H₅₅N₆O₉P₃·2.5H₂O, *M*_{R} = 913.9): C 53.6 (53.9); H 6.8 (6.6); N 8.9 (9.2).

### Example 55: Synthesis of conjugate 49

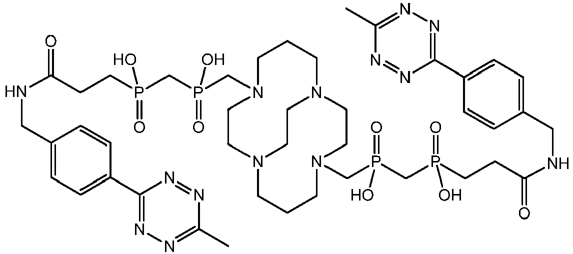

Compound **35**·2H₂O (108 mg; 150 µmol; 1.0 equiv.) and *N*-hydroxysuccinimide (NHS; 69 mg; 600 µmol; 4.0 equiv.) were dissolved in aqueous acetate buffer HOAc/NaOAc (pH 5.5; 0.5 M; 3 mL). To this solution, EDC·HCl (186 mg; 1.20 mmol; 8 equiv.) in the same buffer (1 mL) was added. Carboxylate functions were activated for 30 min and then pH of this solution was adjusted to 9 using aqueous NaOH (0.05 M). Solution of methyltetrazinamine hydrochloride (78.4 mg; 330 µmol; 2.2 equiv.) in dry DMSO (2.0 mL) was then added and the pH was re-adjusted to 8 using aqueous NaOH (0.05 M). The mixture was stirred at room temperature for three hours. The mixture was then concentrated to 2 mL volume and subsequently purified by preparative HPLC (C8; gradient elution H₂O-0.1% TFA-MeCN). Fraction with product was directly lyophilized. Product was obtained as a violet substance (121 mg; 75 %; 1 step; based on **35·**2H₂O).
**NMR** (D₂O): ¹**H** *δ* 1.82 (CH₂-C*H*₂-CH₂, m, 2H); 1.88 (CH₂-C*H*₂-CH₂, m, 2H); 1.99 (P-C*H*₂-C, m, 4H); 2.13 (P-C*H*₂-P, m, 4H); 2.34 (C*H*₃*,* s, 6H); 2.40 (C*H*₂-CO, t, 4H, ³*J*_{HH} = 7 Hz); 2.72-3.22 *(cycle,* bm, 20H); 3.34 (N-C*H*₂-P, m, 4H); 4.31 (C*H*₂-NH-CO, s, 4H); 7.11 (C*H,* d, 2H, ³*J*_{HH} = 8 Hz); 8.11 (C*H,* d, 2H, ³*J*_{HH} = 8 Hz); ¹³C{¹H} *δ* 19.4 (C*H*₃*,* s); 22.1 (CH₂-*C*H₂-CH₂, s); 28.0 (P-*C*H₂-C, d, ¹*J*_{CP} = 93 Hz); 29.3 (*C*H₂-CO, s); 35.5 (P-*C*H₂-P, dd, ¹*J*_{CP} = 79 Hz, ¹*J*_{CP} = 75 Hz); 44.2 (*C*H₂-NH-CO, s); 50.1 (*cycle,* s); 54.2 (*cycle,* s); 55.8 (*cyclus,* d, ³*J*_{CP} = 5 Hz); 56.2 (N-*C*H₂-P, d, ¹*J*_{CP} = 102 Hz); 58.2 (*cycle,* s); 59.0 (*cycle,* d, 3); 122.6 (*arom.,* s); 127.3 (*arom.,* s); 129.6 (*arom.,* s); 138.2 (*arom.,* s); 161.2 (*arom.,* s); 164.2 (*arom.,* s); 174.2 (CO, d, ³*J*_{CP} = 17 Hz); **³¹P**{¹H} *δ* 27.2 (CH₂-*P*-CH₂, bm, 2P); 36.1 (CH₂-*P*-CH₂, d, 2P, ²*J*_{PP} = 8 Hz).
**MS:** (-) 1048.1 [M-H⁺]⁻. (+) 1050.3 [M+H⁺]⁺; 1072.2 [M+Na⁺]⁺; 1087.6 [M+K⁺]⁺. **TLC** (SiO₂, EtOH-conc. aq. NH₄OH 2:1): *R*_{f} = 0.4.
**EA** (C₄₂H₆₄N₁₄O₁₀P₄·1.5H₂O, *M*_{R} = 1076.0): C 46.6 (46.9); H 6.2 (6.0); N 17.8 (18.2).

### Example 56: Synthesis of copper(II) complex of compound 14

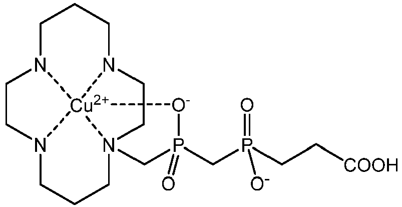

In a glass vial (4 mL), weighted amount of compound **14**·4H₂O (61.3 mg; 122 µmol; 1.0 equiv.) and Cu(OAc)₂·H₂O (34.2 mg; 171 µmol; 1.4 equiv.) was dissolved in aqueous pyridine (2 %; 3 mL) and the mixture was stirred for 10 min. Subsequently, the reaction mixture was evaporated to dryness and then several times co-evaporated with water. The crude complex was purified on ion exchange resin (DOWEX 50; H⁺-forma; water elution). Fractions containing the product were combined and evaporated to dryness. The residue was re-dissolved in water (50 mL) and the solution was lyophilized. The final product was obtained as dark blue material (51.4 mg; 83 %; 1 step; based on **14**·4H₂O).
**MS**: (-) 488,0 [M-H⁺]⁻; 524,0 [M+Cl⁻]⁻. (+) 449,0 [M+H⁺]⁺; 512,0 [M+Na⁺]⁺. **TLC** (SiO₂, *i*PrOH-konc. NH₄OH-H₂O 7:3:3): *R*_{f} = 0,4.
**EA** (C₁₅H₃₂N₄O₆P₂Cu·H₂O, *M*_{R} = 508,0): C 35,5 (35,7); H 6,8 (6,6); N 11,0 (11,1).

### Example 57: Synthesis of copper(II) complex of compound 23

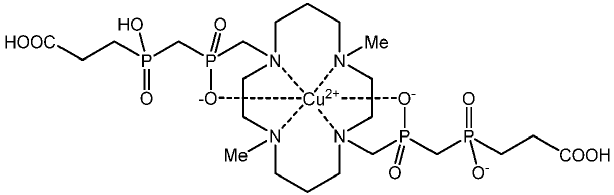

In a glass vial (4 mL), weighted amount of compound **23**·3H₂O (98.7 mg; 134 µmol; 1.0 equiv.) and Cu(OAc)₂·H₂O (41.2 mg; 206 µmol; 1.5 equiv.) was dissolved in aqueous pyridine (5 %; 3 mL) and the mixture was stirred for 10 min. Subsequently, the reaction mixture was evaporated to dryness and then several times co-evaporated with water. The crude complex was purified on ion exchange resin (DOWEX 50; H⁺-forma; water elution). Fractions containing the product were combined and evaporated to dryness. The residue was re-dissolved in water (100 mL) and the solution was lyophilized. The final product was obtained as dark blue material (83.4 mg; 77 %; 1 step; based on **23**·3H₂O).
**MS:** (-) 743,2 [M-H⁺]⁻.
**TLC** (SiO₂, *i*PrOH-konc. NH₄OH-H₂O 7:3:3): *R*_{f} = 0,2.
**EA** (C₂₂H₄₅N₄O₁₂P₄Cu·0,5HCl·2,5H₂O, *M*_{R} = 808,3): C 29,5 (29,6); H 4,8 (4,9); N 7,8 (7,8).

### Example 58: Synthesis of copper(II) complex of compound 37

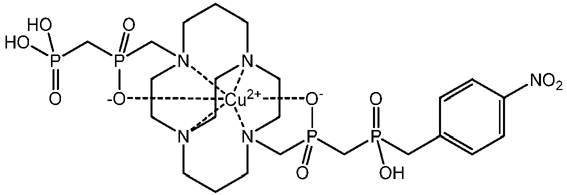

In a glass vial (4 mL), weighted amount of compound **37·**4.5H₂O (29.7 mg; 38.5 µmol; 1.0 equiv.) was dissolved in aqueous solution of CuCl₂ (63 mM; 793 µl; 50.0 µmol; 1.3 equiv.) and the solution pH was slowly adjusted to pH 6 with aqueous solution of NaOH. The crude complex was purified by preparative HPLC (C8; gradient elution H₂O-0.1 % TFA-MeCN). Fraction containing the product was lyophilized. The final product was obtained as light blue material (25.8 mg; 70 %; 1 step; based on **37·**4.5H₂O).
**MS:** (-) 749,1 [M-H⁺]⁻; 785,0 [M+Cl⁻]⁻. (+) 751,1 [M+H⁺]⁺; 774,1 [M+Na⁺]⁺; 790,2 [M+K⁺]⁺.
**TLC** (SiO₂, *i*PrOH-konc. NH₄OH-H₂O 7:3:3): *R*_{f} = 0,2.
**EA** (C₂₃H₄₁N₅O₁₁P₄Cu·0,5TFA·3H₂O, *M*_{R} = 953,5): C 30,2 (30,1); H 5,0 (5,0); N 7,3 (7,4).

### Example 59: Synthesis of bismuth(III) complex of compound 35

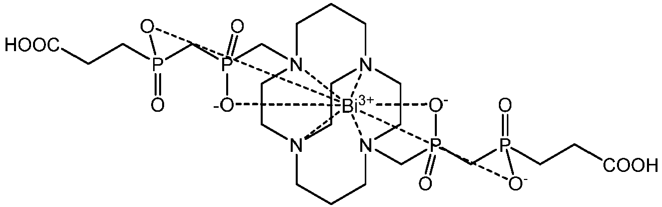

In a glass vial (4 mL), weighted amount of compound **35**·2H₂O (34.4 mg; 47.8 µmol; 1.0 equiv.) was dissolved in aqueous solution of Bi(NO₃)₃ (50.5 mM; 947 µl; 47.8 µmol; 1.0 equiv.) and the solution pH was slowly adjusted to pH 5with solid urotropine. The reaction mixture was heated at 90°C for 2 h. Then, further solid urotropine was added to increase pH to 5. The reaction mixture was heated at 90°C for 6 h. After cooling, the reaction mixture was evaporated to dryness. The crude complex was purified on ion exchange resin (Amberlite CG50; H⁺-forma; water elution). Fractions containing the product were combined and evaporated to dryness. The residue was re-dissolved in water (50 mL) and the solution was lyophilized. The final product was obtained as white material (34.3 mg; 75 %; 1 step; based on **35·**2H₂O).
**MS:** (-) 887,2 [M-H⁺]⁻. (+) 889,2 [M+H⁺]⁺.
**TLC** (SiO₂, *i*PrOH-konc. NH₄OH-H₂O 7:3:3): *R*_{f} = 0,4.
**EA** (C₂₂H₄₃N₄O₁₂P₄Bi·2H₂O, *M*_{R} = 955,5): C 28,6 (28,5); H 5,1 (4,9); N 6,1 (6,3).

### Example 60: Synthesis of lutetium(III) complex of compound 35

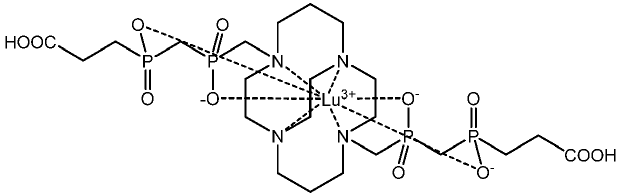

In a glass vial (4 mL), weighted amount of compound **35**·2H₂O (44.1 mg; 61.4 µmol; 1.0 equiv.) was dissolved in aqueous solution of LuCl₃ (50.1 mM; 1.23 ml; 61.6 µmol; 1.0 equiv.) and the solution pH was slowly adjusted to pH 7 with aqueous NaOH. The reaction mixture was heated at 80°C overnight. Next day, the reaction mixture was evaporated to dryness and the crude complex was purified on ion exchange resin (Amberlite CG50; H⁺-forma; water elution). Fractions containing the product were combined and evaporated to dryness. The residue was re-dissolved in water (50 mL) and the solution was lyophilized. The final product was obtained as white material (37.1 mg; 64 %; 1 step; based on **35**·2H₂O).
**MS**: (-) 853,1 [M-H⁺]⁻. (+) 855,1 [M+H⁺]⁺; 893,1 [M+K⁺]⁺.
**TLC** (SiO₂, EtOH-konc. NH₄OH 1:1): *R*_{f} = 0,6.
**EA** (C₂₂H₄₃N₄O₁₂P₄Lu·5H₂O, *M*_{R} = 944,5): C 28,0 (28,0); H 5,7 (5,9); N 5,9 (5,9).

### Example 61: Synthesis of gadolinium(III) complex of compound 43

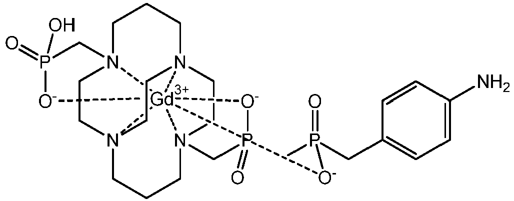

In a glass vial (4 mL), weighted amount of compound **43**·3H₂O (62.5 mg; 66.2 µmol; 1.0 equiv.) was dissolved in aqueous solution of GdCl₃ (49.8 mM; 1.33 ml; 66.2 µmol; 1.0 equiv.) and the solution pH was slowly adjusted to pH 6. The reaction mixture was heated at 80°C for 2 h. Then, pH was increased to 5 with aqueous NaOH and the reaction mixture was heated at 80°C overnight. Next day, the reaction mixture was evaporated to dryness and the crude complex was purified on ion exchange resin (Amberlite CG50; H⁺-forma; water elution followed with 10 % AcOH in mixture of water:EtOH 1:1). Fractions containing the product were combined and evaporated to dryness. The residue was re-dissolved in water (50 mL), evaporated to dryness and dissolved in water again (50 mL) and the solution was lyophilized. The final product was obtained as white material (37.1 mg; 75 %; 1 step; based on **43**·3H₂O).
MS: (-) 853,1 [M-H⁺]⁻. (+) 855,1 [M+H⁺]⁺; 893,1 [M+K⁺]⁺.

### Example 62: Comparative experiment: Labelling of the compounds with ⁶⁴Cu

Aqueous solution of a compound to be labelled (1.0 mM; 1.0 µL) was added to aqueous buffer solution MES-NaOH (1.0 M; pH 6.2; 10.0 µL) in Eppendorf plastic microvial (1.5 mL). The solution was incubated with mild agitation (750 oscillations/min) at 25°C for 10 min. To the mixture, freshly prepared [⁶⁴Cu]CuCl₂ solution in 10 mM aqueous HCl (6 µL; 9-10 MBq) was added. Then, the reaction mixture was incubated with mild agitation (750 oscillations/min) at 25°C for 10 min. After 10 min, the reaction mixture was analysed by TLC (SiO₂ 60 W F₂₅₄ S; aqueous EDTA-NaOH buffer, pH 5; *R*_{f}(free copper) = 0.8-0.9; *R*_{f}(complex) = 0). Examples of radiochemical yields (determined by RitaStar; Raytest) of selected compounds involving also examples of currently used ligands (in italics) are given below (for each ligand, labelling was carried out with three different batches-eluates of [⁶⁴Cu]CuCl₂):

| Compound | Radiochemical yield |
|---|---|
| **1** | 71±10% |
| **8** | 76±14% |
| **14** | 83±8% |
| **19** | 83±9% |
| **32** | 83±3% |
| **34** | 62±12% |
| **41** | 66±8% |
| *NOTA* | *18*±*11*% |
| *DOTA* | *9*±*7%* |
| *cb-TE2A* | *2*±*1*% |

Radiochemical yields for the compounds which are subject of the patent application were, under identical conditions, several times better than those of commercial ligands as NOTA, DOTA a *cb*-TE2A.

### Example 63: Labelling of compound 34 with ¹⁷⁷Lu

Aqueous solution of the non-carrier-added [¹⁷⁷Lu]LuCl₃ in 0.01 M HCl (50 µL, ca. 12 MBq activity) was added to aqueous solution of NH₄OAc (0.2 mL of the buffer; pH 5.5; 1 M) in a 1-mL vial. To the solution, a solution of the compound **34** in ultrapure water was added (40 µL, *c* = 50 µM) and the solution was agitated at 90 °C for 20 min. ITLC analysis (1 M aqueous NH₄OAc : MeOH; 1:1 v/v) confirms presence of the radiochemically pure labelled complex (complex with *R*_{f} ∼0.9; free ¹⁷⁷Lu has *R*_{f} = 0).

### Example 64: Labelling of compound 37 with ²¹³Bi

Aqueous solution of NaOAc (pH 5.5; 1 M; 1.6 mL) was added to the non-carrier-added ²¹³Bi³⁺ present as a solution in 0.15 M aqueous KI (1.4 mL) with a nominal activity ca. 150 MBq (i.e. ca. 107 MBq/mL). To the solution of compound **37** in ultrapure water (10 µL, *c* = 100 µM), a solution containing [²¹³Bi]Bi³⁺ (90 µL, ca. 10 MBq) was added and the solution was incubated at 70 °C for 10 min. Paper liquid chromatography (0.1 M aqueous Na-citrate) showed a quantitative complex formation (*R*_{f}(complex) <0.5, ²¹³Bicitrate moves with the solvent front).

### Example 65: Synthesis of targeting conjugate of the compound 39 and the Rituximab antibody

Water used to prepare reaction solutions and used for purifications in the Example is deionized water which was purified from possible metal impurities by ion exchange on Chelex 100 chelating resin. Throughout the procedure, sterile glassware and plastics was used, used commercial chemicals have the highest available purity in a view of metal ion content.

Aqueous solution (4 mL) of Rituximab antibody (0.14 µmol) having concentratio 5.0 mg/mL was concentrated by ultrafiltration in MWCO-30 kDa Vivaspin 6 vial to 1 mL (final concentration 20 mg/mL). This concentrated antibody solution was incubated with a solution of compound **39**·3.5H₂O (1.1 mg; 1.4 µmol, antibody:ligand 1:10 molar ratio) in anhydrous DMSO (200 µl; dissolved under sonification, sterilized by filtration through syringe microfilter; Millipore, 0.22 µm). Immediately after the reaction mixture preparation, the solution pH was adjusted to 8.2-8.6 with 0.1 M aqueous NaOH solution. The reaction mixture was agitated (Vortex, 450 rev./min.) at room temperature for 2 h. One hour after start of the reaction, pH was re-adjusted to 8.2-8.6 (0.1 M aqueous NaOH). The conjugation was terminated by TRIS-HCl buffer solution (1.0 M; pH 8.8; 50 µL) addition. The conjugate solution was purified from unreacted ligand and other low-molecular-weight impurities by triplicate ultrafiltration with utilization of Vivaspin 6. The ultrafiltration was carried out by the following procedure. The mixture was transferred into washed sterilized Vivaspin 6 vial and the solution volume was adjusted to 6 mL with 0,1 M PBS buffer (pH 8.5). Then, upper part of the Vivaspin vial was incubated in Vortex (1000 oscillations/min) for 5 min. Then, the solution was centrifugated at 4000 rev./min for 45 min to get 0.3 mL solution volume in the upper part of the Vivaspin vial. The antibody solution in the upper part of the vial was adjusted to 6 mL with 0,05 M aqueous NH₄OAc (pH 7.0), and the step vortexing-centrifugation was repeated (carried out 2-times). After the third centrifugation, the solution containing the antibody was added with water to get the final volume 3 mL. The conjugate purity was checked by spectrophotometry at 280 nm and also by HPLC (UV detection, 280 nm); degradation test and test of the antibody significant losses. The purified conjugate must be stored in the fridge at 4-8 °C temperature. An average number of the chelates per the antibody molecule was determined by MS-MALDI/TOF to be 3.

### Example 66: Labelling of targeting conjugate of the compound 39 and the Rituximab antibody with 64-Cu

The conjugate solution from the Example 65 (0.5 mL) was transferred into Eppendorf plastic microvial (volume 1.5 mL) and, if necessary, its final volume (taking into account also the added radionuclide solution) was adjusted to 1 mL with 0.5 M NH₄OAc (pH 6.0). The solution was incubated with mild agitation (450 oscillations/min) at 25 °C for 15 min. To the mixture, non-carrier-added radiocopper [⁶⁴Cu]CuCl₂ solution in 0.01 M aqueous HC1 (0.12 mL; 200 MBq [⁶⁴Cu]CuCl₂) was added. Subsequently, solution pH was checked and was found to be 6.0. Then, the reaction mixture was incubated with mild agitation (450 oscillations/min) at 40 °C for 20 min. Once incubation was finished, the labelled conjugate was sterilized by filtration through syringe microfilter (Millipore; 0,22 µm). ITLC analysis (1 M aqueous NH₄OAc and MeOH; 1:1 v/v) carried out after addition of aqueous DTPA (to bind free 64-Cu) to the sterile sample showed radiochemically pure labelled conjugate (*R*_{f} = 0) and no free 64-Cu (Cu-DTPA, *R*_{f}∼0,9).

### Example 67: Synthesis of conjugate of the compound 39 and the PAMAM dendrimer of the second generation

Commercial form of the G2-PAMAM dendrimer (16 free amino groups; 20 % solution in MeOH; 0.15 mL; 10 µmol) was dissolved in H₂O (5 mL) and the compound **39** (127 mg; 160 µmol; equivalent amount per amino groups on the dendrimer) was dissolved in this solution. Solution pH was carefully adjusted to 9 with aqueous KOH solution and the reaction mixture was stirred at 40 °C for 12 h. Then, compound **39** (63 mg; 0,5 molar equivalent per dendrimer amino groups) was added again, pH was re-adjusted to 9 aqueous KOH solution and the solution was stirred at 40 °C for 12 h. Then, the solution was cooled down, solution pH was decreased to pH = 7 and the solution was filtrated through syringe microfilter (Millipore; 0.22 µm) into ultrafiltration cell. Ultrafiltration (under 3 atm pressure, 3-kDa membrane cut-off) was carried out three times from volume 50 mL to the retenate volume 10 mL, and it was followed by continuous ultrafiltration (4 atm) until 800 ml of the filtrate was obtained. The retenate was lyophilized to give white solids (167 mg; with the active compound content 72 %, the yield is 83 % based on the starting dendrimer). A number of ligand moieties in the dendrimer conjugate (¹H NMR spectrum) was estimated to be 15.5.
**NMR** (D₂O): ¹**H** *δ* 1,88-3,72 (C*H*₂, bm, 39,44H); 7,11-7,33 (CH, bm, 4,00H); **³¹P**{¹H} *δ* 19,0 (HO-*P*-OH, bm, 1P); 23,8 (*P*-*C*H₂-P, bm, 1P); 28,2 (*P*-CH₂-*P,* bm); 35,8 (*P*-*C*H₂-*P*, bm, 1P).
EA (*M*_{w} of the pure dendrimer conjugate ~14482): 31.4% C, 8.0% H, 9.6%N, 2.5% S; i.e. dendrimer conjugate content was 72 %.

### Example 68: Labelling of targeting conjugate of the compound 39 and the PAMAM dendrimer of the second generation with 64-Cu

The conjugate from Example 67 (5 mg, ca. 0.25 µmol, ca. 3.9 µmol of the macrocycle) was dissolved in ultrapure water (50 µL) in an Eppendorf plastic microvial (0.5 mL) and aqueous buffer HOAc/NaOAc (0.5 M, pH 5.8; 0.2 mL) was added. To the mixture, non-carrier-added [⁶⁴Cu]CuCl₂ in 0.01 M aqueous HCl (120 µL, 180-200 MBq [⁶⁴Cu]CuCl₂) was added. Subsequently, solution pH was checked and was found to be 5.8. Then, the reaction mixture was incubated with mild agitation (450 oscillations/min) at 50 °C for 20 min. It led to a solution containing the labelled dendrimer. The product was analysed by thin-layer chromatography (Instant Thin-Layer Chromatography ITLC) on plates with impregnated glass fibres as stationary phase and with 10 mM EDTA in 0.1 M aqueous NH₄OAc (pH 5.5) as a mobile phase (*R*_{f} = 0-0.1) and radioactivity was detected only in the spot of the dendrimer.

### Example 69: Synthesis of targeting conjugate with folic acid, based on conjugate 48

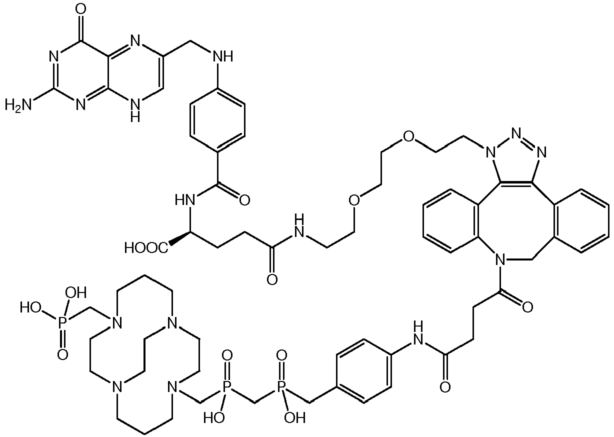

In a glass vial (4 mL), the weighted amount of conjugate **48**·2.5 H₂O (11 mg; 12 µmol; 1.0 equiv.) was dissolved in aqueous NH₄OAc buffer (500 mM; pH 5.0; 0.5 mL). To the solution, freshly prepared solution of δ-(1-azido-3,6-dioxaoktan-8-amide) of folic acid (8.6 mg; 14.4 µmol; 1.2 equiv.) in water (0.3 mL) was added. The mixture was stirred at room temperature for 4 h. The crude product was purified by preparative HPLC (C8; gradient elution H₂O-0.1% TFA-MeCN). Fraction with product was lyophilized. The final product was obtained as a mixture of regioisomers as a white substance (11.4 mg).
**MS:** (+) 1467.6 [M+H⁺]⁺, 1489.2 [M+Na⁺]⁺.

### Example 70: Synthesis of targeting conjugate with biotin, based on conjugate 49

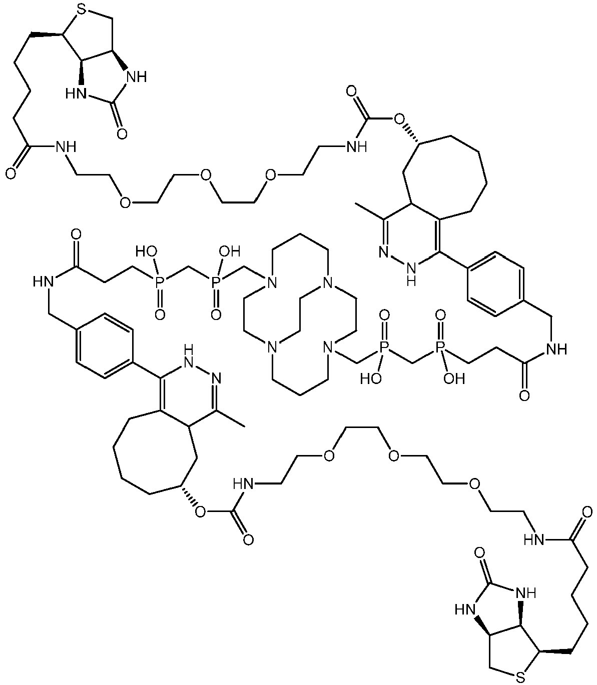

In a glass vial (4 mL), the weighted amount of conjugate **49**·1.5 H₂O (11.8 mg; 11 µmol; 1.0 equiv.) was dissolved in water, NH₄OAc (500 mM; pH 5.0; 0.5 mL). To the solution, freshly prepared solution of commercial biotin derivative TCO-PEG3-biotin (7.5 mg; 13.2 µmol; 1.2 equiv.) in water (0.4 mL) was added. The mixture was stirred at room temperature for 30 min. The crude product was purified by preparative HPLC (C8; gradient elution H₂O-0.1% TFA-MeCN). Fraction with product was lyophilized. The final product was obtained as a mixture of regioisomers as a white substance (15.2 mg).
**MS:** (+) 1590,5 [M+H⁺]⁺, 1612,8 [M+Na⁺]⁺.

### Example 71: Comparative experiment: Labelling of compounds according to the present invention and the compound cb-TE2P, representing the state-of-the-art, with ⁶⁴Cu

Structural formula of cb-TE2P

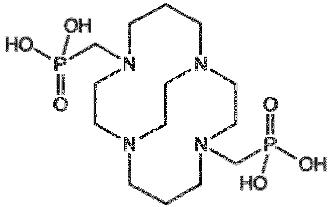

Aqueous solution of a compound to be labelled (1.0 mM; 1.0 µL) was added to aqueous buffer solution MES-NaOH (1.0 M; pH 5.5; 10.0 µL) in Eppendorf plastic microvial (1.5 mL). The solution was incubated with mild agitation (750 oscillations/min) at 25 °C for 10 min. To the mixture, freshly prepared [⁶⁴Cu]CuCl₂ solution in 10 mM aqueous HCl (6 µL; 9-10 MBq) was added. Then, the reaction mixture was incubated with mild agitation (750 oscillations/min) at 25 °C for 60 min. After 60 min, the reaction mixture was analysed by TLC (SiO₂ 60 W F₂₅₄ S; aqueous EDTA-NaOH buffer, pH 5; *R*_{f}(free copper) = 0.8-0.9; *R*_{f}(complex) = 0). Examples of radiochemical yields (determined by RitaStar; Raytest) of the selected compounds involving also examples of currently used ligands (in italics) are given below (for each ligand, labelling was carried out with at least three different batches-eluates of [⁶⁴Cu]CuCl₂). The same procedure was used at pH 6.2, but the reaction mixture was incubated only 10 min. Results are summarized in Table 1.

**Table 1: Radiochemical yield (%) in labelling of compounds of the present invention and their analogues at two pH values.**

| **LIGAND** | **pH 6.2** | **pH 5.5** |
|---|---|---|
| **8** | 76±14 | 79 |
| **32** | 83±3 | 82 |
| **37** | 90±13 | 99 |
| NOTA | 18±11 | 26 |
| DOTA | 9±7 | 35 |
| *cb*-TE2A | 2±1 | 1 |
| *cb*-TE2P | 46±7 | 5 |

Radiochemical yield for compounds which are subject of the present invention at pH 6.2 significantly surpasses those of the state-of-the-art ligands (NOTA, DOTA, *cb-*TE2A, *cb*-TE2P). In the labelling at pH 5.5 (it was chosen as, at this pH, there is a smaller complexation competition with Zn²⁺ a Ni²⁺ which are product of the ⁶⁴Cu radioactive decay), the difference between claimed compounds and the state-of-the-art ligands is huge.

### Example 72: Synthesis of compound 50

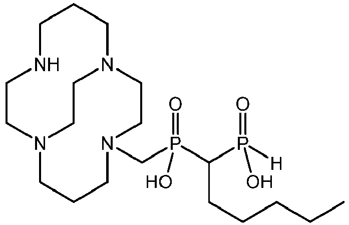

To a glass vial (20 mL), 1,4,8,11-tetraazabicyklo[6.6.2]hexadekan (881 mg; 3.89 mmol; 2.2 equiv.) was weighted. Subsequently, hexan-1,1-diyl-bis(phosphinic acid) (synthesized according to S. Gouault-Bironneau et al. Org. Lett. 2005, 7**,** 5909-5912; 1.14 g; 5.33 mmol; 3.0 equiv.), paraformaldehyd (53.0 mg; 1.77 mmol; 1.0 ekviv.) and aqueous HCl (5 M; 20 mL) were added. The suspension was stirred at 80 °C overnight. Next day, the reaction mixture was evaporated to dryness and the residue was several times co-evaporated with water. The crude product was purified by ion exchange resin chromatography (Amberlite IRA 402; OH⁻-form; H₂O → 10 % aqueous AcOH). Acetic acid fraction with product was evaporated to dryness and then several times co-evaporated with water. The residue was dissolved in water (100 mL) and the solution was lyophilized. The final product was obtained as a white hydroscopic material (412 mg; 52 %; 1 step; based on paraformaldehyde).
**NMR** (D₂O): **¹H** *δ* 0.85 (CH₃, s, 3H) 1.11-2.22 (C*H*₂-C*H*₂-C*H*₂-C*H*₂, *P*-C*H*-P, *cycle,* bm, 11H); 2.31 (*cycle,* m, 1H); 2.35 (*cycle,* m, 1H); 2.42-3.89 (*cycle,* N-C*H*₂-P bm, 22H); 7.09 (P*H*, m, 1H); ¹³C{¹H} *δ* 15.0 (C*H*₃, s); 16.2 (C*H*₂, m); 18.9 (*cycle,* s); 20.1 (*cycle,* s); 22.0 (C*H*₂, s); 28.3 (C*H*₂, s); 30.3 (C*H*₂, s); 41.2 (P-CH-P, m); 41.9 (*cycle,* s); 44.2 (*cycle,* s); 46.3 (*cycle,* s); 48.8 (*cycle,* s); 50.3 (*C*H₂-N-CH₂-P, d, ³*J*_{CP} = 8 Hz); 54.3 (*cycle,* s); 55.2 (N-*C*H₂-P, d, ¹*J*_{CP} = 91 Hz); 56.7 (*cycle,* s); 56.9 (*cycle,* s); 57.3 (*cycle,* s); 58.1 (*cycle,* s); **³¹P** *δ* 23.2 (*P*H, dm, 1P, ¹*J*_{PH} = 528 Hz); 29.8 (*P*-CH₂-N, m, 1P).
**MS:** (-) 451.4 [M-H⁺]⁻; 473.4 [M-2H⁺+Na⁺]⁻. (+) 453.5 [M+H⁺]⁺; 475.4 [M+Na⁺]⁺; 497.5 [M-H⁺+2Na⁺]⁺.
**EA** (C₁₉H₄₂N₄O₄P₂·3H₂O, *M*_{R} = 506.6): C 45.1 (45.4); H 9.6 (9.2); N 11.1 (10.9).

### Example 73: Synthesis of compound 51

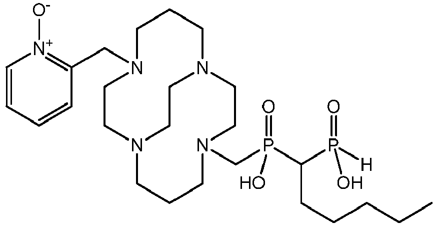

Into a glass vial (20 mL), compound **50**.3H₂O (224 mg; 501 µmol; 1.0 equiv.) a (chloromethyl)pyridine-*N*-oxide (205 mg; 1.25 mmol; 2.5 equiv.) were weighted and, subsequently, H₂O (10 mL) and LiOH·H₂O (630 mg; 15.0 mmol; 30 equiv.) were added. Subsequently, the reaction mixture was directly purified by ion exchange resin chromatography (DOWEX 50; H⁺-form; H₂O → 10 % aqueous pyridine). Pyridine fraction with product was evaporated to dryness and then several times co-evaporated with water. The crude product was further purified by preparative HPLC (C8; H₂O-0,1% TFA-MeCN). Fraction with the product was lyophilized. The final product was obtained as an off-yellow material (133 mg; 36 %; 1 step; based on **50** 3H₂O).
**NMR** (D₂O): **¹H** *δ* 0.87 (CH₃, s, 3H) 1.03-2.28 (C*H*₂-C*H*₂-C*H*₂-C*H*₂, P-C*H*-P, *cycle,* bm, 13H); 2.29-3.75 (*cycle,* N-C*H*₂-P bm, 22H); 4.25 (CH₂, bs, 2H); 7.09 (P*H*, dm, 1H, ¹*J*_{HP} = 533 Hz); 7.26-7.43 (*arom.,* m, 2H); 7.68 (*arom.,* d, 1H, ³*J*_{HH} = 7 Hz); 8.34 (*arom.,* d, 1H, ³*J*_{HH} = 5 Hz); **¹³C**{¹H} *δ* 13.9 (C*H*₃, s); 16.2 (C*H*₂, m); 18.9 (*cycle,* m); 20.0 (C*H*₂, m); 21.8 *(cycle,* s); 28.5 (C*H*₂, s); 30.4 (C*H*₂, s); 41.8 (P-*C*H-P, bm); 42.2 (*cycle,* s); 45.3 (*cycle,* s); 46.4 (*cycle,* s); 47.9 (*cycle,* s); 50.2 (*C*H₂-N-CH₂-P, d, ³*J*_{CP} = 8 Hz); 54.3 (*cycle,* s); 55,0 (N-*C*H₂-P, bm); 56.1 (*cycle,* s); 56.8 (*cycle,* s); 59.2 (*cycle,* s); 59.9 (*cycle,* s); 62.2 (C*H*₂, s); 125.4 (*arom.,* s); 126.8 (*arom.,* s); 143.1 (*arom.,* s); 145.0 (*arom.,* s); 156.0 (*arom.,* s); **³¹P** *δ* 25,8 (*P*H, dm, 1P, ¹*J*_{PH} = 533 Hz); 35,2 (*P*-CH₂-N, m, 1P).
**MS:** (-) 558.1 [M-H⁺]⁻; 580.0 [M-2H⁺+Na⁺]⁻. (+) 582.2 [M+Na⁺]⁺; 598.1 [M+K⁺]⁺; 604.2 [M-H⁺+2Na⁺]⁺.
**EA** (C₂₅H₄₇N₅O₅P₂·TFA·2H₂O, *M*_{R} = 733.7): C 47.5 (47.4); H 7.1 (7.3); N 9.9 (9.5).

### Example 74: Synthesis of compound 52

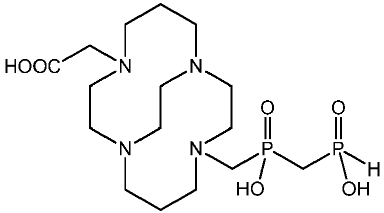

Into a glass vial (20 mL), compound **28**·2.5HCl·H₂O (230 mg; 468 µmol; 1.0 equiv.) and chloroacetic acid (134 mg; 1.42 mmol; 3.0 equiv.) were weighted, H₂O (10 mL) and solid LiOH·H₂O (398 mg; 9.48 mmol; 20 equiv.) were consecutively added and the mixture was heated at 60 °C for 2 days. Subsequently, the reaction mixture was directly purified by ion exchange resin chromatography (DOWEX 50; H⁺-form; H₂O → 10 % aqueous pyridine). Pyridine fraction with product was evaporated to dryness and then several times co-evaporated with water. The residue was dissolved in water (50 mL) and the solution was lyophilized. The final product was obtained as a white material (174 mg; 81 %; 1 step; based on **28**·2.5HCl·H₂O).
**NMR** (D₂O): **¹H** *δ* 1.72 (CH₂-C*H*₂-CH₂, m, 1H); 1.80 (CH₂-C*H*₂-CH₂, m, 1H); 2.08 (P-C*H*₂-P, t, 2H, ²*J*_{PH} = 16 Hz); 2.30 (CH₂-C*H*₂-CH₂, m, 1H); 2.30 (CH₂-C*H*₂-C*H*₂, m, 1H); 2.56 (*cycle,* m, 4H); 2.84 (*cycle,* m, 3H); 3.09 (*cycle,* m, 2H); 3.15 (*cycle,* m, 2H); 3.19 (N-C*H*₂-P, *cycle,* 2H); 3.27 (*cycle,* m, 3H); 3.36 (*cycle,* m, 2H); 3.63 (*cycle,* m, 1H); 3.75 (N-C*H*₂-P, m, 1H); 3.78 (*cycle,* m, 2H); 3.89 (C*H*₂-COOH, s, 2H); 7.14 (P*H*, d, 1H, ¹*J*_{HP} = 535 Hz); **¹³C**{¹H} *δ* 17.2 (CH₂-*C*H₂-CH₂, s); 19.8 (CH₂-*C*H₂-CH₂, s); 34.2 (P-*C*H₂-P, dd, *¹J*_{CP} = 85 Hz; ¹*J*_{CP} = 77 Hz); 42.0 (*cycle,* s); 48.1 (*cycle,* s); 49.4 (*cycle,* s); 49.5 (*cycle,* s); 52.1 (*C*H₂-N-CH₂-P, d, ³*J*_{CP} = 6 Hz); 54.0 (N-*C*H₂-P, d, ¹*J*_{CP} = 93 Hz); 54.4 (*cycle,* s); 55.8 *(cycle,* s); 56.8 (*C*H₂-COOH); 57.8 (*cycle,* s); 59.2 (*cycle,* s); 60.1 (*cycle,* s); 172.4 (CO, s); **³¹P** *δ* 19.9 (*P*H, dt, 1P, ¹*J*_{PH} = 535 Hz, ²*J*_{PH} = 16 Hz); 27.8 (*P*-CH₂-N, m, 1P).
**MS:** (-) 439.0 [M-H⁺]⁻. (+) 441.1 [M+H⁺]⁺; 463.2 [M+Na⁺]⁺; 479.1 [M+K⁺]⁺.
**TLC** (SiO₂, *i*PrOH-conc. aq. NH₄OH-H₂O 7:3:3): *R*_{f} = 0.2.
**EA** (C₁₆H₃₄N₄O₆P₂·H₂O, *M*_{R} = 458.4): C 41.9 (41.8); H 7.9 (7.6); N 12.2 (12.2).

### Example 75: Synthesis of compound 53

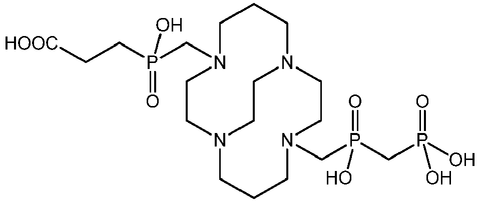

Into a glass vial (4 mL), compound **29·**1.5HCl·0.5H₂O (98.2 mg; 213 µmol; 1.0 equiv.) was weighted, and 2-carboxyethylphosphinic acid (89 mg; 644 µmol; 3.0 equiv.), paraformaldehyde (19.3 mg; 643 µmol; 3.0 equiv.) and aqueous HCl (6 M; 2 mL) were consecutively added. The suspension was stirred at 80 °C for 2 days. Next day, the reaction mixture was evaporated to dryness and the residue was several times co-evaporated with water. The crude product was purified by ion exchange resin chromatography (DOWEX 50; H⁺-form; H₂O). The fraction containing a pure product were unified and lyophilized. The final product was obtained as a white material (61.9 mg; 48 %; 1 step; based on **29**·1.5HCl·0.5H₂O).
**³¹P**{¹H} *δ* 19.8 (HO-*P*-OH, d, 1P, ²*J*_{PP} = 9 Hz); 27.3 (CH₂-*P*-CH₂, bm, 1P); 34.1 (CH₂-*P*-CH₂, bm, 1P).
**MS:** (-) 529.0 [M-(H₃O)⁺]⁻; 547.1 [M-H⁺]⁻. (+) 549.1 [M+H⁺]⁺; 571.2 [M+Na⁺]⁺; 587.2 [M+K⁺]⁺.
**EA** (C₁₈H₃₉N₄O₉P₃·3H₂O, *M*_{R} = 602.5): C 35.9 (36.3); H 7.5 (7.2); N 9.3 (9.3).

### Example 76: Synthesis of compound 54

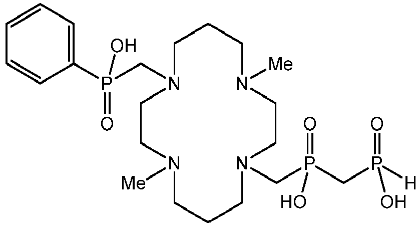

Into a glass vial (4 mL), compound **3**·3.5H₂O (112 mg; 250 µmol; 1.0 equiv.) was weighted, and phenylphosphinic acid (357mg; 2.51 mmol; 10.0 equiv.), paraformaldehyde (15.1 mg; 503 µmol; 2.0 equiv.) and aqueous HCl (12 M; 2 mL) were consecutively added. The suspension was stirred at 60 °C for 3 days. Subsequently, the reaction mixture was evaporated to dryness and the residue was several times co-evaporated with water. The crude product was purified by preparative HPLC (C8; H₂O-0.1 % TFA-MeCN). The fraction containing product was lyophilized. The final product was obtained as a white material (104 mg; 59 %; 1 step; based on **3**·3.5H₂O).
**NMR** (D₂O): **¹H** *δ* 1.73 (CH₂-C*H*₂-CH₂, p, 2H, ³*J*_{HH} = 7 Hz); 1.79 (CH₂-C*H*₂-CH₂, p, 2H, ³*J*_{HH} = 7 Hz); 2.03 (P-C*H*₂-P, dd, 2H, ²*J*_{HP} = 17 Hz, ²*J*_{HP} = 15 Hz); 2.28 (C*H*₃, s, 3H); 2.33 (C*H*₃, s, 3H); 2.44 (*cycle,* t, 2H, ³*J*_{HH} = 7 Hz); 2.48 (*cycle,* m, 4H); *2.71* (*cycle,* t, 2H, ³*J*_{HH} = 6 Hz); 2.66-3.16 (*cycle,* N-C*H*₂-P, m, 12H); 7.09 (PH, dm, ¹*J*_{HP} = 534 Hz); 7.48 (*arom.,* m, 2H); 7.55 (*arom.,* m, 1H); 7.73 (*arom.,* m, 2H); **¹³C**{¹H} *δ* 23.1 (CH₂-*C*H₂-CH₂, s); 23.4 (CH₂-*C*H₂-CH₂, s); 35.9 (P-*C*H₂-P, dd, *¹J*_{CP} = 77 Hz, *¹J*_{CP} = 75 Hz); 43.0 (*cycle,* s); 43.3 (*C*H₃, s); 43.6 (*C*H₃, s); 44.5 (*cycle,* s); 51.0 (*cycle,* d, ³*J*_{CP} = 8 Hz); 52.2 (*cycle,* s); 54.1 (*cycle,* s); 54.3 (*cycle,* s); 54.7 (*cycle,* d, ³*J*_{CP} = 6 Hz); 55.3 (*cycle,* s); 55.5 (N-*C*H₂-P, d, *¹J*_{CP} = 109 Hz); 56.9 (N-*C*H₂-P, d, ¹*J*_{CP} = 101 Hz); 128.8 (*arom.,* d, ³*J*_{CP} = 14 Hz); 131.0 (*arom.,* d, ²*J*_{CP} = 12 Hz); 131.7 (*arom*., d, ¹*J*_{CP} = 135 Hz); 132.8 (*arom.,* d, ⁴*J*_{CP} = 3 Hz); **³¹P** *δ* 23.6 (*P*H, dtm, 1P, ¹*J*_{PH} = 534 Hz, ²*J*_{PH} = 18 Hz); 26.7 (N-CH-*P*, m, 1P) 34.2 (N-CH-*P*, m, 1P).
**MS:** (-) 537.2 [M-H⁺]⁻. (+) 539.4 [M+H⁺]⁺; 561.3 [M+Na⁺]⁺; 577.2 [M+K⁺]⁺.
**EA** (C₂₁H₄₁N₄O₆P₃·TFA·3H₂O, *M*_{R} = 706.6): C 39.1 (39.1); H 6.9 (6.6); N 7.9 (8.3).

### Example 77: Synthesis of compound 55

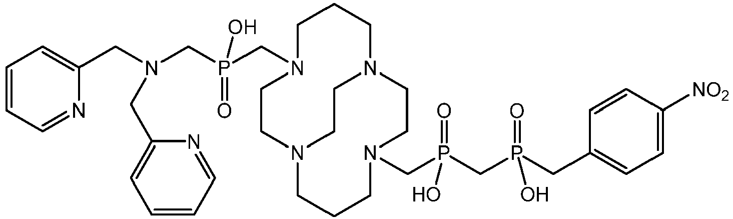

Into a glass vial (20 mL), compound **35·**5.5H₂O (185 mg; 300 µmol; 1.0 equiv.) was weighted, and [di(2-picolyl)amino]methylphosphinic acid (synthesized according to J. Yu et al. J. Am. Chem. Soc. 2015, 137, 14173-14179; 200 mg; 721 µmol; 2.4 equiv.), paraformaldehyde (36.4 mg; 1.21 mmol; 4.0 equiv.) and aqueous HCl (6 M; 4 mL) were consecutively added. The suspension was stirred at 80 °C for 2 days. Subsequently, the reaction mixture was evaporated to dryness and the residue was several times co-evaporated with water. The crude product was purified by preparative HPLC (C8; H₂O-0.1 % TFA-MeCN). The fraction containing product was lyophilized. The final product was obtained as an off-yellow material (203 mg; 74 %; 1 step; based on **35**·5.5H₂O).
**NMR** (D₂O): **¹H** *δ* 1.73 (CH₂-C*H*₂-CH₂, m, 1H); 1.79 (CH₂-C*H*₂-CH₂, m, 1H); 2.08 (P-C*H*₂-P, m, 2H); 2.33 (CH₂-C*H*₂-CH₂, m, 1H); 2.38 (CH₂-C*H*₂-CH₂, m, 1H); 2.63 (*cycle,* bm, 4H); 2.82-3.01 (*cycle,* bm, 5H); 3.07-3.35 (*cycle,* P-C*H*₂-C, N-C*H*₂-P, bm, 13H); 3.42-3.57 (*cycle,* N-C*H*₂-P bm, 4H); 3.62-3.83 *(cycle,* N-C*H*₂-P, bm, 4H); 4.11 (C*H*₂-N-C*H*₂, s, 4H), 7.44 (*arom.,* m, 6H); 7.96 (*arom.,* t, 2H, ²*J*_{HH} = 8 Hz); 8.23 (*CH-C-N,* d, 2H, ²*J*_{HH} = 8 Hz); 8.40 (*arom.,* dm, 2H, ²*J*_{HH} = 6 Hz); **¹³C**{¹H} *δ* 19.2 (CH₂-*C*H₂-CH₂, s); 20.7 (CH₂-*C*H₂-CH₂, s); 33.2 (P-*C*H₂-P, dd, ¹*J*_{CP} = 85 Hz, ¹*J*_{CP} = 81 Hz); 40.8 (P-*C*H₂-C, d, ¹*J*_{CP} = 86 Hz); 42.4 (*cycle,* s); 48.2 (*cycle,* s); 49.5 (*cycle,* s); 50.0 (*cycle,* s); 52.6 (*cycle,* d, ³*J*_{CP} = 7 Hz); 54.2 (*cycle,* s); 55.5 (P-*C*H₂-N, d, ¹*J*_{CP} = 90 Hz); 57.2 (P-*C*H₂-N, d, ¹*J*_{CP} = 97 Hz); 57.5 (P-*C*H₂-N, d, ¹*J*_{CP} = 106 Hz); 59.1 *(cycle,* s); 59.7 (*cycle,* s); 60.2 (*cycle,* s); 60.7 (*C*H₂-N-*C*H₂, s, 4H); 123.8 (*C*H-C-N, s); 125.3 (*arom.,* s); 126.9 (*arom.,* s); 130.8 (*C*H-C-CH₂, d, ³*J*_{CP} = 5 Hz); 142.7 (*arom.,* s); 144.0 (CH-*C*-CH₂, d, ²*J*_{CP} = 8 Hz); 145.4 (*arom.,* s); 147.0 (*arom.,* s); 155.3 (*arom.,* s); **³¹P**{¹H} *δ* 24.8 (CH₂-*P*-CH₂, d, 1P, ²*J*_{PP} = 10 Hz); 30.8 (CH₂-*P*-CH₂, bs, 1P), 33.1 (CH₂-*P*-CH₂, s, 1P);
**MS:** (-) 805.2 [M-H⁺]⁻. (+) 807.3 [M+H⁺]⁺; 829.3 [M+Na⁺]⁺; 845.3 [M+K⁺]⁺; 851.3 [M-H⁺+2Na⁺]⁺.
**EA** (C₃₅H₅₃N₈O₈P₃·2TFA·H₂O, *M*_{R} = 1052.8): C 44.5 (44.5); H 5.5 (5.4); N 10.6 (10.4).

### Example 78: Synthesis of compound 56

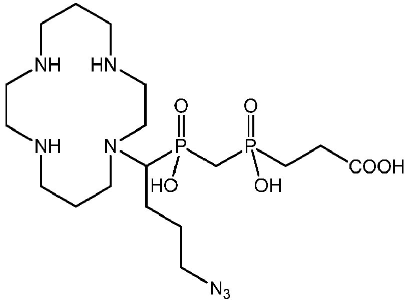

Into a glass vial (20 mL), 1,4,8,11-tetraazacyclotetradecane (463 mg; 2.31 mmol; 4.0 equiv.) was weighted, and, compound **D** (224 mg; 1.04 mmol; 1.8 equiv.), 4-azido-butanal (64.8 mg; 573 µmol; 1.0 equiv.), and aqueous HCl (6 M; 5 mL) were consecutively added. The suspension was stirred at 60 °C overnight. On next day, the reaction mixture was evaporated to dryness and the residue was several times co-evaporated with water. The crude product was purified by ion exchange resin chromatography (DOWEX 50; H⁺-form; H₂O → 10 % aqueous pyridine). The pyridine fraction containing product was evaporated to dryness and the residue was several times co-evaporated with water. Subsequently, the crude product was purified by preparative HPLC (C8; H₂O-0.1 % TFA-MeCN). The fraction containing product was lyophilized. The final product was obtained as a white material (212 mg; 70 %; 1 step; based on 4-azido-butanal).
**NMR** (D₂O): **¹H** *δ* 1.52 (C*H*₂-N₃, t, 2H, ³*J*_{HH} = 7 Hz); 1.65-2.28 (P-C*H*₂-P, C*H*₂, *cycle,* bm, 12H); 2.44-3.52 (*cycle,* bm, 19H); **¹³C**{¹H} *δ* 19.8 (*C*H₂, s); 22.8 (*cycle,* s); 24.4 (*C*H₂, s); 26.5 *(cycle,* s); 28.2 (P-*C*H₂-C, d, ¹*J*_{CP} = 93 Hz); 30.3 (*C*H₂-CO, s); 34.2 (P-*C*H₂-P, t, ¹*J*_{CP} = 76 Hz); 44.2 (*cycle,* s); 45.3 (*cycle,* s); 46.8 (*cycle,* s); 47.0 (*cycle,* s); 49.5 (*cycle,* s); 49.8 (*cycle,* d, ³*J*_{CP} = 12 Hz); 50.0 (*cycle,* s); 50.5 (*cycle,* s); 52.2 (*C*H₂-N₃, s); 53.1 (N-*C*H-P, d, ¹*J*_{CP} = 109 Hz); 177.2 (*C*O, d, ³*J*_{CP} = 14 Hz); **³¹P**{¹H} *δ* 28.4 (*P*-CH₂-C, d, 1P, ²*J*_{PH} = 12 Hz); 34.4 (N-CH-P, m, 1P).
**MS:** (-) 492.2 [M-(H₃O)⁺]⁻; 510.2 [M-H⁺]⁻.
**EA** (C₁₈H₃₉N₇O₆P₂·H₂O, *M*_{R} = 529.5): C 40.8 (50.0); H 7.8 (7.7); N 18.5 (18.6).

### Example 79: Synthesis of compound 57

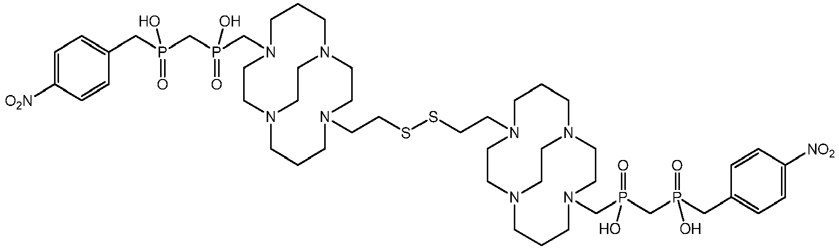

Into a glass vial (20 mL), 35·5.5H₂O (145 mg; 235 µmol; 4.0 equiv.) and bis(2-bromoethyl)disulphide (16.5 mg; 59 µmol; 1.0 equiv.) were weighted, and dry MeCN (10 mL) and anhydrous K₂CO₃ (163 mg; 1.18 mmol; 20 equiv.) were consecutively added. The mixture was stirred at room temperature for 5 days. Subsequently, the mixture was filtered through syringe filter (Millipore; 0.22 µm). The filtrate was purified by preparative HPLC (C8; H₂O-0.1% TFA-MeCN). The fraction containing product was lyophilized. The final product was obtained as an off-yellow material (58.5 mg; 69 %, 1 step, based on bis(2-bromoethyl)disulphide).
**³¹P**{¹H} *δ* 30.4 (CH₂-*P*-CH₂, bm, 2P); 33.2 (CH₂-*P*-CH₂, m, 2P).
**MS:** (-) 1173.5 [M-2H⁺+Na⁺]⁻. (+) 1152.6 [M+H⁺]⁺+ 1175.7 [M+Na⁺]⁺; 1191.6 [M+K⁺]⁺; 1197.6 [M-H⁺+2Na⁺]⁺; 1219.7 [M-2H⁺+3Na⁺]⁺.
**EA** (C₄₆H₈₀N₁₀O₁₂P₄S·2TFA·3H₂O, *M*_{R} = 1435.3): C 41.8 (41.4); H 6.2 (5.9); N 9.8 (9.6).
Disulphidic (oxidized) form of thiols is a stable form suitable for storage and the thiol group could be easily generated from them by *in-situ* reaction with mild reductive agents as tris(2-carboxyethyl)phosphine (TCEP).

### Example 80: Conjugate of compound 35 with a compound targeting the PSMA receptor

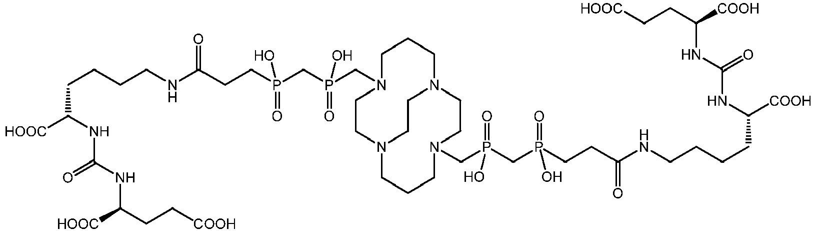

Into a glass vial, compound **35**·2H₂O (79.0 mg; 110 µmol; 1.0 equiv.) was weighted. The following reagents were added in the consecutive order: (*S*)-di-*t*-butyl 2-{3-[(*S*)-6-amino-1-*t*-butoxy-1-oxohexan-2-yl]ureido}pentanedioate (prepared according to R. P. Murelli et al. J. Am. Chem. Soc. 2009, 131, 17090-17092; 108 mg; 220 µmol; 2.2 equiv.), dry DMSO (2 mL), *N*,*N*-diisopropylethylamine (96 µL; 550 µmol; 5.0 equiv.) and TBTU (142 mg; 442 µmol; 4.0 equiv.). After stirring at room temperature for 30 min, another *N*,*N*-diisopropylethylamine (96 µL; 550 µmol; 5.0 equiv.) and TBTU (142 mg; 442 µmol; 4.0 equiv.) were added. After stirring for further 30 min, the mixture was directly purified by preparative HPLC (C8; gradient elution H₂O-0.1 % TFA-MeCN). The fraction containing product was evaporated to dryness. The residue was dissolved in trifluoroacetic acid (2 mL) and the solution was stirred room temperature overnight. Then, the mixture was evaporated to dryness and co-evaporated with water several times. The residue was dissolved in water (50 mL) and lyophilized. The final product was obtained as a white material (79.2 mg; 54 %; based on **35**·2H₂O).
**NMR** (D₂O): **¹H** *δ* 1.45 (C*H*₂, m, 4H); 1.57 (C*H*₂, p, 4H, ³*J*_{HH} = 5 Hz); 1.68 (C*H*₂, m, 4H); 1.76-2.33 (*cycle,* C*H*₂, P-C*H*₂-C bm, 10H); 2.39 (C*H*₂-CO, t, 4H, ³*J*_{HH} = 8 Hz); 2.61-3.92 (*cycle,* C*H*₂-*N*-CO, N-C*H*₂-P, bm, 28H); 4.36 (*CH,* t, 2H, ³*J*_{HH} = 7 Hz); 4.52 (*CH,* t, 2H, ³*J*_{HH} = 6 Hz); **³¹P**{¹H} *δ* 28.3 (CH₂-*P*-CH₂, bm, 2P); 36.0 (CH₂-*P*-CH₂, m, 2P).
**MS:** (-) 1284.2 [M-H⁺]⁻. (+) 1286.5 [M+H⁺]⁺; 1307.0 [M+Na⁺]⁺; 1323.3 [M+K⁺]⁺. **EA** (C₄₆H₈₄N₁₀O₂₄P₄·2.5H₂O, *M*_{R} = 1330.1): C 41.0 (41.4); H 6.9 (6,7); N 10.1 (10.5). The conjugates with other PSMA inhibitors, based on urea derivatives or on organophosphorus acids, can be obtained analogously.

### Example 81: Conjugate of compound 9 with a fluorescence label, BODIPY

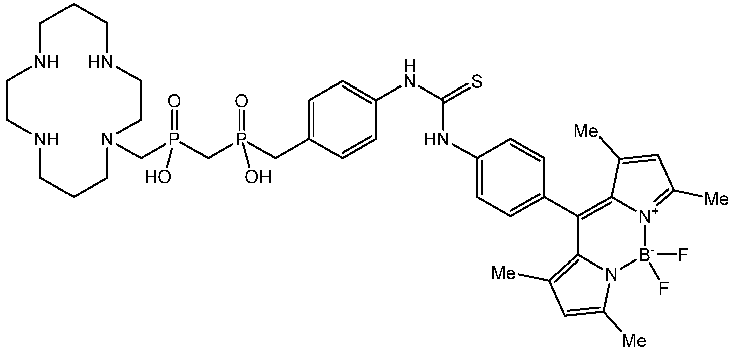

Into a glass vial, **9**·3H₂O (21.7 mg; 42.1 µmol; 2.5 equiv.) and -NCS derivative of phenyl-BODIPY (6.4 mg; 16.8 µmol; 1.0 equiv.) were weighted out and MeCN (250 µL) and aqueous buffer solution (MOPS-NaOH; 0.3 M; pH 8, 550 µL) were consecutively added. The mixture was stirred at room temperature overnight. Subsequently, the mixture was directly purified by preparative HPLC (C8; H₂O-0.1% TFA-MeCN). The fraction containing product was lyophilized. The final product was obtained as an orange material (10.3 mg; 73 %, 1 step, based on NCS derivative of bodipy).
**NMR** (D₂O): **³¹P**{¹H} *δ* 30.1 (*P*-CH₂-N, m, 1P); 31.8 (*P*-CH₂-N, s, 1P).
**MS:** (-) 841.2 [M-H⁺]⁻; (+) 843.3 [M+H⁺]⁺; 865.3 [M+Na⁺]⁺; 881.4 [M+K⁺]⁺.

### Example 82: Conjugate of compound 13 with rhodamine

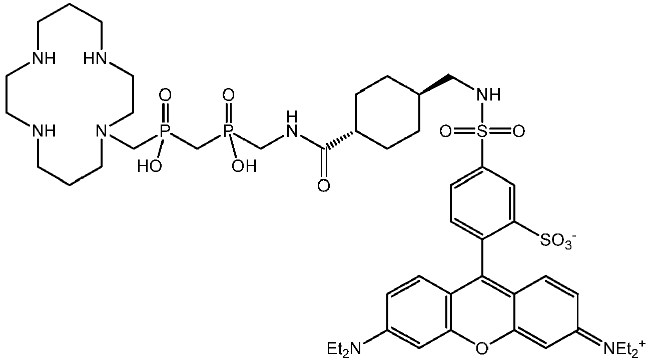

Into a glass vial, **13**·2.5H₂O (10.1 mg; 24.5 µmol; 2.1 equiv.) and *N-*[*trans*-4-(succinimidyloxycarbonyl)cyclohexylmethyl]sulfonamid-disulforhodaminu B (9.3 mg; 11.7 µmol; 1.0 equiv.) were weighted out and MeCN (500 µL) and aqueous buffer solution (MES-NaOH; 0.5 M; pH 7, 500 µL) were consecutively added. The mixture was stirred at room temperature two days. Subsequently, the mixture was directly purified by preparative HPLC (C8; H₂O-0.1% TFA-MeCN). The fraction containing product was lyophilized. The final product was obtained as a pink material (5.2 mg; 45%, 1 step, based on NCS ester of rhodamine B).
**NMR** (D₂O): **³¹P**{¹H} *δ* 29.8 (*P*-CH₂-N, bm, 1P); 36.2 (*P*-CH₂-N, m, 1P).
**MS:** (+) 993.5 [M+H⁺]⁺; 1015.4 [M+Na⁺]⁺.

### Example 83: Synthesis of compound 58

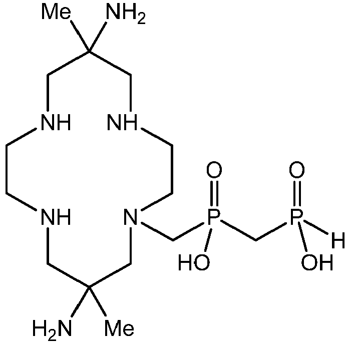

Into a glass vial (4 mL), compound **16·**3HCl·H₂O (48.3 mg; 80.3 µmol) was weighted out. Consequently, aqueous HCl (1 M; 2 mL) and zinc powder (ca. 100 mg) were added. The suspension was stirred at room temperature for 3 h. Subsequently, the mixture was filtered through syringe filter (Millipore; 0.22 µm). The filtrate was evaporated to dryness and the residue was dissolved in water (0.5 mL). Then, aqueous HCl (12 M; 1.5 mL) and *i*PrOH (2 mL) were added. The next day, the precipitate was centrifugated. The supernatant was decanted out the solid material was centrifugated with *i*PrOH (2×2 mL) and Et₂O (1×2 mL). The product was dried in vacuum desiccator. The final product was obtained as a white material (26.6 mg; 54 %, 1 step, based on **16**·3HCl·H₂O).
**NMR** (D₂O): **¹H** *δ* 1.82 (C*H*₃, s, 3H); 1.97 (C*H*₃, s, 3H); 2.15 (P-C*H*₂-P, dd, 2H, ²*J*_{HP} = 18 Hz, ²*J*_{HP} = 16 Hz); 2.64-3.34 (*cycle,* N-C*H*₂-P m, 18H); 7.13 (P*H*, d, 1H, ¹*J*_{HP} = 528 Hz); **¹³C**{¹H} *δ* 23.9 (*C*H₃, s); 25.3 (*C*H₃, s); 34.9 (P-*C*H₂-P, dd, ¹*J*_{CP} = 80 Hz, ¹*J*_{CP} = 76 Hz); 48.0 (*cycle,* s); 48.7 (*cycle,* s); 48.9 (*cycle,* s); 50.3 (*cycle,* s); 51.3 (*cycle,* s); 51.8 (*cycle,* s); 55.3 (*cycle,* s); 57.9 (*C*-NH₂, s); 58.2 (N-*C*H₂-P, d, *¹J*_{CP} = 101 Hz); 58.4 (*C*-NH₂, s); 59.3 (*cycle,* d, *³J*_{CP} = 10 Hz); **³¹P** *δ* 19.8 (PH, dt, 1P, ¹*J*_{PH} = 528 Hz, ²*J*_{PH} = 18 Hz); 33.7 (N-CH₂-*P*, m, 1P).
**MS:** (-) 413.2 [M-H⁺]⁻. (+) 415.3 [M+H⁺]⁺; 437.3 [M+Na⁺]⁺.
**TLC** (SiO₂, EtOH-conc. aq. NH₄OH 1:1): *R*_{f}= 0.4.
**EA** (C₁₄H₃₆N₆O₄P₂·5HCl·H₂O, *M*_{R} = 614.7): C 27.4 (27.2); H 7.1 (7.2); N 13.7 (14.0).

### Example 84: Synthesis of compound 59

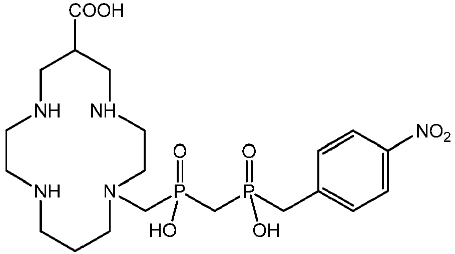

Into a glass vial (4 mL), tetrahydrochloride of 1,4,8,11-tetrazacyclotetradecane-6-carboxylic acid (388 mg; 994 µmol; 3.0 equiv.), compound **B** (135 mg; 484 µmol; 1.5 equiv.) and paraformaldehyde (9.8 mg; 327 µmol; 1.0 equiv.) were weighted, and aqueous HCl (6 M; 1 mL) and trifluoroacetic acid (1 mL) were added. The mixture was stirred at 60 °C for three days. The solution was evaporated to dryness and the residue was co-evaporated with water several times. The crude product was purified by preparative HPLC (C8; H₂O-0.1% TFA-MeCN). The fraction containing product was lyophilized. The final product was obtained as a white material (49.7 mg; 24 %, 1 step, based on paraformaldehyde).
**NMR** (D₂O): **¹H** *δ* 1.77 (*cycle,* p, 2H, ³*J*_{HH} = 4 Hz); 2.01 (P-C*H*₂-P, t, 2H, ²*J*_{HP} = 17 Hz); 2.33-2.99 (*cycle,* N-C*H*₂-P, m, 19H); 3.25 (C*H*₂-C-CH, d, 2H, ²*J*_{HP} = 16 Hz); 7.61 (C*H*-C-CH₂, d, 2H, ²*J*_{HH} = 8 Hz); 8.27 (C*H*-C-N, d, 2H, ²*J*_{HH} = 8 Hz); **³¹P**{¹H} *δ* 30.1 (CH₂-*P*-CH₂, d, 1P, ²*J*_{PP} = 11 Hz); 32.8 (CH₂-*P*-CH₂, d, 1P, ²*J*_{PP} = 11 Hz).
**MS:** (-) 515.9 [M+H⁺]⁺; 534.0 [M+H⁺]⁺. (+) 336.0 [M+H⁺]⁺.
**TLC** (SiO₂, EtOH-conc. aq. NH₄OH 1:1): *R*_{f} = 0.5.
**EA** (C₂₀H₃₅N₆O₈P₂·0.5TFA·2H₂O, *M*_{R} = 667.6): C 40.1 (39.9); H 6.3 (6.6); N 11.1 (10.8).

### Example 85: Synthesis of compound 60

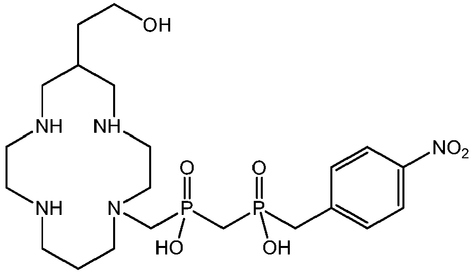

Into a glass vial (4 mL), 2-(1,4,8,11-tetrazacyclotetradecan-6-yl)ethanol hydrate (prepared according to N. Camus et al. RSC Adv. 2015, 5, 85898-85910; 157 mg; 598 µmol; 4.0 equiv.), compound **B** (97 mg; 348 µmol; 2.3 equiv.) and paraformaldehyde (4.5 mg; 150 µmol; 1.0 equiv.) were weighted, and aqueous HCl (12 M; 1 mL) was added. The mixture was stirred at 60 °C for two days. Consequently, water was added just to dissolve all solids. The solution was evaporated to dryness and the residue was co-evaporated with water several times. The crude product was purified by preparative HPLC (C8; H₂O-0.1% TFA-MeCN). The fraction containing product was lyophilized. The final product was obtained as an off-yellow material (22.4 mg; 22 %, 1 step, based on paraformaldehyde).
**NMR** (D₂O): **¹H** *δ* 1.68-1.75 (*cycle,* C*H*₂-C*H*₂-O, bm, 4H); 2.01 (P-C*H*₂-P, dd, 2H, ²*J*_{HP} = 17 Hz, ²*J*_{HP} = 15 Hz); 2.13 (*cycle,* m, 1H); 2.48-3.13 (*cycle,* N-C*H*₂-P, m, 18H); 3.32 (C*H*₂-C-CH, d, 2H, ²*J*_{HP} = 16 Hz); 3.65 (C*H*₂-OH, t, ³*J*_{HH} = 9 Hz); 7.58 (C*H*-C-CH₂, d, 2H, ²*J*_{HH} = 9 Hz); 8.17 (C*H*-C-N, d, 2H, ²*J*_{HH} = 9 Hz); **¹³C**{¹H} *δ* 24.9 (CH₂-*C*H₂-CH₂, s); 31.3 (CH₂-*C*H-CH₂, s); 32.9 (*C*H₂-CH₂-O, s); 34.2 (P-*C*H₂-P, dd, ¹*J*_{CP} = 82 Hz, ¹*J*_{CP} = 79 Hz); 40.5 (P-*C*H₂-C, d, ¹*J*_{CP} = 86 Hz); 44.1 (*cycle,* s); 45.0 (*cycle,* s); 45.6 (*cycle,* s); 45.9 (*cycle,* s); 46.2 (*cycle,* s); 47.1 (*cycle,* s); 55.1 (*cycle,* s); 55.2 (*cycle,* s); 58.3 (P-*C*H₂-N, d, ¹*J*_{CP} = 107 Hz); 62.4 (*C*H₂-OH, s); 125.6 (*C*H-C-N, s); 129.9 (*C*H-C-CH₂, d, ³*J*_{CP} = 5 Hz); 142.3 (CH-*C*-CH₂, s); 147.1 (CH-*C*-N, s); **³¹P**{¹H} *δ* 28.5 (CH₂-*P*-CH₂, d, 1P, ²*J*_{PP} = 10 Hz); 34.3 (CH₂-*P*-CH₂, d, 1P, ²*J*_{PP} = 10 Hz).
**MS:** (-) 534.2 [M-H⁺]⁻. (+) 536.2 [M+H⁺]⁺; 558.2 [M+Na⁺]⁺; 574.2 [M+K⁺]⁺.
**TLC** (SiO₂, *i*PrOH-conc. aq. NH₄OH-H₂O 7:3:3): *R*_{f}= 0.6.
**EA** (C₂₁H₃₉N₅O₇P₂·TFA·H₂O, *M*_{R} = 667.6): C 41.4 (41.7); H 6.3 (5.9); N 10.5 (10.3).

### Example 86: Synthesis of compound 61

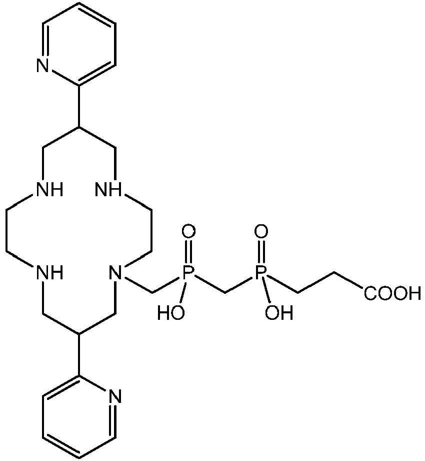

Into a glass vial (20 mL), tetrahydroperchlorate 6,13-di(2-pyridyl)-1,4,8,11-tetrazacyclotetradecane dihydrate (synthesized according to P. Comba et al. Inorg. Chem. 2001, 40, 2335-2345; 1.11 g; 1.40 mmol; 3.3 equiv.), compound **D** (128 mg; 592 µmol; 1.4 equiv.) and paraformaldehyde (12.7 mg; 423 µmol; 1.0 equiv.) were weighted, and aqueous HCl (12 M; 5 mL) was added. The mixture was stirred at 50 °C for two days. The reaction mixture was evaporated to dryness and the residue was co-evaporated with water several times. The crude product was purified by ion exchange chromatography (DOWEX 50; H⁺-form, H₂O → 10 % aq. pyridine). The pyridine fraction containing product was evaporated to dryness and the the residue was co-evaporated with water several times. The residue was then dried by vacuum pump obtained and further drying was accomplished with desiccator over P₂O₅. The final product was obtained as an off-yellow material (184 mg; 66 %, 1 step, based on paraformaldehyde).
**NMR** (D₂O): **¹H** *δ* 1.98 (P-*C*H₂-C, m, 2H); 2.08 (P-C*H*₂-P, t, 2H, ²*J*_{HP} = 17 Hz); 2.41 (C*H*₂-CO, m, 2H); 2.63-2.89 *(cycle,* N-C*H*₂-P, CH₂-C*H*-CH₂, m, 20H); 7.48 (*arom.,* m, 4H); 7.66 (*arom.,* dd, 2H, ³*J*_{HH} = 8 Hz, ³*J*_{HH} = 6 Hz); 8.38 (*arom.,* dm, 2H, ³*J*_{HH} = 6 Hz); **¹³**C{¹H} *δ* 29.5 (P-*C*H₂-C, d, ¹*J*_{CP} = 95 Hz); 31.6 (*C*H₂-CO, s); 34.0 (P-*C*H₂-P, t, ¹*J*_{CP} = 81 Hz); 40.3 (CH₂-*C*H-CH₂, s); 41.5 (CH₂-*C*H-CH₂, s); 44.0 (*cycle,* s); 46.5 (*cycle,* s); 46.9 (*cycle,* s); 47.2 (*cycle,* s); 47.8 (*cycle,* s); 50.3 (*cycle,* s); 54.5 (*cycle,* s); 55.1 (N-*C*H₂-P, d, *¹J*_{CP} = 109 Hz); 55.8 (*cycle,* s); 124.9 (*arom.,* s); 125.3 (*arom.,* s); 127.4 (*arom.,* s); 131.6 (*arom.,* s); 162.0 (*arom.,* s); 182.6 (*C*O, d, ³*J*_{CP} = 17 Hz); **³¹P**{¹H} *δ* 32.2 (*P*-CH₂-N, d, 1P, ²*J*_{PP} = 11 Hz); 37.8 (*P*-CH₂-CH₂, d, 1P, ²*J*_{PP} = 11 Hz).
**MS:** (-) 563.1 [M-H⁺]⁻; 581.0 [M-H⁺]⁻. (+) 583.2 [M+H⁺]⁺; 605.1 [M+Na⁺]⁺; 621.1 [M+K⁺]⁺.
**EA** (C₂₅H₄₀N₆O₆P₂·4H₂O, *M*_{R} = 654.6): C 45.9 (46.1); H 7.4 (7.3); N 12.8 (13.0).

### Example 87: Synthesis of conjugate of compound 38 with tetrazine

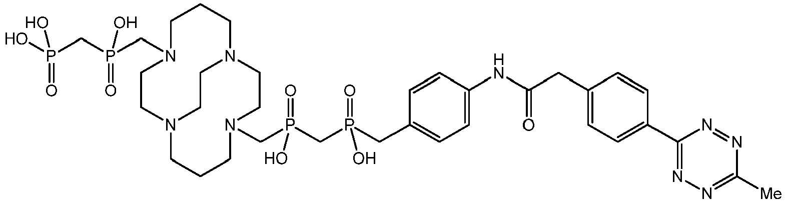

Into a glass vial (4 mL), compound **38**·4H₂O (33.3 mg; 45.5 µmol; 1.0 equiv.) and NHS ester of 2-(4-(6-methyl-1,2,4,5-tetrazin-3-yl)phenyl)acetic acid (16.4 mg; 50.1 µmol; 1.1 equiv.) were weighted, and aqueous buffer MES/NaOH (1.0 M; pH 6.2, 1.5 mL) and MeCN (1,5 mL) were added. The mixture was stirred at room temperature for two days. Consequently, water was then directly purified by preparative HPLC (C8; H₂O-0.1% TFA-MeCN). The fraction containing product was lyophilized. The final product was obtained as a pink material (19.2 mg; 43 %, 1 step, based on **38**·4H₂O). **NMR** (D₂O): **¹H** *δ* 1.97 (*cycle,* bm, 2H); 2.26 (*cycle,* bm, 2H); 2.31 (P-C*H*₂-P, t, 2H, ²*J*_{HP} = 16 Hz); 2.36 (P-C*H*₂-P, t, 2H, ²*J*_{HP} = 19 Hz); 2.71-3,86 (*cycle,* N-C*H*₂-P, P-C*H*₂-C, C*H*₃, bm, 29H); 3.93 (C*H*₂-CO, s, 2H); 7.38 (*CH,* d, 2H, ³*J*_{HH} = 8 Hz); 7.46 (C*H,* d, 2H, ³*J*_{HH} = 8 Hz); 7.67 (C*H,* d, 2H, ³*J*_{HH} = 8 Hz); 8.43 *(CH,* d, 2H, ³*J*_{HH} = 8 Hz); **¹³C**{¹H} 20.4 (CH₂-*C*H₂-CH₂, bm); 20.6 (CH₂-*C*H₂-CH₂, s); 20.7 (*C*H₃, s); 32.8 (2 × P-*C*H₂-P, m); 38.6 (P-*C*H₂-C, d, ¹*J*_{CP} = 91 Hz); 43.6 (*C*H₂-CO, s); 47.4-58.7 (12 × N-*C*H₂, bm); 126.7 (*C*H-C-N, s); 130.1 (CH-*C*-N, s); 131.8 (*C*H-C-CH₂, s); 132.2 (CH-*C*-CH₂, d, ²*J*_{CP} = 8 Hz); 164.7 (N-*C*-N, s); 167.9 (N-*C*-N, s); 173.2 (*C*O, s); **³¹P**{¹H} *δ* 16.0 (HO-*P*-OH, m, 1P); 22.5 (*P*-CH₂-N, bm, 1P); 23.5 (*P*-CH₂-N, bm, 1P); 38.8 (*P*-CH₂-C, m, 1P).
**MS:** (-) 870.3 [M-H⁺]⁻. (+) 872.5 [M+H⁺]⁺; 894.5 [M+Na⁺]⁺; 910.5 [M+K⁺]⁺.
**TLC** (SiO₂, *i*PrOH-conc. aq. NH₄OH-H₂O 7:3:3): *R*_{f} = 0.4.
**EA** (C₃₄H₅₃N₉O₁₀P₄·TFA, *M*_{R} = 985.8): C 43.9 (43.8); H 5.5 (5.3); N 12.8 (13.1).

### Example 88: Conjugation of compound 15 to copolymer containing poly(acrylamide)

Block copolymer of poly[*N*-(2-hydroxopropyl)methakrylamide and poly-L-lysine (ten L-Lys residues) was obtained according to K. Tappertzhofen et al. Macromolec. Biosci. 2015, 15, 1159-1173 (average *M*_{w} 14 kDa, one poly-L-Lys chain per the copolymer). Compound **15** (93 mg; 135 µmol; 1.5 equiv. per an amino group) and N-hydroxysuccinimide (NHS; 15.5 mg; 135 µmol; 1.5 equiv.) were dissolved in aqueous MES/NaOH buffer (pH 5.5; 0.5 M; 2 mL). Subsequently, a DMSO/H₂O 1:1 (3.0 mL) solution containing 140 mg (cca 90 µmol of amino groups; 1 equiv.) of the polymer was added. To this solution, solid EDC·HCl (26 mg; 135 µmol; 1.5 equiv.) was added and the mixture was stirred at room temperature for 2 days. Then, the reaction mixture was concentrated to 2 mL and the solution was filtered through a syringe filter (Millipore; 0.22 µm) into ultrafiltration cell. Ultrafiltration (pressure 3 atm) on 3-kDa membrane was carried out three times from 50 mL to 10 mL of the retenate and it was followed by continuous ultrafiltration (pressure 4 atm) until 1000 mL of eluate was obtained. The retenate was lyophilized to give a white solid (175 mg). An average ligand number bound to the polymer was determined (¹H NMR spectrum) to be ca. 8.0

### Example 89 Conjugate of compound 39 and poly(acrylamide) with free amino groups

Poly[*N*-(2-hydroxopropyl)methakrylamide with free amino groups was prepared the polymer active ester consecutive reactions, first with NH₂-(CH₂)₆NH-C(O)CH₂NHBoc and later with 2-hydroxopropylaminem, which were followed by Boc protecting group removal, according to L. Nuhn et al. Angew. Chem. Int. Ed. 2013, 52, 10652-10656 (average *M*_{w} 16 kDa, ca. 22 free amino groups per molecule). A buffer MOPS/NaOH (5 mL, pH 7.5, 0.5 M) solution was added to the polymer with free glycine amino groups (105 mg, ca. 140 µmol amino groups) and the compound **39** (111 mg; 140 µmol, equivalent amount to a number of the amine groups) and the solution was stirred at room temperature for 12 h.. The solution was filtered through a syringe filter (Millipore; 0.22 µm) into ultrafiltration cell. Ultrafiltration (pressure 3 atm) on 3-kDa membrane was carried out three times from 50 mL to 10 mL of the retenate and it was followed by continuous ultrafiltration (pressure 4 atm) until 1000 mL of eluate was obtained. The retenate was lyophilized to give a white solid (185 mg). An average ligand number bound to the polymer was determined (¹H NMR spectrum) to be 19.6

### Example 90 Conjugate of compound 57 and poly(acrylamide) through thiol coupling

Poly[*N*-(2-hydroxopropyl)methakrylamide with free maleimide groups was prepared the polymer active ester by consecutive reactions, first with aminohexamethylene-maleimide and later with 2-hydroxopropylaminem according to L. Nuhn et al. Angew. Chem. Int. Ed. 2013, 52, 10652-10656 (average *M*_{w} 18 kDa, ca. 19 free maleimide groups per polymer). Polymer (115 mg, ca. 120 µmol maleimide groups) was dissolved in aqueous buffer MOPS/NaOH (5 mL; 0.5 M, pH 7.5) and compound **57** (86 mg; 60 µmol) was added, i.e. equivalent amount of -SH groups per a number of reactive groups per polymer was used. The solution was bubbled with argon (10 min). Free -SH group was generated *in-situ* after addition of tris(2-karboxyethyl)phosphine hydrochloride (TCEP·HCl, 17 mg, 60 µmol) in the argon flow and the mixture was stirred with no air access for 12 h. In the argon flow, compound **57** (43 mg; 30 µmol, i.e. ca. 0.5 molar equivalent with regard to a number of reactive groups on the polymer) dissolved in aqueous buffer (2 mL; 0.1 M, pH 7.5) was added, it was followed by addition of TCEP·HCl (11 mg, cca 35 µmol) and the mixture was stirred with no air access for 12 h. The solution was filtered through a syringe filter (Millipore; 0.22 µm) into ultrafiltration cell. Ultrafiltration (pressure 3 atm) on 3-kDa membrane was carried out three times from 50 mL to 10 mL of the retenate and it was followed by continuous ultrafiltration (pressure 4 atm) until 900 mL of eluate was obtained. The retenate was lyophilized to give a white solid (185 mg). An average ligand number bound to the polymer was determined (¹H NMR spectrum) to be 16.5

## Claims

1. Cyclam based compounds of the general formula (I) 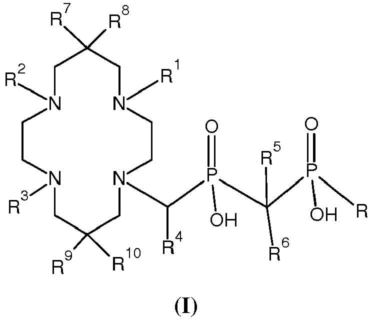 wherein
R, R^{a}, R¹, R², R³ are independently selected from the group comprising
H, OH, (C1 to C6)alkyl, which may be linear or branched, (C3 to C6)cycloalkyl, benzyl, and/or R¹ and/or R² and/or R³ is a bis-phosphorus acid of the general formula (II) 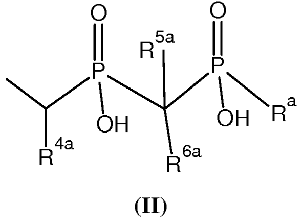
and/or R¹ and R³ together form (C2 to C3)alkylene, which may be substituted with one or more linear or branched (C1 to C6)alkyls,
and/or R, R^{a} are independently selected from phenyl and (C5 to C6)heterocycle, containing at least one heteroatom of N, S, O,
wherein the (C1 to C6)alkyl, (C3 to C6)cycloalkyl, phenyl, benzyl and (C5 to C6) heterocycle, containing at least one heteroatom of N, S, O, may be independently substituted with one or more groups selected from COOH; NH₂; NO₂, NX₂; C(O)NX₂; NHX; C(O)NHX; OH; SH; -NCS; tetrazine of the general formula **(III);** 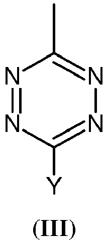 (C7 to C8)cycloalkenyl in *trans* configuration, which may eventually be further substituted with one or more groups selected from COOH, NH₂, NO₂, -NCS, -NCO, N₃ and SH; norbornenyl, eventually substituted with one or more groups selected from COOH, NH₂, NO₂, -NCS, -NCO, N₃ and SH; -N₃; (C5 to C6)heterocycle, containing at least one heteroatom of N, S, O; (C2 to C6)alkynyl, eventually substituted with one or more groups selected from COOH, NH₂, NO₂,-NCS, -NCO, N₃ and SH; cyclooctynyl, eventually substituted with one or more groups chosen from COOH, NH₂, NO₂, -NCS,-NCO, N₃ and SH; cycloazaoctynyl, eventually substituted with one or more groups selected from COOH, NH₂, NO₂, -NCS, -NCO, N₃ and SH; maleimide; phenyl; benzyl and -P(O)(OH)Z;
and wherein R⁴, R^{4a}, R⁵ and R^{5a} are independently H, (C1 to C6)alkyl, which may be linear or branched, (C3 to C6)cycloalkyl, benzyl;
wherein (C1 to C6)alkyl, (C3 to C6)cycloalkyl and benzyl may be independently substituted with one or more groups selected from COOH; NH₂; NO₂, NX₂; C(O)NX₂; NHX; C(O)NHX; OH; SH; -NCS; tetrazine of the general formula **(III);** (C7 to C8)cycloalkenyl in *trans* configuration, which may eventually be further substituted with one or more groups selected from COOH, NH₂, NO₂, -NCS, -NCO, N₃ and SH; norbornenyl, eventually substituted with one or more groups selected from COOH, NH₂, NO₂, -NCS, -NCO, N₃ and SH; -N₃; (C5 to C6)heterocycle, containing at least one heteroatom of N, S, O; (C2 to C6)alkynyl, eventually substituted with one or more groups selected from COOH, NH₂, NO₂, -NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂, -NCS, -NCO, N₃ and SH; maleimide; phenyl; benzyl and-P(O)(OH)Z;
and wherein R⁶ and R^{6a} is H;
and wherein R⁷, R⁸, R⁹ and R¹⁰ are independently selected from the group comprising H, (C1 to C6)alkyl, (C3 to C6)cycloalkyl, benzyl, phenyl, NO₂, COOH, (C5 to C6)heterocycle, containing at least one heteroatom of N, S, O, and/or altogether R⁷ and R⁸ contain (C2 to C3)alkylene or vinylene and/or altogether R⁹ and R¹⁰ contain (C2 to C3)alkylene or vinylene,
wherein (C1 to C6)alkyl, (C3 to C6)cycloalkyl, phenyl, benzyl, (C5 to C6)heterocycle, containing at least one heteroatom of N, S, O, (C2 to C3)alkylene and vinylene may be independently substituted with a group or groups selected from COOH; NH₂; NO₂; NX₂; C(O)NX₂; SH; tetrazine of the general formula **(III);** (C7 to C8)cycloalkenyl in *trans* configuration, eventually substituted with one or more groups selected from COOH, NH₂, NO₂, -NCS, -NCO, N₃ and SH; norbornenyl, eventually substituted with one or more groups selected from COOH, NH₂, NO₂, -NCS, -NCO, N₃ and SH;-N₃; (C5 to C6)heterocycle, containing at least one heteroatom of N, S, O; (C2 to C6)alkynyl, eventually substituted with one or more groups selected from COOH, NH₂, NO₂, -NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂, -NCS, -NCO, N₃ and SH; maleimide; phenyl; benzyl and -P(O)(OH)Z,
wherein Y is H, (C1 to C6)alkyl, -CH₂COOH, (C5 to C6)heterocycle, containing at least one heteroatom of N, S, O, or heteroaryl, containing at least one heteroatom of N, S, O, preferably Y is an atom of hydrogen, methyl or pyridyl;
wherein Z is H; OH; (C1 to C6)alkyl, which may be linear or branched; (C1 to C6)hydroxyalkyl; (C1 to C6)alkoxyl; phenyl; (C5 to C6)heterocycle, containing at least one heteroatom of N, S, O; or benzyl, wherein phenyl, (C5 to C6)heterocycle or benzyl may eventually be substituted with one or more groups selected from COOH, NH₂, NO₂, N₃ and SH, preferably the substituent is in *para* position;
and wherein X is independently H; (C1 to C6)alkyl, which may be linear or branched; (C3 to C6)cycloalkyl; phenyl and/or benzyl;
while for X, Y and Z applies, that (C1 to C6)alkyl, (C3 to C6)cycloalkyl, phenyl and/or benzyl may be independently substituted with one or more groups selected from COOH; NH₂; NO₂, NX₂; C(O)NX₂; NHX; C(O)NHX; SH; tetrazine of the general formula (**III**); (C7 to C8)cycloalkenyl in *trans* configuration, eventually substituted with one or more groups selected from COOH, NH₂, NO₂, -NCS, -NCO, N₃ and SH; norbornenyl, eventually substituted with one or more groups selected from COOH, NH₂, NO₂, -NCS, -NCO, N₃ and SH; -N₃; (C2 to C6)alkynyl, eventually substituted with one or more groups selected from COOH, NH₂, NO₂, -NCS, -NCO, N₃ and SH; cyclooctynyl, eventually substituted with one or more groups selected from COOH, NH₂, NO₂, -NCS, -NCO, N₃ and SH; cycloazaoctynyl, eventually substituted with one or more groups selected from COOH, NH₂, NO₂, -NCS, -NCO, N₃ and SH; maleimide; phenyl; benzyl and -P(O)(OH)Z;
wherein at least one of the groups R, R^{a}, R¹, R², R³, R⁴, R^{4a}, R⁵, R^{5a}, R⁶, R^{6a}, R⁷, R⁸, R⁹ and
R¹⁰ contains at least one of the groups -COOH; NH₂; NO₂, NX₂, NHX; C(O)NHX; N₃; SH; -NCS; tetrazine of the general formula (**III**); (C7 to C8)cycloalkenyl in *trans* configuration, eventually substituted with one or more groups selected from COOH, NH₂, NO₂, NX₂, -NCS, -NCO, NHX, C(O)NHX, N₃ and SH; norbornenyl, eventually substituted with one or more groups selected from COOH, NH₂, NO₂, NX₂, -NCS, -NCO, NHX, C(O)NHX, N₃ and SH; -N₃; (C2 to C6)alkynyl; cyclooctynyl, eventually substituted with one or more groups selected from COOH, NH₂, NO₂, NX₂, -NCS, -NCO, NHX, C(O)NHX, N₃ and SH; cycloazaoctynyl, eventually substituted with one or more groups selected from COOH, NH₂, NO₂, NX₂, -NCS, -NCO, NHX, C(O)NHX, N₃ and SH; maleimide; phenyl, eventually substituted with one or more groups selected from COOH, NH₂, NO₂, NX₂, -NCS, -NCO, NHX, C(O)NHX, N₃ and SH; benzyl, eventually substituted with one or more groups selected from COOH, NH₂, NO₂, NX₂, -NCS, -NCO, NHX, C(O)NHX, N₃ and SH; (C1 to C6)alkyl, eventually substituted with one or more groups selected from COOH, NH₂, NO₂, NX₂, -NCS, -NCO, NHX, C(O)NHX, N₃ and SH; and -P(O)(OH)Z.

2. The cyclam based compounds according to claim 1, **characterized in that** R¹ and R³ together form (C2 to C3)alkylene, which may be substituted with (C1 to C6)alkyl, which may be linear or branched.

3. The cyclam based compounds according to claim 1, **characterized in that** R, R^{a}, R¹, R², R³, R⁴, R^{4a}, R⁵, R^{5a}, R⁶ and R^{6a} are independently selected from the group comprising H; (C1 to C6)alkyl, which may be linear or branched; (C3 to C6) cycloalkyl; benzyl; phenyl; (C5 to C6)heterocycle, containing at least one heteroatom of N, S, O;
and/or R¹ and/or R² and/or R³ is the bis-phosphorus acid of the general formula (**II**), preferably at least one of the groups R¹, R² and R³ is the bis-phosphorus acid of the general formula (**II**), most preferably R² is the bis-phosphorus acid of the general formula (**II**).

4. The cyclam based compounds according to claim 1, **characterized in that** R² is selected from the group comprising H; (C1 to C6)alkyl, which may be linear or branched; (C3 to C6)cycloalkyl; benzyl; phenyl; (C5 to C6)heterocycle, containing at least one heteroatom of N, S, O; and bis-phosphorus acid of the general formula (**II**).

5. The cyclam based compounds according to claim 1, **characterized in that** they are selected from the group comprising compounds of the general formula (**I**), whose the substituents are represented in the following combinations:
| Compound number | R | R¹ | | R³ | R² | R⁴ | R⁵ | R⁶ | R⁷ | R⁸ | R⁹ | R¹⁰ |
|---|---|---|---|---|---|---|---|---|---|---|---|---|
| 1 | H | H | | H | H | CH₃ | H | H | H | H | H | H |
| 2 | H | CH₃ | | CH₃ | CH₃ | CH₃ | H | H | H | H | H | H |
| 3 | H | CH₃ | | CH₃ | H | H | H | H | H | H | H | H |
| 4 | H | CH₃ | | CH₃ | CH₃ | H | H | H | H | H | H | H |
| 5 | OH | benzyl | | benzyl | H | H | H | H | H | H | H | H |
| 7 | OH | H | | benzyl | benzyl | H | H | H | H | H | H | H |
| 8 | *p*-nitrobenzyl | H | | H | H | H | H | H | H | H | H | H |
| 9 | *p*-aminobenzyl | H | | H | H | H | H | H | H | H | H | H |
| 10 | *p*-SCN-benzyl | H | | H | H | H | H | H | H | H | H | H |
| 11 | *p*-azidobenzyl | H | | H | H | H | H | H | H | H | H | H |
| 12 | -CH₂N(BZ)₂ | H | | H | H | H | H | H | H | H | H | H |
| 13 | -CH₂NH₂ | H | | H | H | H | H | H | H | H | H | H |
| 14 | -(CH₂)₂COOH | H | | H | H | H | H | H | H | H | H | H |
| 15 | H | H | | H | H | H | -(CH₂)₄COOH | H | H | H | H | H |
| 16 | H | H | | H | H | H | H | H | CH₃ | NO₂ | NO₂ | CH₃ |
| 22 | H | CH₃ | | CH₃ | acid of formula (**II**), wherein R^{a} = R, R^{4a} to R^{6a} = H | H | H | H | H | H | H | H |
| 23 | -(CH₂)₂COOH | CH₃ | | CH₃ | acid of formula (**II**), wherein R^{a} = R, R^{4a} to R^{6a} = H | H | H | H | H | H | H | H |
| 24 | OH | benzyl | | benzyl | -CH₂P(O)(OH)₂ | H | H | H | H | H | H | H |
| 25 | OH | H | | H | -CH₂P(O)(OH)₂ | H | H | H | H | H | H | H |
| 26 | OH | -CH₂P(O)(OH)₂ | | benzyl | benzyl | H | H | H | H | H | H | H |
| 27 | OH | -CH₂P(O)(OH)₂ | | H | H | H | H | H | H | H | H | H |
| 30 | H | -(CH₂)₂- | | | H | CH₃ | H | H | H | H | H | H |
| 31 | *p*-nitrobenzyl | -(CH₂)₂- | | | H | H | H | H | H | H | H | H |
| 32 | H | -(CH₂)₂- | | | acid of formula (**II**), wherein R^{a} = R, R^{4a} to R^{6a} = H | H | H | H | H | H | H | H |
| 33 | -CH₂OH | -(CH₂)₂- | | | acid of formula (**II**), wherein R^{a} = R, R^{4a} to R^{6a} = H | H | H | H | H | H | H | H |
| 34 | OH | -(CH₂)₂- | | | acid of formula (**II**), wherein R^{a} = R, R^{4a} to R^{6a} = H | H | H | H | H | H | H | H |
| 35 | -(CH₂)₂COOH | -(CH₂)₂- | | | acid of formula **(II)**, wherein R^{a} = R, R^{4a} to R^{6a} = H | H | H | H | H | H | H | H |
| 36 | H | -(CH₂)₂- | | | acid of formula (**II**), wherein R^{a} = OH, R^{4a} to R^{6a} = H | H | H | H | H | H | H | H |
| 37 | *p*-nitrobenzyl | -(CH₂)₂- | | | acid of formula (**II**), wherein R^{a} = OH, R^{4a} to R^{6a} = H | H | H | H | H | H | H | H |
| 38 | *p*-aminobenzyl | -(CH₂)₂- | | | acid of formula (**II**), wherein R^{a} = OH, R^{4a} to R^{6a} = H | H | H | H | H | H | H | H |
| 39 | *p*-SCN-benzyl | -(CH₂)₂- | | | acid of formula (**II**), wherein R^{a} = OH, R^{4a} to R^{6a} = H | H | H | H | H | H | H | H |
| 40 | *p*-azidobenzyl | -(CH₂)₂- | | | acid of formula (**II**), wherein R^{a} = OH, R^{4a} to R^{6a} = H | H | H | H | H | H | H | H |
| 41 | *p*-nitrobenzyl | -(CH₂)₂- | | | -CH₂P(O)(OH)(CH₂OH) | H | H | H | H | H | H | H |
| 42 | *p*-nitrobenzyl | -(CH₂)₂- | | | -CH₂P(O)(OH)₂ | H | H | H | H | H | H | H |
| 43 | *p*-aminobenzyl | -(CH₂)₂- | | | -CH₂P(O)(OH)₂ | H | H | H | H | H | H | H |
| 50 | H | -(CH₂)₂- | | | H | H | -(CH₂)₄CH₃ | H | H | H | H | H |
| 51 | H | -(CH₂)₂- | | | 2-picolyl-*N*-oxide | H | -(CH₂)₄CH₃ | H | H | H | H | H |
| 52 | H | -(CH₂)₂- | | | -CH₂COOH | H | H | H | H | H | H | H |
| 53 | OH | -(CH₂)₂- | | | -CH₂P(O)(OH)-(CH₂)₂COOH | H | H | H | H | H | H | H |
| 54 | H | -CH₃ | -CH₃ | | -CH₂P(O)(OH)Ph | H | H | H | H | H | H | H |
| 55 | *p*-nitrobenzyl | -(CH₂)₂- | | | -CH₂P(O)(OH)-CH₂N(2-picolyl)₂ | H | H | H | H | H | H | H |
| 56 | -(CH₂)₂COOH | H | H | | H | -(CH₂)₃N₃ | H | H | H | H | H | H |
| 57 | *p*-nitrobenzyl | -(CH₂)₂- | | | -(CH₂)₂S-(dimer) | H | H | H | H | H | H | H |
| 58 | H | H | | H | H | H | H | H | -CH₃ | -NH₂ | -NH₂ | -CH₃ |
| 59 | *p*-nitrobenzyl | H | | H | H | H | H | H | -COOH | H | H | H |
| 60 | *p*-nitrobenzyl | H | | H | H | H | H | H | -(CH₂)₂OH | H | H | H |
| 61 | -(CH₂)₂COOH | H | | H | H | H | H | H | 2-pyridyl | H | 2-pyridyl | H |

6. A conjugate of the cyclam-based compound according to any one of the preceding claims and of at least one conjugation group, which is covalently bound to the cyclam based compound, and which is selected from the group containing OH, -COOH; NH₂; NO₂, NX₂, NHX; C(O)NHX; N₃; SH; -NCS; tetrazine of the general formula (**III**); (C7 to C8)cycloalkenyl in *trans* configuration, eventually substituted with one or more groups selected from COOH, NH₂, NO₂, NX₂, -NCS, -NCO, NHX; C(O)NHX; N₃ and SH; norbornenyl, eventually substituted with one or more groups selected from COOH, NH₂, NO₂, NX₂, -NCS, -NCO, NHX; C(O)NHX; N₃ and SH; -N₃; (C2 to C6)alkynyl; cyclooctynyl, eventually substituted with one or more groups selected from COOH, NH₂, NO₂, NX₂, -NCS, -NCO, NHX; C(O)NHX; N₃ and SH; cycloazaoctynyl, eventually substituted with one or more groups selected from COOH, NH₂, NO₂, NX₂, -NCS, -NCO, NHX, C(O)NHX, (C1 to C6)alkyl, (C6)aryl, N₃ and SH; maleimide; and -P(O)(OH)Z; wherein the conjugate may further contain a spacer between the cyclam-based compound and the conjugation group and/or between two conjugation groups, the spacer selected from the group containing (C1 to C6)*n*-alkyl, eventually substituted with C=O and/or-NH- group; phenylene; amino-acids chains with the length of 1 to 5 amino-acids; polyethylene glycols of 1 to 10 monomeric units.

7. Coordination compounds of the cyclam based compounds of the general formula (**I**) according to any one of the claims 1 to 5 or of the conjugates according to the claim 6, with metal cations selected from the group consisting of Cu²⁺, Bi³⁺, lanthanide(III) cations, Sc³⁺, Y³⁺, Pb²⁺, Zr⁴⁺, Ac³⁺, Mn²⁺ and Mn³⁺, preferably lanthanide(III) cations, Y³⁺ a Cu²⁺, more preferably Lu³⁺, Y³⁺ and CU²⁺.

8. A targeting conjugate, which contains the cyclam based compound of the general formula (I) according to any one of the claims 1 to 5 and/or the conjugate according to the claim 6 and/or the coordination compound according to the claim 7; and a targeting vector, selected from the group comprising bis(phosphonates); groups capable of fluorescence, preferably fluoresceines, rhodamines or the boron-dipyrromethene type substances; oligopeptides of 1 to 15 aminoacids; antibodies or fragments thereof, preferably the Fab fragments; folic acid; biotin; compounds targeting to PSMA receptor, preferably urea derivatives or organo-phosphorous compounds; cyclodextrins; dendrimers; hydrophilic polymers on the basis of derivatives of acrylic acid, especially hydroxoalkylamides of acrylic acid.

9. A method of preparation of cyclam based compounds of the general formula (**I**) according to any one of the claims 1 to 5, wherein an intermediate product of the general formula (**IV**), 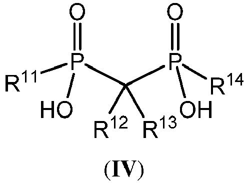 wherein R¹¹, R¹², R¹³ and R¹⁴ are in the following combinations:
| Intermediate | R¹¹ | R¹² | R¹³ | R¹⁴ |
|---|---|---|---|---|
| A | H | H | H | OH |
| B | H | H | H | *p*-nitrobenzyl |
| C | H | H | H | -CH₂N(benzyl)₂ |
| D | H | H | H | -(CH₂)₂COOH |
| E | H | -(CH₂)₄COOH | H | H |
| F | OH | -(CH₂)₃NH(4-dibenzylcyclooktyn amidobutoxyl) | OH | OH |
or a compound selected from the group containing methylene-bis(phosphinic acid), phosphorous acid, hydroxymethylphosphinic acid, reacts with a cyclam derivative and an aldehyde in water solution of an acid of a concentration within the range of from 10 to 40 % (weight), preferably from 18 to 36 % (weight), at the temperature in the range of from 40 °C to 80 °C for the period of time of at least 12 hours;
wherein the cyclam derivative is 1,4,8,11-tetraazacyclotetradecane and derivatives thereof and 1,4,8,11-tetraazabicyclo[6.6.2]hexadecane and derivatives thereof, preferably the cyclam derivative is selected from the group containing 1,4,8,11-tetraazacyclotetradecane, 1,4,8,11-tetraazabicyclo[6.6.2]hexadecane, 1,4,8-trimethyl-1,4,8,11-tetraazacyclotetradecane, 1,8-dimethyl-1,4,8,11-tetraazacyclotetradecane, 1,8-dibenzyl-1,4,8,11-tetraazacyclotetradecane, 1,4-dibenzyl-1,4,8,11-tetraazacyclotetradecane, 6,13-dimethyl-6,13-dinitro-1,4,8,11-tetraazacyclotetradecane; the aldehyde is selected from the group containing acetaldehyde, formaldehyde and paraformaldehyde, and the acid is preferably HC1 and/or CF₃COOH; and wherein the reaction may be preceded by the step of preparation of the intermediate product.

10. An intermediate product of the general formula (**IV**) for the preparation of cyclam based compounds, 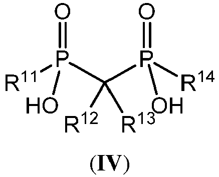 wherein R¹¹, R¹², R¹³ and R¹⁴ are in the following combinations:
| Intermediate | R¹¹ | R¹² | R¹³ | R¹⁴ |
|---|---|---|---|---|
| B | H | H | H | *p*-nitrobenzyl |
| C | H | H | H | -CH₂N(benzyl)₂ |
| D | H | H | H | -(CH₂)₂COOH |
| E | H | -(CH₂)₄COOH | H | H |
| F | OH | -(CH₂)₃NH(4-dibenzylcyclooktyn amidobutoxyl) | OH | OH |

11. A pharmaceutical preparation, **characterized in that** it contains at least one cyclam based compound according to any one of the claims 1 to 5 and/or at least one conjugate according to the claim 6 and/or at least one coordination compound according to the claim 7 and/or at least one targeting conjugate according to the claim 8, and a pharmaceutically acceptable substance.

12. The cyclam based compound according to any one of the claims 1 to 5 and/or at least one conjugate according to claim 6 and/or at least one coordination compound according to the claim 7 and/or at least one targeting conjugate according to the claim 8 and/or the pharmaceutical preparation according to the claim 11, for use as a medicament, preferably in the treatment of tumor diseases, inflammations, and/or atherosclerosis.

13. The cyclam based compound according to any one of the claims 1 to 5 and/or at least one conjugate according to claim 6 and/or at least one coordination compound according to the claim 7 and/or at least one targeting conjugate according to the claim 8 and/or the pharmaceutical preparation according to the claim 11, for use as a contrast agent in medical diagnostics, preferably in the diagnostics of tumor diseases, inflammations, and/or atherosclerosis.

14. Use of cyclam based compounds according to any one of the claims 1 to 5 and/or at least one conjugate according to claim 6 and/or at least one coordination compound according to the claim 7 and/or at least one targeting conjugate according to the claim 8 and/or the pharmaceutical preparation according to the claim 11, in radiochemistry as markers or precursors of markers when labelling with the use of radioisotopes of copper, lutetium, bismuth and yttrium.

15. Use of cyclam based compounds according to any one of the claims 1 to 5 and/or at least one conjugate according to claim 6 and/or at least one coordination compound according to the claim 7 and/or at least one targeting conjugate according to the claim 8, for the complexation and/or purification of copper radioisotopes.

## Patentansprüche

1. Verbindungen auf Cyclambasis der allgemeinen Formel (I) 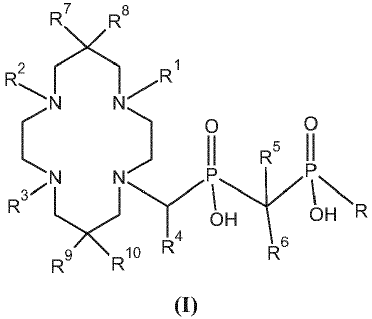 worin
R, R^{a}, R¹, R², R³ sind unabhängig ausgewählt aus der Gruppe umfassend H, OH, (C1-C6)Alkyl, das linear oder verzweigt sein kann, (C3-C6)Cycloalkyl, Benzyl,
und/oder R¹ und/oder R² und/oder R³ ist eine Bisphosphorsäure der allgemeinen Formel (II) 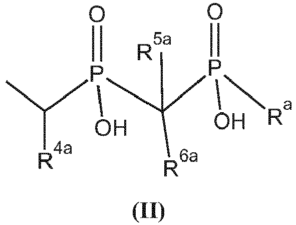
und/oder R¹ und R³ bilden zusammen (C2-C3)Alkylen, das mit einem oder mehreren linearen oder verzweigten (C1-C6)Alkylen substituiert sein kann;
und/oder R, R^{a} sind unabhängig ausgewählt aus Phenyl- und (C5-C6)Heterocyclus, der mindestens ein Heteroatom ausgewählt aus N, S, O enthält;
worin der (C1-C6)Alkyl, (C3-C6)Cycloalkyl, Phenyl, Benzyl und (C5-C6)Heterocyclus, der mindestens ein Heteroatom ausgewählt aus N, S, O enthält, unabhängig mit einer oder mehreren Gruppen substituiert sein kann ausgewählten aus COOH; NH₂; NO₂, NX₂; C(O)NX₂; NHX; C(O)NHX; OH; SH; -NCS; Tetrazin der allgemeinen Formel (III); 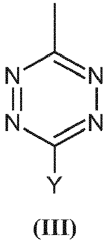 (C7-C8)Cycloalkenyl in *trans*-Konfiguration, das optional weiter mit einer oder mehreren Gruppen substituiert sein kann, ausgewählt aus COOH, NH₂, NO₂, -NCS, -NCO, N₃ und SH; Norbornenyl, optional substituiert mit einer oder mehreren Gruppen, ausgewählt aus COOH, NH₂, NO₂, -NCS, -NCO, N₃ und SH; -N₃; (C5-C6)Heterocyclus, der mindestens ein Heteroatom ausgewählt aus N, S, O enthält; (C2-C6)Alkinyl, optional substituiert mit einer oder mehreren Gruppen, ausgewählt aus COOH, NH₂, NO₂, -NCS, -NCO, N₃ und SH; Cyclooctinyl, optional substituiert mit einer oder mehreren Gruppen, ausgewählt aus COOH, NH₂, NO₂, -NCS, -NCO, N₃ und SH; Cycloazaoctinyl, optional substituiert mit einer oder mehreren Gruppen, ausgewählt aus COOH, NH₂, NO₂, -NCS, -NCO, N₃ und SH; Maleimid; Phenyl; Benzyl und -P(O)(OH)Z;
und worin R⁴, R^{4a}, R⁵ und R^{5a} unabhängig voneinander H, (C1 -C6)Alkyl sind, das linear oder verzweigt sein kann, (C3-C6)Cycloalkyl, Benzyl;
worin (C1-C6)Alkyl, (C3-C6)Cycloalkyl und Benzyl unabhängig voneinander mit einer oder mehreren Gruppen substituiert sein können, ausgewählten aus COOH; NH₂; NO₂, NX₂; C(O)NX₂; NHX; C(O)NHX; OH; SH; -NCS; Tetrazin der allgemeinen Formel (III); (C7-C8)Cycloalkenyl in *trans*-Konfiguration, das optional weiter mit einer oder mehreren Gruppen substituiert sein kann, ausgewählt aus COOH, NH₂, NO₂, -NCS, -NCO, N₃ und SH; Norbornenyl, optional substituiert mit einer oder mehreren Gruppen, ausgewählt aus COOH, NH₂, NO₂, -NCS, -NCO, N₃ und SH; -N₃; (C5-C6)Heterocyclus, der mindestens ein Heteroatom ausgewählt aus N, S, O enthält; (C2-C6)Alkinyl, optional substituiert mit einer oder mehreren Gruppen, ausgewählt aus COOH, NH₂, NO₂, -NCS, -NCO, N₃ und SH; Cyclooctinyl, optional substituiert mit einer oder mehreren Gruppen, ausgewählt aus COOH, NH₂, NO₂, -NCS, -NCO, N₃ und SH; Cycloazaoctinyl, optional substituiert mit einer oder mehreren Gruppen, ausgewählt aus COOH, NH₂, NO₂, -NCS, -NCO, N₃ und SH; Maleimid; Phenyl; Benzyl und -P(O)(OH)Z;
und worin R⁶ und R^{6a} sind H;
und worin R⁷, R⁸, R⁹ und R¹⁰ unabhängig ausgewählt sind aus der Gruppe umfassend H, (C1-C6)Alkyl, (C3-C6)Cycloalkyl, Benzyl, Phenyl, NO₂, COOH, (C5-C6)Heterocyclus, der mindestens ein Heteroatom ausgewählt aus N, S, O enthält, und/oder R⁷ und R⁸ bilden zusammen (C2-C3)Alkylen oder Vinylen und/oder R⁹ und R¹⁰ bilden zusammen (C2-C3)Alkylen oder Vinylen,
worin die (C1-C6)Alkyl, (C3-C6)Cycloalkyl, Phenyl, Benzyl, (C5-C6)Heterocyclus, der mindestens ein Heteroatom ausgewählt aus N, S, O enthält, (C2-C3)Alkylen und Vinylen unabhängig substituiert werden können mit einer Gruppe oder Gruppen ausgewählt aus COOH; NH₂; NO₂; NX₂; C(O)NX₂; SH; Tetrazin der allgemeinen Formel (III); (C7-C8)Cycloalkenyl in *trans*-Konfiguration, optional substituiert mit einer oder mehreren Gruppen, ausgewählt aus COOH, NH₂, NO₂, -NCS, -NCO, N₃ und SH; Norbornenyl, optional substituiert mit einer oder mehreren Gruppen, ausgewählt aus COOH, NH₂, NO₂, -NCS, -NCO, N₃ und SH; -N₃; (C5-C6)Heterocyclus, der mindestens ein Heteroatom ausgewählt aus N, S, O enthält; (C2-C6)Alkinyl, optional substituiert mit einer oder mehreren Gruppen, ausgewählt aus COOH, NH₂, NO₂, -NCS, -NCO, N₃ und SH; Cyclooctinyl, optional substituiert mit einer oder mehreren Gruppen, ausgewählt aus COOH, NH₂, NO₂, -NCS, -NCO, N₃ und SH; Cycloazaoctinyl, optional substituiert mit einer oder mehreren Gruppen, ausgewählt aus COOH, NH₂, NO₂, -NCS, -NCO, N₃ und SH; Maleimid; Phenyl; Benzyl und -P(O)(OH)Z,
worin Y ist H, (C1-C6)Alkyl, -CH₂COOH, (C5-C6)Heterocyclus, der mindestens ein Heteroatom ausgewählt aus N, S, O enthält, oder Heteroaryl, das mindestens ein Heteroatom ausgewählt aus N, S, O enthält; vorzugsweise Y ist ein Atom von Wasserstoff, Methyl oder Pyridyl; worin Z ist H; OH; (C1-C6)Alkyl, das linear oder verzweigt sein kann; (C1-C6)Hydroxyalkyl; (C1-C6)Alkoxyl; Phenyl; (C5-C6)Heterocyclus, der mindestens ein Heteroatom ausgewählt aus N, S, O enthält; oder Benzyl; wobei Phenyl, (C5-C6)Heterocyclus oder Benzyl optional mit einer oder mehreren Gruppen substituiert sein können, ausgewählt aus COOH, NH₂, NO₂, N₃ und SH, vorzugsweise befindet sich der Substituent in para-Position;
und worin X unabhängig H; (C1-C6)Alkyl, das linear oder verzweigt sein kann; (C3-C6)Cycloalkyl; Phenyl und/oder Benzyl ist;
worin für X, Y und Z gilt, dass (C1 -C6)Alkyl, (C3-C6)Cycloalkyl, Phenyl und/oder Benzyl unabhängig voneinander mit einer oder mehreren Gruppen substituiert sein können, ausgewählt aus COOH; NH₂; NO₂, NX₂; C(O)NX₂; NHX; C(O)NHX; SH; Tetrazin der allgemeinen Formel (III); (C7-C8)Cycloalkenyl in *trans*-Konfiguration, optional substituiert mit einer oder mehreren Gruppen, ausgewählt aus COOH, NH₂, NO₂, -NCS, - NCO, N₃ und SH; Norbornenyl, optional substituiert mit einer oder mehreren Gruppen, ausgewählt aus COOH, NH₂, NO₂, -NCS, -NCO, N₃ und SH; -N₃; (C2-C6)Alkinyl, optional substituiert mit einer oder mehreren Gruppen, ausgewählt aus COOH, NH₂, NO₂, -NCS, - NCO, N₃ und SH; Cyclooctinyl, optional substituiert mit einer oder mehreren Gruppen, ausgewählt aus COOH, NH₂, NO₂, -NCS, -NCO, N₃ und SH; Cycloazaoctinyl, optional substituiert mit einer oder mehreren Gruppen, ausgewählt aus COOH, NH₂, NO₂, -NCS, - NCO, N₃ und SH; Maleimid; Phenyl; Benzyl und -P(O)(OH)Z;
wobei mindestens eine der Gruppen R, R^{a}, R¹, R², R³, R⁴, R^{4a}, R⁵, R^{5a}, R⁶, R^{6a}, R⁷, R⁸, R⁹ und R¹⁰ mindestens eine der folgenden Gruppen enthält: -COOH; NH₂; NO₂, NX₂, NHX; C(O)NHX; N₃; SH; - NCS; Tetrazin der allgemeinen Formel (III); (C7-C8)Cycloalkenyl in *trans*-Konfiguration, optional substituiert mit einer oder mehreren Gruppen, ausgewählt aus COOH, NH₂, NO₂, NX₂, -NCS, -NCO, NHX, C(O)NHX, N₃ und SH; Norbornenyl, optional substituiert mit einer oder mehreren Gruppen, ausgewählt aus COOH, NH₂, NO₂, NX₂, -NCS, -NCO, NHX, C(O)NHX, N₃ und SH; -N₃; (C2-C6)Alkinyl; Cyclooctinyl, optional substituiert mit einer oder mehreren Gruppen, ausgewählt aus COOH, NH₂, NO₂, NX₂, -NCS, -NCO, NHX, C(O)NHX, N₃ und SH; Cycloazaoctinyl, optional substituiert mit einer oder mehreren Gruppen, ausgewählt aus COOH, NH₂, NO₂, NX₂, -NCS, -NCO, NHX, C(O)NHX, N₃ und SH; Maleimid; Phenyl, optional substituiert mit einer oder mehreren Gruppen, ausgewählt aus COOH, NH₂, NO₂, NX₂, -NCS, -NCO, NHX, C(O)NHX, N₃ und SH; Benzyl, optional substituiert mit einer oder mehreren Gruppen, ausgewählt aus COOH, NH₂, NO₂, NX₂, -NCS, -NCO, NHX, C(O)NHX, N₃ und SH; (C1-C6)Alkyl, optional substituiert mit einer oder mehreren Gruppen, ausgewählt aus COOH, NH₂, NO₂, NX₂, -NCS, -NCO, NHX, C(O)NHX, N₃ und SH; und -P(O)(OH)Z.

2. Verbindungen auf Cyclambasis nach Anspruch 1, **dadurch gekennzeichnet, dass** R¹ und R³ zusammen (C2-C3)Alkylen bilden, das mit (C1-C6)Alkyl substituiert sein kann, das linear oder verzweigt sein kann.

3. Verbindungen auf Cyclambasis nach Anspruch 1, **dadurch gekennzeichnet, dass** R, R^{a}, R¹, R², R³, R⁴, R^{4a}, R⁵, R^{5a}, R⁶ und R^{6a} unabhängig ausgewählt sind aus der Gruppe, umfassend H; (C1-C6)Alkyl, das linear oder verzweigt sein kann; (C3-C6)Cycloalkyl; Benzyl; Phenyl; (C5-C6)Heterocyclus, der mindestens ein Heteroatom ausgewählt aus N, S, O enthält;
und/oder R¹ und/oder R² und/oder R³ die Bisphosphorsäure der allgemeinen Formel (II) ist, vorzugsweise ist mindestens eine der Gruppen R¹, R² und R³ die Bisphosphorsäure der allgemeinen Formel (II), am meisten bevorzugt ist R² die Bisphosphorsäure der allgemeinen Formel (II).

4. Verbindungen auf Cyclambasis nach Anspruch 1, **dadurch gekennzeichnet, dass** R² ausgewählt ist aus der Gruppe umfassend H; (C1-C6)Alkyl, das linear oder verzweigt sein kann; (C3-C6)Cycloalkyl; Benzyl; Phenyl; (C5-C6)Heterocyclus, der mindestens ein Heteroatom ausgewählt aus N, S, O enthält; und Bisphosphorsäure der allgemeinen Formel (II).

5. Verbindungen auf Cyclambasis nach Anspruch 1, **dadurch gekennzeichnet, dass** sie aus der Gruppe ausgewählt sind, die Verbindungen der allgemeinen Formel (I) umfasst, deren Substituenten in den folgenden Kombinationen dargestellt sind:
| Verbindung Nr. | R | R¹ | | R³ | R² | R⁴ | R⁵ | R⁶ | R⁷ | R⁸ | R⁹ | R¹⁰ |
|---|---|---|---|---|---|---|---|---|---|---|---|---|
| 1 | H | H | | H | H | CH₃ | H | H | H | H | H | H |
| 2 | H | CH₃ | | CH₃ | CH₃ | CH₃ | H | H | H | H | H | H |
| 3 | H | CH₃ | | CH₃ | H | H | H | H | H | H | H | H |
| 4 | H | CH₃ | | CH₃ | CH₃ | H | H | H | H | H | H | H |
| 5 | OH | Benzyl | | Benzyl | H | H | H | H | H | H | H | H |
| 7 | OH | H | | Benzyl | Benzyl | H | H | H | H | H | H | H |
| 8 | *p*-Nitrobenzyl | H | | H | H | H | H | H | H | H | H | H |
| 9 | *p-*Aminobenzyl | H | | H | H | H | H | H | H | H | H | H |
| 10 | *p*-SCN-benzyl | H | | H | H | H | H | H | H | H | H | H |
| 11 | *p*-Azidobenzyl | H | | H | H | H | H | H | H | H | H | H |
| 12 | -CH₂N(Bz)₂ | H | | H | H | H | H | H | H | H | H | H |
| 13 | -CH₂NH₂ | H | | H | H | H | H | H | H | H | H | H |
| 14 | -(CH₂)₂COOH | H | | H | H | H | H | H | H | H | H | H |
| 15 | H | H | | H | H | H | -(CH₂)₄COOH | H | H | H | H | H |
| 16 | H | H | | H | H | H | H | H | CH₃ | NO₂ | NO₂ | CH₃ |
| 22 | H | CH₃ | | CH₃ | Säure der Formel (**II**), worin R^{a} = R, R^{4a} bis R^{6a} = H | H | H | H | H | H | H | H |
| 23 | -(CH₂)₂COOH | CH₃ | | CH₃ | Säure der Formel (**II**), worin R^{a} = R^{a}, R^{4a} bis R^{6a} = H | H | H | H | H | H | H | H |
| 24 | OH | Benzyl | | Benzyl | -CH₂P(O)(OH)₂ | H | H | H | H | H | H | H |
| 25 | OH | H | | H | -CH₂P(O)(OH)₂ | H | H | H | H | H | H | H |
| 26 | OH | -CH₂P(O) (OH)₂ | | Benzyl | Benzyl | H | H | H | H | H | H | H |
| 27 | OH | - CH₂P(O)(OH)₂ | | H | H | H | H | H | H | H | H | H |
| 30 | H | -(CH₂)₂- | | | H | CH₃ | H | H | H | H | H | H |
| 31 | p-Nitrobenzyl | -(CH₂)₂- | | | H | H | H | H | H | H | H | H |
| 32 | H | -(CH₂)₂- | | | Säure der Formel (**II**), worin R^{a} = R, R^{4a} bis R^{6a} = H | H | H | H | H | H | H | H |
| 33 | -CH₂OH | -(CH₂)₂- | | | Säure der Formel (**II**), worin R^{a} = R, R^{4a} bis R^{6a} = H | H | H | H | H | H | H | H |
| 34 | OH | -(CH₂)₂- | | | Säure der Formel (**II**), worin R^{a} = R, R^{4a} bis R^{6a} = H | H | H | H | H | H | H | H |
| 35 | -(CH₂)₂COOH | -(CH₂)₂- | | | Säure der Formel (**II**), worin R^{a} = R, R^{4a} bis R^{6a} = H | H | H | H | H | H | H | H |
| 36 | H | -(CH₂)₂- | | | Säure der Formel (**II**), worin R^{a} = OH, R^{4a} bis R^{6a} = H | H | H | H | H | H | H | H |
| 37 | *p*-Nitrobenzyl | -(CH₂)₂- | | | Säure der Formel (**II**), worin R^{a} = OH, R^{4a} bis R^{6a} = H | H | H | H | H | H | H | H |
| 38 | *p-*Aminobenzyl | -(CH₂)₂- | | | Säure der Formel (**II**), worin R^{a} = OH, R^{4a} bis R^{6a} = H | H | H | H | H | H | H | H |
| 39 | *p*-SCN -benzyl | -(CH₂)₂- | | | Säure der Formel (**II**), worin R^{a} = OH, R^{4a} bis R^{6a} = H | H | H | H | H | H | H | H |
| 40 | *p*-Azidobenzyl | -(CH₂)₂- | | | Säure der Formel (**II**), worin R^{a} = OH, R^{4a} bis R^{6a} = H | H | H | H | H | H | H | H |
| 41 | *p*-Nitrobenzyl | -(CH₂)₂- | | | -CH₂P(O)(OH)(CH₂OH) | H | H | H | H | H | H | H |
| 42 | *p*-Nitrobenzyl | -(CH₂)₂- | | | -CH₂P(O)(OH)₂ | H | H | H | H | H | H | H |
| 43 | *p-*Aminobenzyl | -(CH₂)₂- | | | -CH₂P(O)(OH)₂ | H | H | H | H | H | H | H |
| 50 | H | -(CH₂)₂- | | | H | H | -(CH₂)₄CH₃ | H | H | H | H | H |
| 51 | H | -(CH₂)₂- | | | 2-Picolyl-*N*-oxide | H | -(CH₂)₄CH₃ | H | H | H | H | H |
| 52 | H | -(CH₂)₂- | | | -CH₂COOH | H | H | H | H | H | H | H |
| 53 | OH | -(CH₂)₂- | | | -CH₂P(O)(OH)- (CH₂)₂COOH | H | H | H | H | H | H | H |
| 54 | H | -CH₃ | -CH₃ | | -CH₂P(O)(OH)Ph | H | H | H | H | H | H | H |
| 55 | *p*-Nitrobenzyl | -(CH₂)₂- | | | -CH₂P(O)(OH)- CH₂N(2-picolyl)₂ | H | H | H | H | H | H | H |
| 56 | -(CH₂)₂COOH | H | H | | H | -(CH₂)₃N₃ | H | H | H | H | H | H |
| 57 | *p*-Nitrobenzyl | -(CH₂)₂- | | | -(CH₂)₂S- (Dimer) | H | H | H | H | H | H | H |
| 58 | H | H | | H | H | H | H | H | -CH₃ | -NH₂ | -NH₂ | -CH₃ |
| 59 | *p*-Nitrobenzyl | H | | H | H | H | H | H | -COOH | H | H | H |
| 60 | *p*-Nitrobenzyl | H | | H | H | H | H | H | -(CH₂)₂OH | H | H | H |
| 61 | -(CH₂)₂COOH | H | | H | H | H | H | H | *2*-Pyridyl | H | *2*-Pyridyl | H |

6. Konjugat der Verbindung auf Cyclambasis nach einem der vorhergehenden Ansprüche und mindestens einer Konjugationsgruppe, die kovalent an die Verbindung auf Cyclambasis gebunden ist und ausgewählt ist aus der Gruppe, die enthält: OH; -COOH; NH₂; NO₂, NX₂, NHX; C(O)NHX; N₃; SH; -NCS; Tetrazin der allgemeinen Formel (III); (C7-C8)Cycloalkenyl in *trans*-Konfiguration, optional substituiert mit einer oder mehreren Gruppen, ausgewählt aus COOH, NH₂, NO₂, NX₂, -NCS, -NCO, NHX; C(O)NHX; N₃ und SH; Norbornenyl, optional substituiert mit einer oder mehreren Gruppen, ausgewählt aus COOH, NH₂, NO₂, NX₂, -NCS, -NCO, NHX; C(O)NHX; N₃ und SH; -N₃; (C2-C6)Alkinyl; Cyclooctinyl, optional substituiert mit einer oder mehreren Gruppen, ausgewählt aus COOH, NH₂, NO₂, NX₂, -NCS, -NCO, NHX; C(O)NHX; N₃ und SH; Cycloazaoctinyl, optional substituiert mit einer oder mehreren Gruppen, ausgewählt aus COOH, NH₂, NO₂, NX₂, -NCS, -NCO, NHX, C(O)NHX, (C1-C6)Alkyl, C6-Aryl, N₃ und SH; Maleimid; und -P(O)(OH)Z; wobei das Konjugat ferner einen Spacer zwischen der Verbindung auf Cyclambasis und der Konjugationsgruppe und/oder zwischen zwei Konjugationsgruppen enthalten kann, wobei der Spacer ausgewählt ist aus der Gruppe, die enthält: (C1-C6)n-Alkyl, optional substituiert mit C=O und/oder - NH- Gruppe; Phenylen; Aminosäureketten mit einer Länge von 1 bis 5 Aminosäuren; Polyethylenglykole mit 1 bis 10 Monomereinheiten.

7. Koordinationsverbindungen der Verbindungen auf Cyclambasis der allgemeinen Formel (I) nach einem der Ansprüche 1 bis 5 oder der Konjugate nach Anspruch 6, mit Metallkationen ausgewählten aus der Gruppe bestehend aus Cu²⁺, Bi³⁺, Lanthanid(III) Kationen, Sc³⁺, y³⁺, Pb²⁺, Zr⁴⁺, Ac³⁺, Mn²⁺ und Mn³⁺, vorzugsweise Lanthanoid (III) Kationen, Y³⁺ und Cu²⁺, besonders vorzugsweise aus Lu³⁺, Y³⁺ und Cu²⁺.

8. Zielkonjugat, das die Verbindung auf Cyclambasis der allgemeinen Formel (I) nach einem der Ansprüche 1 bis 5 und/oder das Konjugat nach Anspruch 6 und/oder die Koordinationsverbindung nach Anspruch 7 enthält; und einen Zielvektor, ausgewählt aus der Gruppe umfassend Bis(phosphonate); fluoreszenzfähige Gruppen, vorzugsweise Fluoresceine, Rhodamine oder Substanzen vom Bor-Dipyrromethen-Typ; Oligopeptide mit 1 bis 15 Aminosäuren; Antikörper oder Fragmente davon, vorzugsweise die Fab-Fragmente; Folsäure; Biotin; Verbindungen, die auf den PSMA-Rezeptor abzielen, vorzugsweise Harnstoffderivate oder Organophosphorverbindungen; Cyclodextrine; Dendrimere; hydrophile Polymere auf der Basis von Derivaten der Acrylsäure, insbesondere Hydroxoalkylamiden der Acrylsäure.

9. Verfahren zur Herstellung von Verbindungen auf Cyclambasis der allgemeinen Formel (I) nach einem der Ansprüche 1 bis 5, wobei ein Zwischenprodukt der allgemeinen Formel (IV), 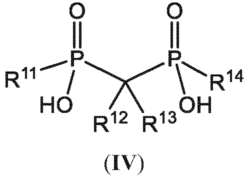 worin R¹¹, R¹², R¹³ und R¹⁴ in den folgenden Kombinationen vorliegen:
| Zwischenprodukt | R¹¹ | R¹² | R¹³ | R¹⁴ |
|---|---|---|---|---|
| A | H | H | H | OH |
| B | H | H | H | *p*-Nitrobenzyl |
| C | H | H | H | -CH₂N(Benzyl)₂ |
| D | H | H | H | -(CH₂)₂COOH |
| E | H | -(CH₂)₄COOH | H | H |
| F | OH | -(CH₂)₃NH(4-Dibenzylcyclooktin amidobutoxyl) | OH | OH |
oder eine Verbindung ausgewählt aus der Gruppe, die Methylen-bis(phosphinsäure), Phosphorsäure, Hydroxymethylphosphinsäure enthält, reagiert mit einem Cyclamderivat und einem Aldehyd in Wasserlösung einer Säure einer Konzentration im Bereich von 10 bis 40 Gew.%, vorzugsweise 18 bis 36 Gew.%, bei einer Temperatur im Bereich von 40 °C bis 80 °C für den Zeitraum von mindestens 12 Stunden;
wobei das Cyclamderivat 1,4,8,11-Tetraazacyclotetradecan und Derivate davon und 1,4,8,11-Tetraazabicyclo[6.6.2]hexadecan und Derivate davon ist, vorzugsweise ist das Cyclamderivat ausgewählt aus der Gruppe, die enthält 1,4,8,11-Tetraazacyclotetradecan, 1,4,8,11-Tetraazabicyclo[6.6.2]hexadecan, 1,4,8-Trimethyl-1,4,8,11-tetraazacyclotetradecan, 1,8-Dimethyl-1,4,8,11-Tetraazacyclotetradecan, 1,8-Dibenzyl-1,4,8,11-tetraazacyclotetradecan, 1,4-Dibenzyl-1,4,8,11-tetraazacyclotetradecan, 6,13-Dimethyl-6,13-dinitro-1,4,8,11-Tetraazacyclotetradecan,
der Aldehyd ist ausgewählt aus der Gruppe, die Acetaldehyd, Formaldehyd und para-Formaldehyd enthält;
und die Säure ist vorzugsweise HCl und/oder CF₃COOH;
und wobei der Reaktion der Schritt der Herstellung des Zwischenprodukts vorausgehen kann.

10. Ein Zwischenprodukt der allgemeinen Formel (IV) zur Herstellung von Verbindungen auf Cyclambasis, 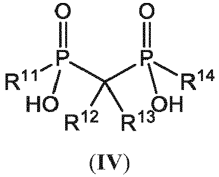 worin R¹¹, R¹², R¹³ und R¹⁴ in den folgenden Kombinationen vorliegen:
| Zwischenprodukt | R¹¹ | R¹² | R¹³ | R¹⁴ |
|---|---|---|---|---|
| B | H | H | H | *p*-Nitrobenzyl |
| C | H | H | H | -CH₂N(Benzyl)₂ |
| D | H | H | H | -(CH₂)₂COOH |
| E | H | -(CH₂)₄COOH | H | H |
| F | OH | -(CH₂)₃NH(4-Dibenzylcyclooktin amidobutoxyl) | OH | OH |

11. Pharmazeutisches Präparat, **dadurch gekennzeichnet, dass** es mindestens eine Verbindung auf Cyclambasis nach einem der Ansprüche 1 bis 5 und/oder mindestens ein Konjugat nach Anspruch 6 und/oder mindestens eine Koordinationsverbindung nach dem Anspruch 7 und/oder mindestens ein Zielkonjugat nach dem Anspruch 8 und eine pharmazeutisch verträgliche Substanz enthält.

12. Verbindung auf Cyclambasis nach einem der Ansprüche 1 bis 5 und/oder mindestens ein Konjugat nach Anspruch 6 und/oder mindestens eine Koordinationsverbindung nach dem Anspruch 7 und/oder Zielkonjugat nach dem Anspruch 8 und/oder das pharmazeutische Präparat nach Anspruch 11 zur Verwendung als Medikament, vorzugsweise bei der Behandlung von Tumorerkrankungen, Entzündungen und/oder Atherosklerose.

13. Verbindung auf Cyclambasis nach einem der Ansprüche 1 bis 5 und/oder mindestens ein Konjugat nach Anspruch 6 und/oder mindestens eine Koordinationsverbindung nach dem Anspruch 7 und/oder Zielkonjugat nach dem Anspruch 8 und/oder das pharmazeutische Präparat nach Anspruch 11 zur Verwendung als Kontrastmittel in der medizinischen Diagnostik, vorzugsweise bei der Diagnose von Tumorerkrankungen, Entzündungen und/oder Atherosklerose.

14. Verwendung von Verbindungen auf Cyclambasis nach einem der Ansprüche 1 bis 5 und/oder mindestens einem Konjugat nach dem Anspruch 6 und/oder mindestens einer Koordinationsverbindung nach dem Anspruch 7 und/oder mindestens einem Zielkonjugat nach dem Anspruch 8 und/oder einem pharmazeutischen Präparat nach Anspruch 11 in der Radiochemie als Marker oder Vorläufer von Markern bei der Markierung unter Verwendung von Radioisotopen von Kupfer, Lutetium, Wismut und Yttrium.

15. Verwendung von Verbindungen auf Cyclambasis nach einem der Ansprüche 1 bis 5 und/oder mindestens einem Konjugat nach dem Anspruch 6 und/oder mindestens einer Koordinationsverbindung nach dem Anspruch 7 und/oder mindestens einem Zielkonjugat nach dem Anspruch 8 zur Komplexierung und/oder Reinigung von Kupferradioisotopen.

## Revendications

1. Composés à base de cyclame de formule générale **(I)** 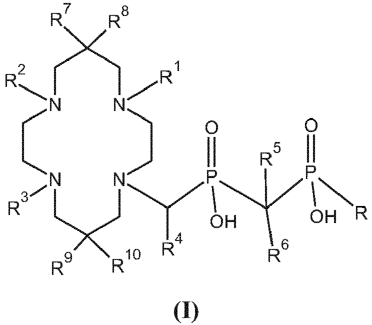 où
R, R^{a}, R¹, R², R³ sont indépendamment sélectionnés dans le groupe comprenant H, OH, (C1 à C6)alkyle, qui peut être linéaire ou ramifié, (C3 à C6)cycloalkyle, benzyle, et/ou R¹ et/ou R² et/ou R³ est un acide bisphosphore de formule générale **(II)** 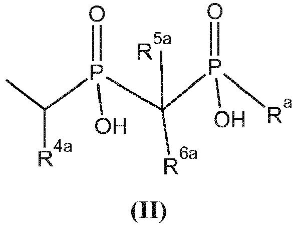
et/ou R¹ et R³ ensemble forment un (C2 à C3)alkylène, qui peut être substitué par un ou plusieurs (C1 à C6)alkyles linéaires ou ramifiés,
et/ou R, R^{a} sont choisis indépendamment parmi phényle et (C5 à C6)hétérocycle, contenant au moins un hétéroatome de N, S, O,
où (C1 à C6)alkyle, (C3 à C6)cycloalkyle, phényle, benzyle et (C5 à C6)hétérocycle, contenant au moins un hétéroatome de N, S, O, peuvent être indépendamment substitués par un ou plusieurs groupes choisis parmi COOH; NH₂; NO₂, NX₂; C(O)NX₂; NHX; C(O)NHX; OH; SH; -NCS; tétrazine de formule générale **(III);** 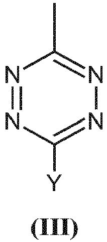 (C7 à C8)cycloalcényle en configuration *trans,* qui peut éventuellement être en outre substitué par un ou plusieurs groupes choisis parmi COOH, NH₂, NO₂, -NCS, -NCO, N₃ et SH; norbornényle, éventuellement substitué par un ou plusieurs groupes choisis parmi COOH, NH₂, NO₂, -NCS, -NCO, N₃ et SH; -N₃; (C5 à C6)hétérocycle, contenant au moins un hétéroatome de N, S, O; (C2 à C6)alcynyle, éventuellement substitué par un ou plusieurs groupes choisis parmi COOH, NH₂, NO₂, -NCS, -NCO, N₃ et SH; cyclooctynyle, éventuellement substitué par un ou plusieurs groupes choisis parmi COOH, NH₂, NO₂, -NCS, -NCO, N₃ et SH; cycloazaoctynyle, éventuellement substitué par un ou plusieurs groupes choisis parmi COOH, NH₂, NO₂, -NCS, -NCO, N₃ et SH; maléimide; phényle; benzyle et -P(O)(OH)Z;
et où R⁴, R^{4a}, R⁵ et R^{5a} sont indépendamment H, (C1 à C6)alkyle, qui peut être linéaire ou ramifié, (C3 à C6)cycloalkyle, benzyle;
où (C1 à C6)alkyle, (C3 à C6)cycloalkyle et benzyle peuvent être indépendamment substitués par un ou plusieurs groupes choisis parmi COOH; NH₂; NO₂, NX₂; C(O)NX₂; NHX; C(O)NHX; OH; SH; -NCS; tétrazine de formule générale **(III);** (C7 à C8)cycloalcényle en configuration *trans,* qui peut éventuellement être en outre substitué par un ou plusieurs groupes choisis parmi COOH, NH₂, NO₂, -NCS, -NCO, N₃ et SH; norbornényle, éventuellement substitué par un ou plusieurs groupes choisis parmi COOH, NH₂, NO₂, -NCS, -NCO, N₃ et SH; -N₃; (C5 à C6)hétérocycle, contenant au moins un hétéroatome de N, S, O; (C2 à C6)alcynyle, éventuellement substitué par un ou plusieurs groupes choisis parmi COOH, NH₂, NO₂, -NCS, -NCO, N₃ et SH; cyclooctynyle, éventuellement substitué par un ou plusieurs groupes choisis parmi COOH, NH₂, NO₂, -NCS, -NCO, N₃ et SH; cycloazaoctynyle, éventuellement substitué par un ou plusieurs groupes choisis parmi COOH, NH₂, NO₂, -NCS, -NCO, N₃ et SH; maléimide; phényle; benzyle et -P(O)(OH)Z;
et où R⁶ et R^{6a} est H;
et dans laquelle R⁷, R⁸, R⁹ et R¹⁰ sont indépendamment sélectionnés dans le groupe comprenant H, (C1 à C6)alkyle, (C3 à C6)cycloalkyle, benzyle, phényle, NO₂, COOH, (C5 à C6)hétérocycle, contenant au moins un hétéroatome de N, S, O et/ou R⁷ et R⁸ ensemble contient (C2 à C3)alkylène ou vinylène et/ou R⁹ et R¹⁰ ensemble contiennent (C2 à C3)alkylène ou vinylène,
où (C1 à C6)alkyle, (C3 à C6)cycloalkyle, phényle, benzyle, (C5 à C6)hétérocycle, contenant au moins un hétéroatome de N, S, O, (C2 à C3)alkylène et vinylène peuvent être substitués indépendamment avec un groupe ou des groupes choisis parmi COOH; NH₂; NO₂; NX₂; C(O)NX₂; SH; tétrazine de formule générale **(III);** (C7 à C8)cycloalcényle en configuration trans, éventuellement substitué par un ou plusieurs groupes choisis parmi COOH, NH₂, NO₂, -NCS, -NCO, N₃ et SH; norbornényle, éventuellement substitué par un ou plusieurs groupes choisis parmi COOH, NH₂, NO₂, -NCS, -NCO, N₃ et SH; -N₃; (C5 à C6)hétérocycle, contenant au moins un hétéroatome de N, S, O; (C2 à C6)alcynyle, éventuellement substitué par un ou plusieurs groupes choisis parmi COOH, NH₂, NO₂, -NCS, -NCO, N₃ et SH; cyclooctynyle, éventuellement substitué par un ou plusieurs groupes choisis parmi COOH, NH₂, NO₂, -NCS, -NCO, N₃ et SH; cycloazaoctynyle, éventuellement substitué par un ou plusieurs groupes choisis parmi COOH, NH₂, NO₂, -NCS, -NCO, N₃ et SH; maléimide; phényle; benzyle et -P(O)(OH)Z,
où Y est H, (C1 à C6)alkyle, -CH₂COOH, (C5 à C6)hétérocycle, contenant au moins un hétéroatome de N, S, O ou hétéroaryle, contenant au moins un hétéroatome de N, S, O, de préférence Y est un atome d'hydrogène, de méthyle ou de pyridyle;
où Z est H; OH; (C1 à C6)alkyle, qui peut être linéaire ou ramifié; (C1 à C6)hydroxyalkyle; (C1 à C6)alcoxyle; phényle; (C5 à C6)hétérocycle, contenant au moins un hétéroatome de N, S, O; ou benzyle, où phényle, (C5 à C6)hétérocycle ou benzyle peuvent éventuellement être substitués par un ou plusieurs groupes choisis parmi COOH, NH₂, NO₂, N₃ et SH, de préférence le substituant est en position para;
et où X est indépendamment H; (C1 à C6)alkyle, qui peut être linéaire ou ramifié; (C3 à C6)cycloalkyle; phényle et/ou benzyle;
dans lequel pour X, Y et Z s'applique, que cet (C1 à C6)alkyle, (C3 à C6)cycloalkyle, phényle et/ou benzyle peut être indépendamment substitué par un ou plusieurs groupes choisis parmi COOH; NH₂; NO₂, NX₂; C(O)NX₂; NHX; C(O)NHX; SH; tétrazine de formule générale **(III);** (C7 à C8)cycloalcényle en configuration *trans,*éventuellement substitué par un ou plusieurs groupes choisis parmi COOH, NH₂, NO₂, -NCS, -NCO, N₃ et SH; norbornényle, éventuellement substitué par un ou plusieurs groupes choisis parmi COOH, NH₂, NO₂, -NCS, - NCO, N₃ et SH; -N₃; (C2 à C6)alcynyle, éventuellement substitué par un ou plusieurs groupes choisis parmi COOH, NH₂, NO₂,-NCS, -NCO, N₃ et SH; cyclooctynyle, éventuellement substitué par un ou plusieurs groupes choisis parmi COOH, NH₂, NO₂, -NCS, -NCO, N₃ et SH; cycloazaoctynyle, éventuellement substitué par un ou plusieurs groupes choisis parmi COOH, NH₂, NO₂, -NCS, - NCO, N₃ et SH; maléimide; phényle; benzyle et -P(O)(OH)Z;
où au moins l'un des groupes R, R^{a}, R¹, R², R³, R⁴, R^{4a}, R⁵, R^{5a}, R⁶, R^{6a}, R⁷, R⁸, R⁹ et R¹⁰ contient au moins l'un des groupes -COOH; NH₂; NO₂, NX₂, NHX; C(O)NHX; N₃; SH; -NCS; tétrazine de formule générale **(III);** (C7 à C8)cycloalcényle en configuration trans, éventuellement substitué par un ou plusieurs groupes choisis parmi COOH, NH₂, NO₂, NX₂, -NCS, -NCO, NHX, C(O)NHX, N₃ et SH; norbornényle, éventuellement substitué par un ou plusieurs groupes choisis parmi COOH, NH₂, NO₂, NX₂, -NCS, -NCO, NHX, C(O)NHX, N₃ et SH; -N₃; (C2 à C6)alcynyle; cyclooctynyle, éventuellement substitué par un ou plusieurs groupes choisis parmi COOH, NH₂, NO₂, NX₂, -NCS, -NCO, NHX, C(O)NHX, N₃ et SH; cycloazaoctynyle, éventuellement substitué par un ou plusieurs groupes choisis parmi COOH, NH₂, NO₂, NX₂,-NCS, -NCO, NHX, C(O)NHX, N₃ et SH; maléimide; phényle, éventuellement substitué par un ou plusieurs groupes choisis parmi COOH, NH₂, NO₂, NX₂, -NCS, -NCO, NHX, C(O)NHX, N₃ et SH; benzyle, éventuellement substitué par un ou plusieurs groupes choisis parmi COOH, NH₂, NO₂, NX₂, -NCS, -NCO, NHX, C(O)NHX, N₃ et SH; (C1 à C6)alkyle, éventuellement substitué par un ou plusieurs groupes choisis parmi COOH, NH₂, NO₂, NX₂, -NCS, -NCO, NHX, C(O)NHX, N₃ et SH; et -P(O)(OH)Z.

2. Les composés à base de cyclame selon la revendication 1, **caractérisés en ce que** R¹ et R³ ensemble forment un (C2 à C3)alkylène, qui peut être substitué par (C1 à C6)alkyle, qui peut être linéaire ou ramifié.

3. Les composés à base de cyclame selon la revendication 1,**caractérisés en ce que** R, R^{a},R¹, R², R³, R⁴, R^{4a}, R⁵, R^{5a}, R⁶ et R^{6a} sont choisis indépendamment dans le groupe comprenant H; (C1 à C6)alkyle, qui peut être linéaire ou ramifié; (C3 à C6)cycloalkyle; benzyle; phényle; (C5 à C6)hétérocycle, contenant au moins un hétéroatome de N, S, O; et/ou R1 et/ou R2 et/ou R3 est l'acide bis-phosphore de formule générale **(II)**, de préférence au moins l'un des groupes R¹, R² et R³ est l'acide bis-phosphore de formule générale **(II)**, le plus préférablement R² est l'acide bisphosphore de formule générale **(II)**.

4. Les composés à base de cyclame selon la revendication 1, **caractérisés en ce que** R² est choisi dans le groupe comprenant H; (C1 à C6)alkyle, qui peut être linéaire ou ramifié; (C3 à C6)cycloalkyle; benzyle; phényle; (C5 à C6)hétérocycle,contenant au moins un hétéroatome de N, S, O; et l'acide bisphosphore de formule générale **(II).**

5. Les composés à base de cyclame selon la revendication 1,**caractérisés en ce qu'**ils sont choisis dans le groupe comprenant les composés de formule générale **(I),** dont les substituants sont représentés dans les combinaisons suivantes:
| Nombre de composé | R | R¹ | | R³ | R² | R⁴ | R⁵ | R⁶ | R⁷ | R⁸ | R⁹ | R¹⁰ |
|---|---|---|---|---|---|---|---|---|---|---|---|---|
| 1 | H | H | | H | H | CH₃ | H | H | H | H | H | H |
| 2 | H | CH₃ | | CH₃ | CH₃ | CH₃ | H | H | H | H | H | H |
| 3 | H | CH₃ | | CH₃ | H | H | H | H | H | H | H | H |
| 4 | H | CH₃ | | CH₃ | CH₃ | H | H | H | H | H | H | H |
| 5 | OH | benzyle | | benzy le | H | H | H | H | H | H | H | H |
| 7 | OH | H | | benzy le | benzyle | H | H | H | H | H | H | H |
| 8 | *p-*nitrobenzyle | H | | H | H | H | H | H | H | H | H | H |
| 9 | *p-*aminobenzyle | H | | H | H | H | H | H | H | H | H | H |
| 10 | *p*-SCN-benzyle | H | | H | H | H | H | H | H | H | H | H |
| 11 | *p-*azidobenzyle | H | | H | H | H | H | H | H | H | H | H |
| 12 | -CH₂N(B_{Z})₂ | H | | H | H | H | H | H | H | H | H | H |
| 13 | -CH₂NH₂ | H | | H | H | H | H | H | H | H | H | H |
| 14 | (CH₂)₂COOH | H | | H | H | H | H | H | H | H | H | H |
| 15 | H | H | | H | H | H | - (CH₂)₄COOH | H | H | H | H | H |
| 16 | H | H | | H | H | H | H | H | CH₃ | NO₂ | NO₂ | CH₃ |
| 22 | H | CH₃ | | CH₃ | acide de formula **(II)**, où R^{a} = R, R^{4a} to R^{6a} = H | H | H | H | H | H | H | H |
| 23 | - (CH₂)₂COOH | CH₃ | | CH₃ | acide de formula **(II)**,où R^{a} = R, R^{4a} to R^{6a} = H | H | H | H | H | H | H | H |
| 24 | OH | benzyle | | benzy le | -CH₂P(O)(OH)₂ | H | H | H | H | H | H | H |
| 25 | OH | H | | H | -CH₂P(O)(OH)₂ | H | H | H | H | H | H | H |
| 26 | OH | - CH₂P(O)(OH)₂ | | benzy le | benzyle | H | H | H | H | H | H | H |
| 27 | OH | - CH₂P(O)(OH)₂ | | H | H | H | H | H | H | H | H | H |
| 30 | H | -(CH₂)₂- | | | H | CH₃ | H | H | H | H | H | H |
| 31 | *p-*nitrobenzyle | -(CH₂)₂- | | | H | H | H | H | H | H | H | H |
| 32 | H | -(CH₂)₂- | | | acide de formula **(II)**, où R^{a} = R, R^{4a} to R^{6a} = H | H | H | H | H | H | H | H |
| 33 | -CH₂OH | -(CH₂)₂- | | | acide de formula **(II)**,où R^{a} = R, R^{4a} to R^{6a} = H | H | H | H | H | H | H | H |
| 34 | OH | -(CH₂)₂- | | | acide de formula **(II)**,où R^{a} = R, R^{4a} to R^{6a} = H | H | H | H | H | H | H | H |
| 35 | - (CH₂)₂COOH | -(CH₂)₂- | | | acide de formula **(II)**,où R^{a} = R, R^{4a} to R^{6a} = H | H | H | H | H | H | H | H |
| 36 | H | -(CH₂)₂- | | | acide de formula **(II)**,où R^{a} = OH, R^{4a} to R^{6a} = H | H | H | H | H | H | H | H |
| 37 | *p-*ni tro benzyle | -(CH₂)₂- | | | acide de formula **(II)**, où R^{a} = OH, R^{4a} to R^{6a} = H | H | H | H | H | H | H | H |
| 38 | *p-*aminobenzyl e | -(CH₂)₂- | | | acide de formula **(II)**, où R^{a} = OH, R^{4a} to R^{6a} = H | H | H | H | H | H | H | H |
| 39 | *p*-SCN-benzyle | -(CH₂)₂- | | | acide de formula **(II)**, où R^{a} = OH, R^{4a} to R^{6a} = H | H | H | H | H | H | H | H |
| 40 | *p-*azidobenzyle | -(CH₂)₂- | | | acide de formula **(II)**, où R^{a} = OH, R^{4a} to R^{6a} = H | H | H | H | H | H | H | H |
| 41 | *p-*nitrobenzyle | -(CH₂)₂- | | | - CH₂P(O)(OH)(CH₂OH) | H | H | H | H | H | H | H |
| 42 | *p-*nitrobenzyle | -(CH₂)₂- | | | -CH₂P(O)(OH)₂ | H | H | H | H | H | H | H |
| 43 | *p-*aminobenzyl e | -(CH₂)₂- | | | -CH₂P(O)(OH)₂ | H | H | H | H | H | H | H |
| 50 | H | -(CH₂)₂- | | | H | H | -(CH₂)₄CH₃ | H | H | H | H | H |
| 51 | H | -(CH₂)₂- | | | 2-picolyl-*N*-oxide | H | -(CH₂)₄CH₃ | H | H | H | H | H |
| 52 | H | -(CH₂)₂- | | | -CH₂COOH | H | H | H | H | H | H | H |
| 53 | OH | -(CH₂)₂- | | | -CH₂P(O)(OH)-(CH₂)₂COOH | H | H | H | H | H | H | H |
| 54 | H | -CH₃ | -CH₃ | | -CH₂P(O)(OH)Ph | H | H | H | H | H | H | H |
| 55 | *p-*nitrobenzyle | -(CH₂)₂- | | | -CH₂P(O)(OH)-CH₂N(2-picolyl)₂ | H | H | H | H | H | H | H |
| 56 | - (CH₂)₂COOH | H | H | | H | -(CH₂)₃N₃ | H | H | H | H | H | H |
| 57 | *p-*nitrobenzyle | -(CH₂)₂- | | | -(CH₂)₂S- (dimer) | H | H | H | H | H | H | H |
| 58 | H | H | | H | H | H | H | H | -CH₃ | - NH₂ | -NH₂ | -CH₃ |
| 59 | *p-*nitrobenzyle | H | | H | H | H | H | H | -COOH | H | H | H |
| 60 | *p-*nitrobenzyle | H | | H | H | H | H | H | (CH₂)₂OH | H | H | H |
| 61 | - (CH₂)₂COOH | H | | H | H | H | H | H | *2*-pyridyle | H | *2-*pyridyle | H |

6. Un conjugué du composé à base de cyclame selon l'une quelconque des revendications précédentes et d'au moins un groupe de conjugaison, qui est lié de manière covalente au composé à base de cyclame, et qui est choisi dans le groupe contenant OH, -COOH; NH₂; NO₂, NX₂, NHX; C(O)NHX; N₃; SH; -NCS; tétrazine de formule générale **(III);** (C7 à C8)cycloalcényle en configuration *trans,* éventuellement substitué par un ou plusieurs groupes choisis parmi COOH, NH₂, NO₂, NX₂, -NCS, -NCO, NHX; C(O)NHX; N₃ et SH; norbornényle, éventuellement substitué par un ou plusieurs groupes choisis parmi COOH, NH₂, NO₂, NX₂,-NCS, -NCO, NHX; C(O)NHX; N₃ et SH; -N₃; (C2 à C6)alcynyle; cyclooctynyle, éventuellement substitué par un ou plusieurs groupes choisis parmi COOH, NH₂, NO₂, NX₂,-NCS, -NCO, NHX; C(O)NHX; N₃ et SH; cycloazaoctynyle, éventuellement substitué par un ou plusieurs groupes choisis parmi COOH, NH₂, NO₂, NX₂, -NCS, -NCO, NHX, C(O)NHX, (C1 à C6)alkyle, (C6)aryle, N₃ et SH; maléimide; et -P(O)(OH)Z; où le conjugué peut en outre contenir un espaceur entre le composé à base de cyclame et le groupe de conjugaison et/ou entre deux groupes de conjugaison, l'espaceur choisi dans le groupe contenant (C1 à C6)*n*-alkyle, éventuellement substitué par C=O et/ou groupe -NH-; phénylène; chaînes d'acides aminés d'une longueur de 1 à 5 acides aminés; polyéthylène glycols de 1 à 10 unités monomères.

7. Composés de coordination des composés à base de cyclame de formule générale **(I)** selon l'une quelconque des revendications 1 à 5 ou des conjugués selon la revendication 6, avec des cations métalliques choisis dans le groupe constitué par Cu²⁺, Bi³⁺, lanthanide (III) cations, Sc³⁺, Y³⁺, Pb²⁺, Zr⁴⁺, Ac³⁺, Mn²⁺ et Mn³⁺, de préférence les cations lanthanide (III), Y³⁺ a Cu²⁺, plus préférablement Lu³⁺, Y³⁺ et Cu²⁺.

8. Un conjugué de ciblage, qui contient le composé à base de cyclame de formule générale **(I)** selon l'une quelconque des revendications 1 à 5 et/ou le conjugué selon la revendication 6 et/ou le composé de coordination selon la revendication 7; et un vecteur de ciblage, choisi dans le groupe comprenant les bis(phosphonates); les groupes capables de fluorescence, de préférence les fluorescéines, les rhodamines ou les substances de type bore-dipyrrométhène; des oligopeptides de 1 à 15 acides aminés; les anticorps ou leurs fragments, de préférence les fragments Fab; acide folique; la biotine; les composés ciblant le récepteur PSMA, de préférence les dérivés de l'urée ou les composés organophosphorés; cyclodextrines; dendrimères; les polymères hydrophiles à base de dérivés de l'acide acrylique, notamment les hydroxoalkylamides de l'acide acrylique.

9. Un procédé de préparation de composés à base de cyclame de formule générale **(I)** selon l'une quelconque des revendications 1 à 5, dans lequel un produit intermédiaire de formule générale **(IV),** 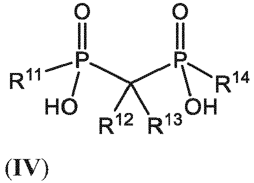 où R¹¹, R¹², R¹³ et R¹⁴ sont dans les combinaisons suivantes:
| Intermédiaire | R¹¹ | R¹² | R¹³ | R¹⁴ |
|---|---|---|---|---|
| A | H | H | H | OH |
| B | H | H | H | *p*-nitrobenzyle |
| C | H | H | H | -CH₂N(benzyle)₂ |
| D | H | H | H | -(CH₂)₂COOH |
| E | H | -(CH₂)₄COOH | H | H |
| F | OH | -(CH₂)₃NH(4-dibenzylcyclookt ynamidobutoxyle) | OH | OH |
ou un composé choisi dans le groupe contenant le méthylène-bis(acide phosphinique), l'acide phosphoreux, l'acide hydroxyméthylphosphinique, réagit avec un dérivé de cyclame et un aldéhyde dans une solution aqueuse d'un acide d'une concentration dans la rangée de 10 à 40 % (poids), de préférence de 18 à 36 % (poids), à une température dans la rangée de 40 °C à 80 °C pendant une période d'au moins 12 heures;
où le dérivé de cyclame est le 1,4,8,11-tétraazacyclotétradécane et ses dérivés et le 1,4,8,11-tétraazabicyclo[6.6.2]hexadécane et ses dérivés, de préférence le dérivé de cyclame est choisi dans le groupe contenant 1,4,8,11-tétraazacyclotétradécane, 1,4,8,11-tétraazabicyclo[6.6.2] hexadécane, 1,4,8-triméthyl-1,4,8,11-tétraazacyclotétradécane, 1,8-diméthyl-1,4,8,11-tétraazacyclotétradécane, 1,8-dibenzyl-1,4,8,11-tétraazacyclotétradécane, 1,4-dibenzyl-1,4,8,11-tétraazacyclotétradécane, 6,13-diméthyl-6,13-dinitro-1,4,8,11-tétraazacyclotétradécane;
l'aldéhyde est choisi dans le groupe contenant l'acétaldéhyde, le formaldéhyde et le para-formaldéhyde,
et l'acide est de préférence HCl et/ou CF₃COOH;
et dans lequel la réaction peut être précédée de l'étape de préparation du produit intermédiaire.

10. Un produit intermédiaire de formule générale **(IV)** pour la préparation de composés à base de cyclame, 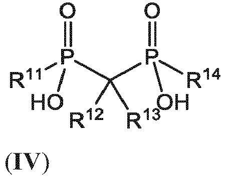 où R¹¹, R¹², R¹³ et R¹⁴ sont dans les combinaisons suivantes:
| Intermédiaire | R¹¹ | R¹² | R¹³ | R¹⁴ |
|---|---|---|---|---|
| B | H | H | H | *p*-nitrobenzyle |
| C | H | H | H | -CH₂N(benzyle)₂ |
| D | H | H | H | -(CH₂)₂COOH |
| E | H | -(CH₂)₄COOH | H | H |
| F | OH | -(CH₂)₃NH(4-dibenzylcyclooktynamidobutoxyle) | OH | OH |

11. Une préparation pharmaceutique, **caractérisée en ce qu'**elle contient au moins un composé à base de cyclame selon l'une quelconque des revendications 1 à 5 et/ou au moins un conjugué selon la revendication 6 et/ou au moins un composé de coordination selon le la revendication 7 et/ou au moins un conjugué de ciblage selon la revendication 8, et une substance pharmaceutiquement acceptable.

12. Le composé à base de cyclame selon l'une quelconque des revendications 1 à 5 et/ou au moins un conjugué selon la revendication 6 et/ou au moins un composé de coordination selon la revendication 7 et/ou au moins un conjugué de ciblage selon la la revendication 8 et/ou la préparation pharmaceutique selon la revendication 11, pour utilisation en tant que médicament, de préférence dans le traitement des maladies tumorales, des inflammations et/ou de l'athérosclérose.

13. Le composé à base de cyclame selon l'une quelconque des revendications 1 à 5 et/ou au moins un conjugué selon la revendication 6 et/ou au moins un composé de coordination selon la revendication 7 et/ou au moins un conjugué de ciblage selon la revendication 8 et/ou la préparation pharmaceutique selon la revendication 11, pour utilisation en tant que l'agent de contraste dans le diagnostic médical, de préférence dans le diagnostic des maladies tumorales, des inflammations et/ou de l'athérosclérose.

14. Utilisation de composés à base de cyclame selon l'une quelconque des revendications 1 à 5 et/ou au moins un conjugué selon la revendication 6 et/ou au moins un composé de coordination selon la revendication 7 et/ou au moins un conjugué de ciblage selon selon la revendication 8 et/ou la préparation pharmaceutique selon la revendication 11, en radiochimie en tant que marqueurs ou précurseurs de marqueurs lors de l'étiquetage à l'aide de radioisotopes de cuivre, lutétium, bismuth et yttrium.

15. Utilisation de composés à base de cyclame selon l'une quelconque des revendications 1 à 5 et/ou au moins un conjugué selon la revendication 6 et/ou au moins un composé de coordination selon la revendication 7 et/ou au moins un conjugué de ciblage selon à la revendication 8, pour la complexation et/ou la purification de radioisotopes de cuivre.
